(19) Europäisches Patentamt
European Patent Office
Office européen des brevets

(11) EP 2 722 380 B1

(12) **EUROPÄISCHE PATENTSCHRIFT**

(45) Veröffentlichungstag und Bekanntmachung des Hinweises auf die Patenterteilung:
**21.02.2018 Patentblatt 2018/08**

(51) Int Cl.:
***C09K 19/54*** *(2006.01)* ***C09K 19/30*** *(2006.01)*
***H04L 7/04*** *(2006.01)* ***H04L 7/08*** *(2006.01)*
***C09K 19/12*** *(2006.01)* ***C09K 19/42*** *(2006.01)*

(21) Anmeldenummer: **13004947.1**

(22) Anmeldetag: **16.10.2013**

(54) **Flüssigkristallines Medium, Methode zu seiner Stabilisierung und Flüssigkristallanzeige**

Liquid crystal medium, method for the stabilisation thereof and liquid crystal display

Fluide à base de cristaux liquides, son procédé de stabilisation et affichage à base de cristaux liquides

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.10.2012 DE 102012020372**

(43) Veröffentlichungstag der Anmeldung:
**23.04.2014 Patentblatt 2014/17**

(73) Patentinhaber: **Merck Patent GmbH**
**64293 Darmstadt (DE)**

(72) Erfinder:
- **Goebel, Mark**
**64289 Darmstadt (DE)**
- **Baron, Eveline**
**64285 Darmstadt (DE)**
- **Fortte, Rocco**
**65933 Frankfurt am Main (DE)**
- **Pauluth, Detlef**
**64372 Ober-Ramstadt (DE)**

(56) Entgegenhaltungen:
**EP-A2- 2 514 800**
**WO-A1-02/18515**
**WO-A1-2009/021671**
**WO-A1-2009/115186**
**WO-A1-2009/129911**
**WO-A1-2012/076104**
**DE-A1-102008 006 875**
**DE-A1-102011 008 687**
**DE-A1-102011 117 937**
**DE-A1-102012 004 871**
**US-A1- 2011 278 502**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 722 380 B1

## Beschreibung

Die vorliegende Erfindung betrifft neue Verbindungen insbesondere zur Verwendung in Flüssigkristallmedien, aber auch die Verwendung dieser Flüssigkristallmedien in Flüssigkristallanzeigen sowie diese Flüssigkristallanzeigen, besonders Flüssigkristallanzeigen, die den ECB-(electrically controlled birefringence) Effekt mit dielektrisch negativen Flüssigkristallen in einer homeotropen Ausgangsorientierung verwenden. Die erfindungsgemäßen Flüssigkristallmedien zeichnen sich durch eine besonders niedrige Schaltzeit in den erfindungsgemäßen Anzeigen bei gleichzeitig hohem Spannungshaltevermögen (Englisch "voltage holding ratio", kurz VHR oder auch nur HR) aus.

Das Prinzip der elektrisch kontrollierten Doppelbrechung, der ECB-Effekt oder auch DAP-Effekt (Deformation aufgerichteter Phasen) wurde erstmals 1971 beschrieben (M.F. Schieckel und K. Fahrenschon, "Deformation of nematic liquid crystals with vertical orientation in electrical fields", Appl. Phys. Lett. 19 (1971), 3912). Es folgten Arbeiten von J.F. Kahn (Appl. Phys. Lett. 20 (1972), 1193) und G. Labrunie und J. Robert (J. Appl. Phys. 44 (1973), 4869).

Die Arbeiten von J. Robert und F. Clerc (SID 80 Digest Techn. Papers (1980), 30), J. Duchene (Displays 7 (1986), 3) und H. Schad (SID 82 Digest Techn. Papers (1982), 244) haben gezeigt, dass flüssigkristalline Phasen hohe Werte für das Verhältnis der elastischen Konstanten $K_3/K_1$, hohe Werte für die optische Anisotropie $\Delta n$ und Werte für die dielektrische Anisotropie $\Delta\varepsilon \leq -0{,}5$ aufweisen müssen, um für hochinformative Anzeigeelemente basierend auf dem ECB-Effekt eingesetzt werden zu können. Auf dem ECB-Effekt basierende elektrooptische Anzeigeelemente weisen eine homöotrope Randorientierung auf (VA-Technologie = Vertical Aligned). Auch bei Anzeigen, die den so genannten IPS- (In Plane Switching) Effekt verwenden, können dielektrisch negative Flüssigkristallmedien zum Einsatz kommen.

Für die technische Anwendung dieses Effektes in elektrooptischen Anzeigeelementen werden FK-Phasen benötigt, die einer Vielzahl von Anforderungen genügen müssen. Besonders wichtig sind hier die chemische Beständigkeit gegenüber Feuchtigkeit, Luft und physikalischen Einflüssen wie Wärme, Strahlung im infraroten, sichtbaren und ultravioletten Bereich und elektrischen Gleich- und Wechselfeldern.

Ferner wird von technisch verwendbaren FK-Phasen eine flüssigkristalline Mesophase in einem geeigneten Temperaturbereich und eine niedrige Viskosität gefordert.

In keiner der bisher bekannten Reihen von Verbindungen mit flüssigkristalliner Mesophase gibt es eine Einzelverbindung, die allen diesen Erfordernissen entspricht. Es werden daher in der Regel Mischungen von zwei bis 25, vorzugsweise drei bis 18, Verbindungen hergestellt, um als FK-Phasen verwendbare Substanzen zu erhalten.

Matrix-Flüssigkristallanzeigen (MFK-Anzeigen) sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei im Allgemeinen Dünnfilm-Transistoren (TFT) verwendet werden, die in der Regel auf einer Glasplatte als Substrat angeordnet sind.

Man unterscheidet zwei Technologien: TFT's aus Verbindungshalbleitern wie z.B. CdSe oder TFT's auf der Basis von polykristallinem und u. a. amorphem Silizium. Letztere Technologie hat derzeit weltweit die größte kommerzielle Bedeutung.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die bisher am meisten verwendeten TFT-Anzeigen arbeiten üblicherweise mit gekreuzten Polarisatoren in Transmission und sind von hinten beleuchtet. Für TV Anwendungen werden IPS-Zellen oder ECB- (bzw. VAN-) Zellen verwendet, wohingegen für Monitore meist IPS-Zellen oder TN-(Twisted Nematic) Zellen und für "Note Books", "Lap Tops" und für mobile Anwendungen meist TN-Zellen Verwendung finden.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen, Monitore und "Note Books" oder für Displays mit hoher Informationsdichte z.B. in Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch einen nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige. Da der spezifische Widerstand der Flüssigkristallmischung durch Wechselwirkung mit den inneren Oberflächen der Anzeige im Allgemeinen über die Lebenszeit einer MFK-Anzeige abnimmt, ist ein hoher (Anfangs)-Widerstand sehr wichtig für Anzeigen die akzeptable Widerstandswerte über eine lange Betriebsdauer aufweisen müssen.

Anzeigen, die den ECB-Effekt verwenden haben sich als so genannte VAN- (Vertically Aligned Nematic) Anzeigen neben IPS-Anzeigen (z.B.: Yeo, S.D., Vortrag 15.3: "A LC Display for the TV Application", SID 2004 International Symposium, Digest of Technical Papers, XXXV, Buch II, S. 758 und 759) und den lange bekannten TN-Anzeigen, als eine der drei zur Zeit wichtigsten neueren Typen von Flüssigkristallanzeigen insbesondere für Fernsehanwendungen etabliert.

Als wichtigste Bauformen sind hier zu nennen: MVA (Multi-Domain Vertical Alignment, z. B.: Yoshide, H. et al., Vortrag 3.1: "MVA LCD for Notebook or Mobile PCs ...", SID 2004 International Symposium, Digest of Technical Papers, XXXV, Buch I, S. 6 bis 9 und Liu, C.T. et al., Vortrag 15.1: "A 46-inch TFT-LCD HDTV Technology ...", SID 2004 International Symposium, Digest of Technical Papers, XXXV, Buch II, S. 750 bis 753), PVA (Patterned Vertical Alignment, z. B.: Kim, Sang Soo, Vortrag 15.4: "Super PVA Sets New State-of-the-Art for LCD-TV", SID 2004 International Symposium, Digest of Technical Papers, XXXV, Buch II, S. 760 bis 763) und ASV (Avanced Super View, z. B.: Shigeta, Mitzuhiro und Fukuoka, Hirofumi, Vortrag 15.2: "Development of High Quality LCDTV", SID 2004 International Symposium, Digest of Technical Papers, XXXV, Buch II, S. 754 bis 757).

In allgemeiner Form werden die Technologien z.B. in Souk, Jun, SID Seminar 2004, Seminar M-6: "Recent Advances in LCD Technology", Seminar Lecture Notes, M-6/1 bis M-6/26 und Miller, Ian, SID Seminar 2004, Seminar M-7: "LCD-Television", Seminar Lecture Notes, M-7/1 bis M-7/32, verglichen. Obwohl die Schaltzeiten moderner ECB-Anzeigen durch Ansteuerungsmethoden mit Übersteuerung (overdrive) bereits deutlich verbessert wurden, z.B.: Kim, Hyeon Kyeong et al., Vortrag 9.1: "A 57-in. Wide UXGA TFT-LCD for HDTV Application", SID 2004 International Symposium, Digest of Technical Papers, XXXV, Buch I, S. 106 bis 109, ist die Erzielung von videotauglichen Schaltzeiten insbesondere beim Schalten von Graustufen immer noch ein noch nicht zufriedenstellend gelöstes Problem.

ECB-Anzeigen verwenden wie ASV-Anzeigen flüssigkristalline Medien mit negativer dielektrischer Anisotropie ($\Delta\varepsilon$), wohingegen TN- und bislang alle gebräuchlichen IPS-Anzeigen flüssigkristalline Medien mit positiver dielektrischer Anisotropie verwenden.

In derartigen Flüssigkristallanzeigen werden die Flüssigkristalle als Dielektrika verwendet, deren optische Eigenschaften sich bei Anlegen einer elektrischen Spannung reversibel ändern.

Da bei Anzeigen im allgemeinen, also auch bei Anzeigen nach diesen erwähnten Effekten, die Betriebsspannung möglichst gering sein soll, werden Flüssigkristallmedien eingesetzt, die in der Regel überwiegend aus Flüssigkristallverbindungen zusammengesetzt sind, die alle das gleiche Vorzeichen der dielektrischen Anisotropie aufweisen und einen möglichst großen Betrag der dielektrischen Anisotropie haben. Es werden in der Regel allenfalls geringere Anteile an neutralen Verbindungen und möglichst keine Verbindungen mit einem Vorzeichen der dielektrischen Anisotropie, das dem des Mediums entgegengesetzt ist, eingesetzt. Bei den Flüssigkristallmedien mit negativer dielektrischer Anisotropie für ECB-Anzeigen werden somit überwiegend Verbindungen mit negativer dielektrischer Anisotropie eingesetzt. Die eingesetzten Flüssigkristallmedien bestehen in der Regel überwiegend und meist sogar weitestgehend aus Flüssigkristallverbindungen mit negativer dielektrischer Anisotropie.

Bei den gemäß der vorliegenden Anmeldung verwendeten Medien werden typischerweise allenfalls nennenswerte Mengen an dielektrisch neutralen Flüssigkristallverbindungen und in der Regel nur sehr geringe Mengen an oder gar keine dielektrisch positiven Verbindungen eingesetzt, da generell die Flüssigkristallanzeigen möglichst niedrige Ansteuerspannungen haben sollen.

In DE 2008 006 875 A1 werden Flüssigkristallmischungen offenbart, die die Alkenylverbindungen der Formeln

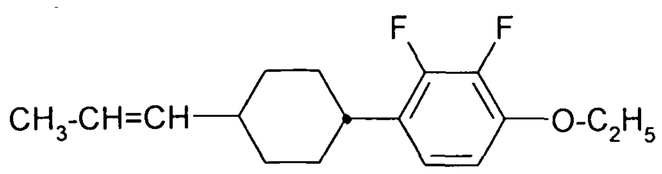

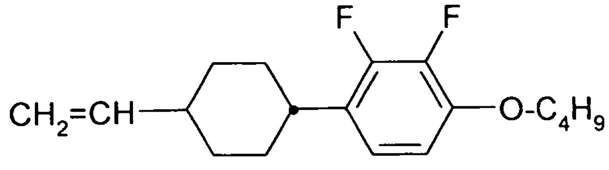

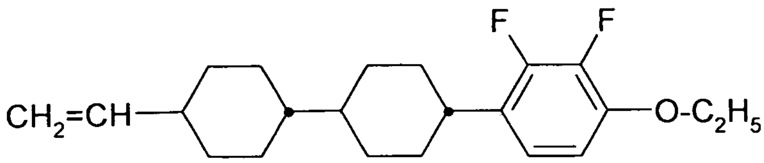

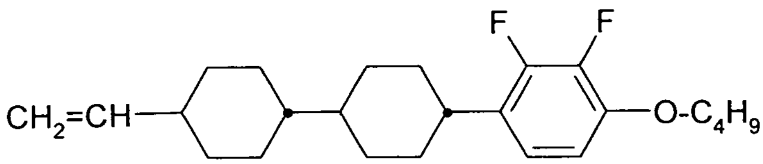

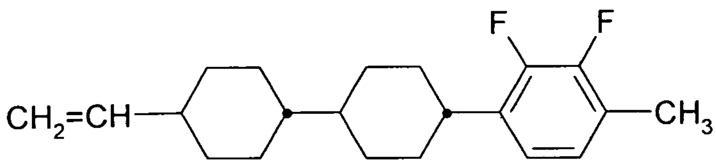

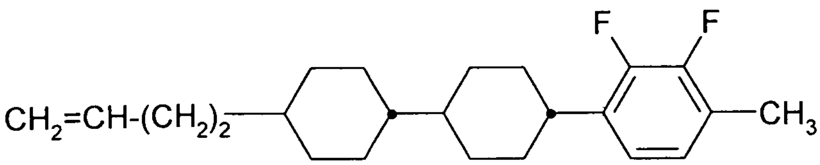

bzw. der Formeln

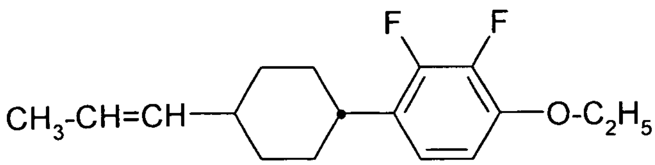

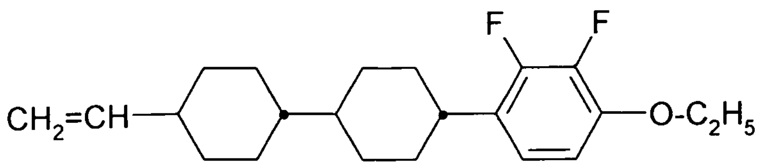

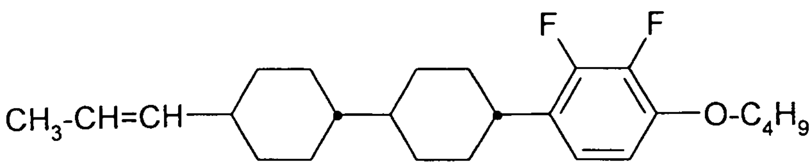

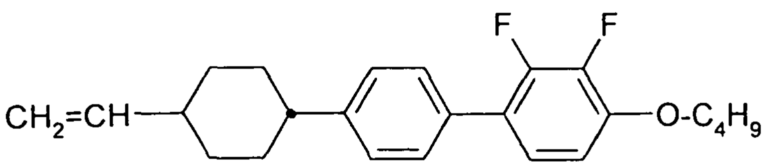

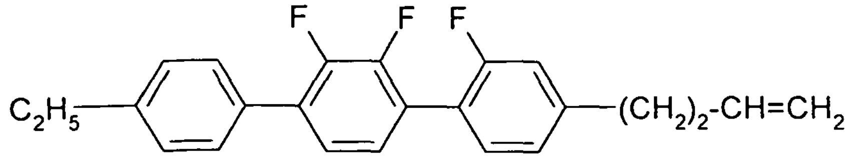

enthalten. Diese Mischungen weisen jedoch Eigenschaften auf, die für viele anspruchsvolle Anwendungen keinesfalls ausreichen. Auch werden keine Stabilisatoren in diesen Mischungen verwendet.

DE 10 2011 008 687 A1 erwähnt, neben vielen anderen Verbindungen, auch Alkenylverbindungen der Formeln

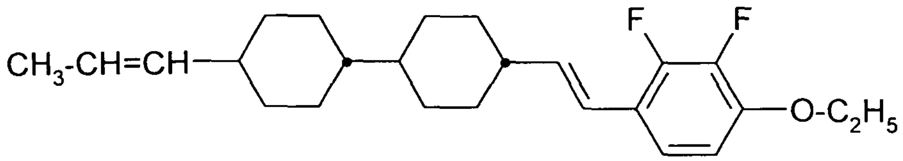

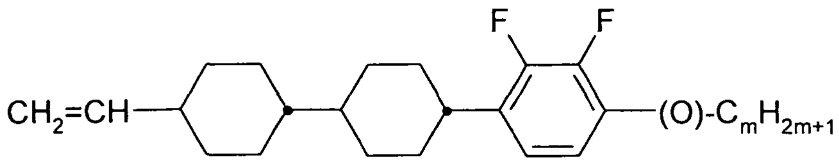

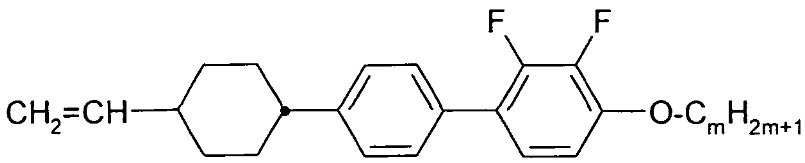

zur Verwendung in Flüssigkristallmischungen. Die Flüssigkristallmischungen dieser Schrift sollen jedoch zur Verhinderung einer Verschlechterung der SEigenschaften durch die Bestrahlung mit einer Hintergrundbeleuchtung in einer Anzeige mit einem Kantenfilter in der Hintergrundbeleuchtung betrieben werden.

Für viele praktische Anwendungen in Flüssigkristallanzeigen sind die bekannten Flüssigkristallmedien jedoch nicht stabil genug. Insbesondere ihre Stabilität gegen die Bestrahlung mit UV, aber auch bereits mit den üblichen Hintergrundbeleuchtungen führt zu einer Verschlechterung insbesondere der elektrischen Eigenschaften. So nimmt z. B. die Leitfähigkeit signifikant zu.

Zur Stabilisierung von Flüssigkristallmischungen wurde bereits die Verwendung von sogenannten "Hindered Amine Light Stabilizers", kurz HALS, vorgeschlagen.

Nematische Flüssigkristallmischungen mit negativer dielektrische Anisotropie, die eine geringe Menge an TINUVIN®770, einer Verbindung der Formel

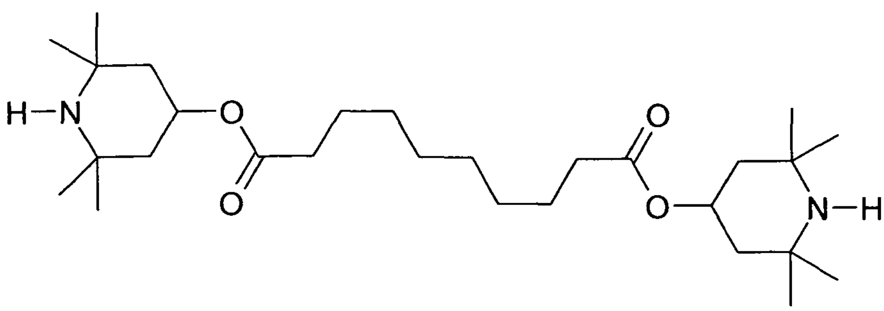

,

als Stabilisatoren enthalten, werden z.B. in WO 2009/129911 A1 vorgeschlagen. Die entsprechenden Flüssigkristallmischungen haben jedoch für einige praktische Anwendungen nicht ausreichende Eigenschaften. Unter anderem sind sie

nicht genügend stabil gegen die Belastung durch die Bestrahlung mit typischen CCFL-((Cold Cathod Flourescent Lamp) Hintergrundbeleuchtungen.

Ähnliche Flüssigkristallmischungen sind z.B. auch aus EP 2 182 046 A1, WO 2008/009417 A1, WO 2009/021671 A1 und WO 2009/115186 A1 bekannt. Dort wird jedoch nicht auf die Verwendung von Stabilisatoren hingewiesen.

Diese Flüssigkristallmischungen können gemäß der dortigen Offenbarung optional auch Stabilisatoren verschiedener Arten, wie z. B. Phenole und sterisch gehinderte Amine (Englisch: hindered amine light stabilizers, kurz: HALS) enthalten. Diese Flüssigkristallmischungen sind jedoch durch relativ hohe Schwellenspannungen und durch bestenfalls moderate Stabilitäten gekennzeichnet. Insbesondere sinkt deren "Voltage Holding Ratio" nach Belastung. Außerdem tritt oft eine gelbliche Verfärbung auf.

Die Verwendung verschiedener Stabilisatoren in flüssigkristallinen Medien wird z. B. in JP (S)55-023169 (A), JP (H)05-117324 (A), WO 02/18515 A1 und JP (H) 09-291282 (A) beschrieben.

Auch TINUVIN®123, eine Verbindung der Formel

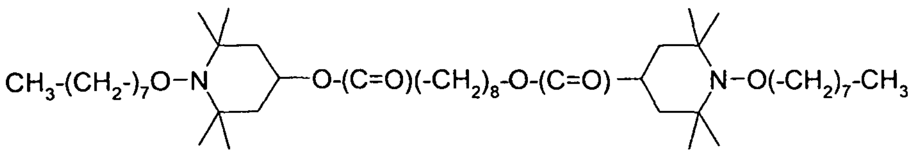

wurde zu Stabilisierungszwecken vorgeschlagen.

Mesogene Verbindungen mit einer oder zwei HALS-Einheiten werden in EP 1 1784 442 A1 offenbart.

HALS mit verschiedenen Substituenten am Stickstoffatom werden in Ohkatsu, Y., J. of Japan Petroleum Institute, 51, 2008, Seiten 191-204 bezüglich ihrer $pK_B$-Werte verglichen. Dabei werden die folgenden Typen von Strukturformeln offenbart.

| Typ | Aktive Gruppe des Stabilisators |
|---|---|
| "HALS" | 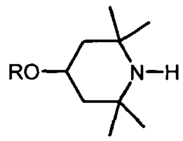 |
| "R-HALS" oder "NR-HALS" | 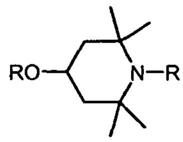 |
| "NOR-HALS" | 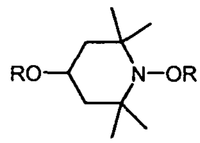 |

Die Verbindung TEMPOL, der folgenden Formel

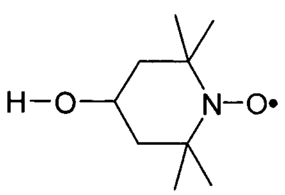

TEMPOL

ist bekannt, sie wird z.B. in Miéville, P. et al, Angew. Chem. 2010, 122, Seiten 6318-6321 erwähnt. Sie ist von verschiedenen Herstellern kommerziell erhältlich und wird z. B. in Formulierungen für Vorläufer für Polyolefine, Polystyrole, Polyamide, Beschichtungen und PVC als Polymerisationsinhibitor und, insbesondere in Kombination mit UV-Absorbern, als Licht- bzw. UV-Schutz eingesetzt.

Auch in US 2011/278502 A1, WO 2012/076104 A1, DE 10 2011 117937 A12, DE 10 2012 004871 A1 und EP 2 514 800 A2 werden Flüssigkristallmischungen, die optional auch Additive, wie z.B. Stabilisatoren, enthalten können,

für die Anwendung in Anzeigen beschrieben.

Die Flüssigkristallmedien des Standes der Technik mit entsprechend niedrigen Ansteuerspannungen haben relativ geringe elektrische Widerstände bzw. eine geringe VHR und führen in den Anzeigen oft zu unerwünschtem "Flicker" und/oder ungenügender Transmission. Außerdem sind sie nicht ausreichend stabil gegen Temperatur- und/oder UV-Belastung, zumindest dann, wenn sie eine entsprechend hohe Polarität aufweisen, wie sie für niedrige Ansteuerspannungen nötig ist.

Andererseits ist die Ansteuerspannung der Anzeigen des Standes der Technik, die eine hohe VHR aufweisen, oft zu groß, insbesondere für Anzeigen die nicht direkt oder nicht durchgehend an das Stromversorgungsnetz angeschlossen werden wie z. B. Anzeigen für mobile Anwendungen.

Außerdem muss der Phasenbereich der Flüssigkristallmischung ausreichend breit für die beabsichtigte Anwendung der Anzeige sein.

Die Schaltzeiten der Flüssigkristallmedien in den Anzeigen müssen verbessert, also verringert, werden. Dies ist besonders für Anzeigen für Fernseh- oder Multi-Media Anwendungen wichtig. Zur Verbesserung der Schaltzeiten ist in der Vergangenheit wiederholt vorgeschlagen worden, die Rotationsviskosität der Flüssigkristallmedien ($\gamma 1$) zu optimieren, also Medien mit einer möglichst geringen Rotationsviskosität zu realisieren. Die dabei erzielten Ergebnisse sind jedoch nicht ausreichend für viele Anwendungen und lassen es daher wünschenswert erscheinen, weitere Optimierungsansätze aufzufinden.

Ganz besonders wichtig ist eine ausreichende Stabilität der Medien gegen extreme Belastungen, insbesondere gegen UV- und Temperaturbelastung. Besonders bei Anwendungen in Anzeigen in mobilen Geräten wie z. B. Mobiltelefonen kann dieses entscheidend sein.

Der Nachteil der bisher bekannten MFK-Anzeigen beruht auf ihrem vergleichsweise niedrigen Kontrast, der relativ hohen Blickwinkelabhängigkeit und der Schwierigkeit in diesen Anzeigen Graustufen zu erzeugen, sowie ihrer ungenügenden VHR und ihrer ungenügenden Lebensdauer.

Es besteht somit immer noch ein großer Bedarf nach MFK-Anzeigen mit sehr hohem spezifischen Widerstand bei gleichzeitig großem Arbeitstemperaturbereich, kurzen Schaltzeiten und niedriger Schwellenspannung, mit deren Hilfe verschiedene Graustufen erzeugt werden können und die insbesondere eine gute und stabile VHR aufweisen.

Der Erfindung liegt die Aufgabe zugrunde MFK-Anzeigen, nicht nur für Monitor- und TV-Anwendungen, sondern auch für Mobiltelefone und Navigationssysteme, welche auf dem ECB- oder auf dem IPS-Effekt beruhen, bereitzustellen, die die oben angegebenen Nachteile nicht oder nur in geringerem Maße und gleichzeitig sehr hohe spezifische Widerstände aufweisen. Insbesondere muss für Mobiltelefone und Navigationssysteme gewährleistet sein, dass diese auch bei extrem hohen und extrem niedrigen Temperaturen arbeiten.

Überraschend wurde gefunden, dass Flüssigkristallanzeigen realisiert werden können, die insbesondere in ECB-Anzeigen eine niedrige Schwellenspannung bei geringen Schaltzeiten aufweisen und gleichzeitig eine ausreichend breite nematische Phase, eine günstige, relativ niedrige Doppelbrechung ($\Delta n$), gute Stabilität gegen Zersetzung durch thermische und durch UV-Belastung und eine stabile hohe VHR aufweisen, wenn man in diesen Anzeigeelementen nematische Flüssigkristallmischungen verwendet, die mindestens eine Verbindung der Formel I, sowie jeweils mindestens eine Verbindung der Formel II, bevorzugt ausgewählt aus der Gruppe der Verbindungen der Unterformeln II-1 bis II-4, besonders bevorzugt der Unterformeln II-1 und/oder II-2, und bevorzugt zusätzlich mindestens eine Verbindung ausgewählt aus der Gruppe der Verbindungen der Formeln III-1 bis III-4, bevorzugt der Formel III-3, enthalten und/oder mindestens eine Verbindung der Formeln IV und/oder V.

Derartige Medien sind insbesondere für elektrooptische Anzeigen mit einer Aktivmatrix-Adressierung basierend auf dem ECB-Effekt sowie für IPS-Anzeigen (In plane switching) zu verwenden.

Gegenstand der Erfindung ist somit ein flüssigkristallines Medium auf der Basis eines Gemisches von polaren Verbindungen, welches mindestens eine Verbindung der Formel I und eine oder mehrere Verbindungen der Formel II und bevorzugt zusätzlich eine oder oder mehrere Verbindungen ausgewählt aus der Gruppe der Verbindungen der Formeln III-1 bis III-4 und/oder zusätzlich eine oder mehrere Verbindungen der Formeln IV und/oder V enthält.

Die erfindungsgemäßen Mischungen zeigen sehr breite nematische Phasenbereiche mit Klärpunkten $\geq$ 70°C, sehr günstige Werte für die kapazitive Schwelle, relativ hohe Werte für die Holding Ratio und gleichzeitig gute Tieftemperaturstabilitäten bei -20°C und -30°C, sowie sehr geringe Rotationsviskositäten. Weiterhin zeichnen sich die erfindungsgemäßen Mischungen durch ein gutes Verhältnis von Klärpunkt und Rotationsviskosität und durch eine hohe negative dielektrische Anisotropie aus.

Überraschenderweise wurde nun gefunden, dass flüssigkristalline Medien mit einem geeignet hohen $\Delta\varepsilon$, einem geeigneten Phasenbereich und $\Delta n$ verwirklicht werden können, welche die Nachteile der Materialien des Standes der Technik nicht oder zumindest nur in erheblich geringerem Maße aufweisen.

Hier wurde überraschend gefunden, dass die Verbindungen der Formel I, auch wenn sie alleine ohne zusätzliche Temperaturstabilisatoren verwendet werden, zu einer erheblichen, in vielen Fällen ausreichenden, Stabilisierung von Flüssigkristallmischungen sowohl gegen UV-Belastung, als auch gegen Temperaturbelastung führen.

Eine ausreichende Stabilisierung von Flüssigkristallmischungen sowohl gegen UV-Belastung, als auch gegen

Temperaturbelastung kann aber auch insbesondere dann erreicht werden, wenn zusätzlich zu der Verbindung der Formel I, bzw. den Verbindungen der Formel I, eine oder mehrere weitere Verbindungen, bevorzugt phenolische Stabilisatoren, in der Flüssigkristallmischung zugegen sind. Diese weiteren Verbindungen sind als Stabilisatoren gegen thermische Belastungen geeignet.

Die Erfindung betrifft somit Verbindungen der Formel I, sowie ein flüssigkristallines Medium mit einer nematischen Phase und einer negativen dielektrischen Anisotropie, welches

a) eine oder mehrere Verbindungen der Formel I, bevorzugt in einer Konzentration im Bereich von 1 ppm bis zu 1.000 ppm, bevorzugt im Bereich von 1 ppm bis zu 500 ppm, besonders bevorzugt im Bereich von 1 ppm bis zu 250 ppm,

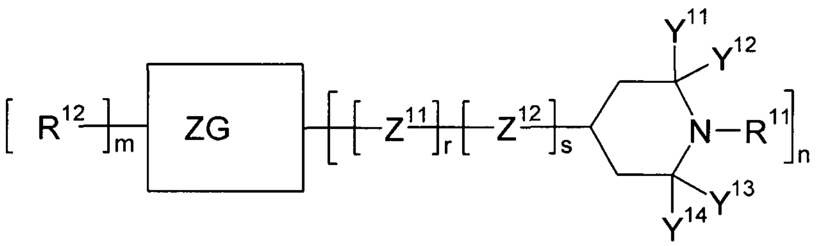 I

worin

n eine ganze Zahl von 1 bis 4, bevorzugt, 1, 2 oder 3, besonders bevorzugt 1 oder 2, und ganz besonders bevorzugt 2,

m (4-n),

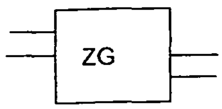

ein organischer Rest mit 4 Bindungsstellen, bevorzugt eine Alkantetrayleinheit mit 1 bis 20 C-Atomen in der zusätzlich zu den im Molekül vorhandenen m Gruppen $R^{12}$, aber unabhängig davon, ein weiteres H-Atom durch $R^{12}$ ersetzt sein kann oder mehrere weitere H-Atome durch $R^{12}$ ersetzt sein können, bevorzugt eine geradkettige Alkantetrayleinheit mit je einer Valenz an je einem der beiden endständigen C-Atome worin eine $-CH_2-$ -Gruppe oder mehrere $-CH_2-$ -Gruppen durch -O- oder -(C=O)- so ersetzt sein können, dass nicht zwei O-Atome direkt miteinander verbunden sind, oder ein substituierter oder unsubstituierter aromatischer oder heteroaromatischer Kohlenwasserstoffrest mit 1 bis 4 Valenzen, in dem zusätzlich zu den im Molekül vorhandenen m Gruppen $R^{12}$, aber unabhängig davon, ein weiteres H-Atom durch $R^{12}$ ersetzt sein kann oder mehrere weitere H-Atome durch $R^{12}$ ersetzt sein können,

$Z^{11}$ und $Z^{12}$ unabhängig voneinander -O-, -(C=O)-, $-(N-R^{14})-$ oder eine Einfachbindung, jedoch nicht beide gleichzeitig -O-,

r und s unabhängig voneinander 0 oder 1,

$Y^{11}$ bis $Y^{14}$ jeweils unabhängig voneinander Alkyl mit 1 bis 4 C-Atomen, bevorzugt Methyl oder Ethyl, besonders bevorzugt alle entweder Methyl oder Ethyl und ganz besonders bevorzugt Methyl, und alternativ auch unabhängig voneinander eines oder beide der Paare ($Y^{11}$und $Y^{12}$) und ($Y^{13}$ und $Y^{14}$) gemeinsam eine zweibindige Gruppe mit 3 bis 6 C-Atomen, bevorzugt mit 5 C-Atomen, besonders bevorzugt 1,5-Pentylen,

$R^{12}$ bei jedem Auftreten unabhängig voneinander H, F, $OR^{14}$, $NR^{14}R^{15}$ eine geradkettige oder verzweigte Alkylkette mit 1-20 C-Atomen, in der eine $-CH_2-$ -Gruppe oder mehrere $-CH_2-$ -Gruppen durch -O- oder -C(=O)- ersetzt sein können, jedoch nicht zwei benachbarte $-CH_2-$ -Gruppen durch -O- ersetzt sein können, einen Kohlenwasserstoffrest, der eine Cycloalkyl- oder eine Alkylcycloalkyleinheit enthält, und, in dem eine $-CH_2-$-Gruppe oder mehrere $-CH_2-$ -Gruppen durch -O- oder -C(=O)- ersetzt sein können, jedoch nicht zwei benachbarte $-CH_2-$ -Gruppen durch -O- ersetzt sein können, und in dem ein H-Atom oder mehrere H-Atome durch $OR^{14}$, $N(R^{14})(R^{15})$

oder $R^{16}$ ersetzt sein können, oder ein aromatischer oder heteroaromatischen Kohlenwasserstoffrest, in denen ein H-Atom oder mehrere H-Atome durch $OR^{14}$, $N(R^{14})(R^{15})$ oder $R^{16}$ ersetzt sein können,

$R^{14}$ bei jedem Auftreten unabhängig voneinander eine geradkettige oder verzweigte Alkyl- oder Acylgruppe mit 1 bis 10 C-Atomen, bevorzugt *n*-Akyl, oder einen aromatischen Kohlenwasserstoff- oder Carbonsäurerest mit 6-12 C-Atomen, bevorzugt mit der Maßgabe, dass bei $N(R^{14})(R^{15})$ mindestens ein Acylrest vorhanden ist,

$R^{15}$ bei jedem Auftreten unabhängig voneinander eine geradkettige oder verzweigte Alkyl- oder Acylgruppe mit 1 bis 10 C-Atomen, bevorzugt *n*-Akyl, oder einen aromatischen Kohlenwasserstoff- oder Carbonsäurerest mit 6-12 C-Atomen, bevorzugt mit der Maßgabe, dass bei $N(R^{14})(R^{15})$ mindestens ein Acylrest vorhanden ist,

$R^{16}$ bei jedem Auftreten unabhängig voneinander eine geradkettige oder verzweigte Alkylgruppe mit 1 bis 10 C-Atomen in dem eine $-CH_2-$-Gruppe oder mehrere $-CH_2-$-Gruppen durch -O- oder -C(=O)- ersetzt sein können, jedoch nicht zwei benachbarte $-CH_2-$-Gruppen durch -O- ersetzt sein können,

bedeutet, mit den Maßgaben, dass,
Verbindungen der Formel

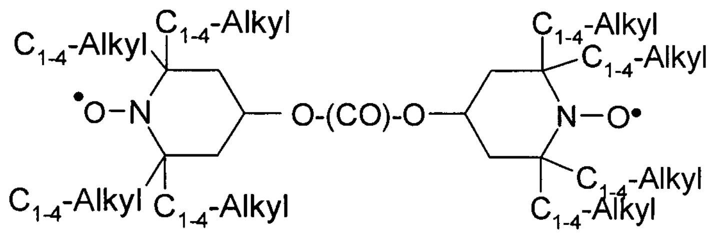

von den Verbindungen der Formel I ausgeschlossen sind, und
im Fall n = 1 und
$-[Z^{11}-]_r-[Z^{12}]_s-$ = -O-, -(CO)-O-, -O-(CO)-, -O-(CO)-O-, $-NR^{14}-$ oder $-NR^{14}-(CO)-$,

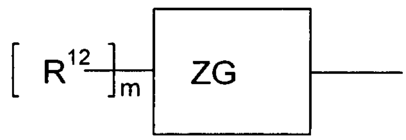

nicht
geradkettiges oder verzweigtes Alkyl mit 1 bis 10 C-Atomen, auch Cycloalkyl, Cycloalkylalkyl, oder Alkylcycloalkyl, wobei in allen diesen Gruppen eine oder mehrere $-CH_2-$ Gruppen durch -O- so ersetzt sein können, dass im Molekül keine zwei O-Atome direkt miteinander verbunden sind, bedeutet, und

b) eine oder mehrere Verbindungen der Formel II

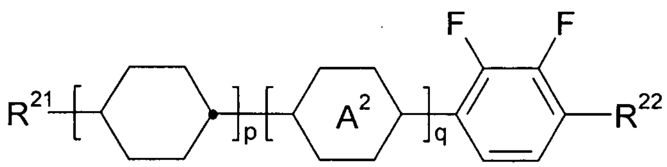

II

worin

$R^{21}$ einen unsubstituierten Alkenylrest mit 2 bis 7 C-Atomen, bevorzugt einen geradkettigen Alkenylrest, besonders bevorzugt mit 2 bis 5 C-Atomen,

$R^{22}$ einen unsubstituierten Alkylrest mit 1 bis 7 C-Atomen, bevorzugt mit 2 bis 5 C-Atomen, einen unsubstituierten Alkoxyrest mit 1 bis 6 C-Atomen, bevorzugt mit 1, 2, 3 oder 4 C-Atomen oder einen unsubstituierten Alkenyloxyrest mit 2 bis 6 C-Atomen,

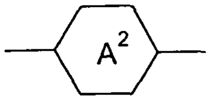

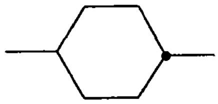 , 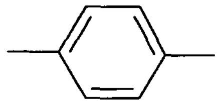 , 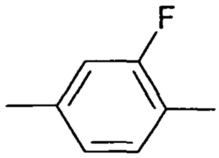 ,

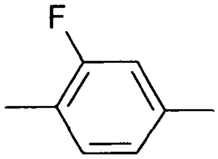 oder 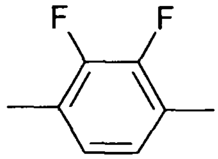 ,

bevorzugt

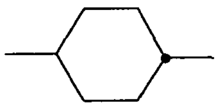 oder 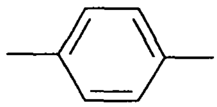 ,

p und q jeweils unabhängig voneinander 0, 1 oder 2, p bevorzugt 1 oder 2, q bevorzugt 1 und

(p + q) 1, 2, oder 3, bevorzugt 1 oder 2,

bedeuten,

c) optional, bevorzugt obligatorisch, eine oder mehrere Verbindungen ausgewählt aus der Gruppe der Formeln III-1 bis III-4, bevorzugt der Formel III-3,

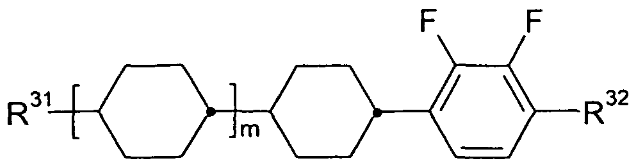

III-1

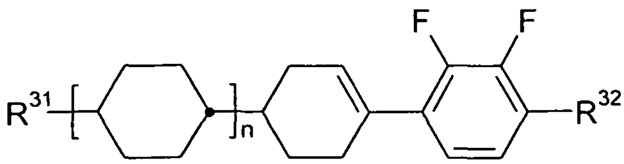

III-2

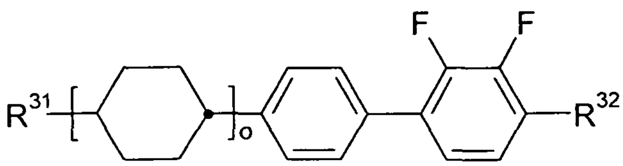

III-3

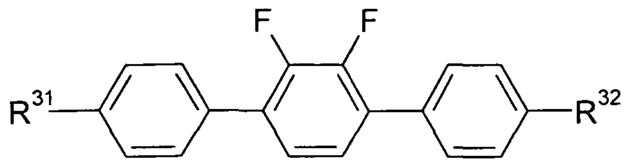

III-4

worin

R31 einen unsubstituierten Alkylrest mit 1 bis 7 C-Atomen, bevorzugt einen *n*-Alkylrest, besonders bevorzugt mit 2 bis 5 C-Atomen,

R32 einen unsubstituierten Alkylrest mit 1 bis 7 C-Atomen, bevorzugt mit 2 bis 5 C-Atomen, einen unsubstituierten Alkoxyrest mit 1 bis 6 C-Atomen, bevorzugt mit 2, 3 oder 4 C-Atomen oder einen unsubstituierten Alkenyloxyrest mit 2 bis 6 C-Atomen, und

m, n und o jeweils unabhängig voneinander 0 oder 1,

bedeuten,
und

d) optional, bevorzugt obligatorisch, eine oder mehrere Verbindungen ausgewählt aus der Gruppe der Verbindungen der Formeln IV und V, bevorzugt der Formel IV,

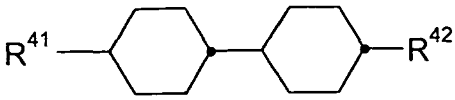

IV

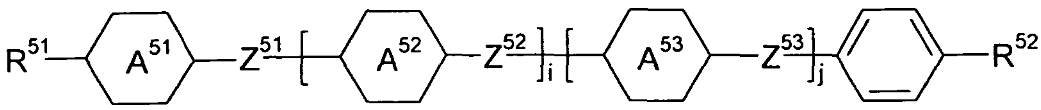

V

worin

$R^{41}$ einen unsubstituierten Alkylrest mit 1 bis 7 C-Atomen oder einen unsubstituierten Alkenylrest mit 2 bis 7 C-Atomen, bevorzugt einen *n*-Alkylrest, besonders bevorzugt mit 2, 3, 4 oder 5 C-Atomen, und

$R^{42}$ einen unsubstituierten Alkylrest mit 1 bis 7 C-Atomen oder einen unsubstituierten Alkoxyrest mit 1 bis 6 C-Atomen, beide bevorzugt mit 2 bis 5 C-Atomen, einen unsubstituierten Alkenylrest mit 2 bis 7 C-Atomen, bevorzugt mit 2, 3 oder 4 C-Atomen, stärker bevorzugt einen Vinylrest oder einen 1-Propenylrest und insbesondere einen Vinylrest,

$R^{51}$ und $R^{52}$ unabhängig voneinander eine der für $R^{21}$ und $R^{22}$ gegebenen Bedeutung haben und bevorzugt Alkyl mit 1 bis 7 C-Atomen, bevorzugt *n*-Alkyl, besonders bevorzugt *n*-Alkyl mit 1 bis 5 C-Atomen, Alkoxy mit 1 bis 7 C-Atomen, bevorzugt *n*-Alkoxy, besonders bevorzugt *n*-Alkoxy mit 2 bis 5 C-Atomen, Alkoxyalkyl, Alkenyl oder Alkenyloxy mit 2 bis 7 C-Atomen, bevorzugt mit 2 bis 4 C-Atomen, bevorzugt Alkenyloxy,

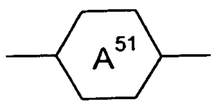

bis

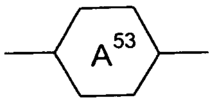

, soweit vorhanden, jeweils unabhängig voneinander

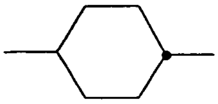 , 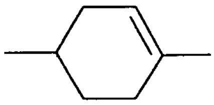 , 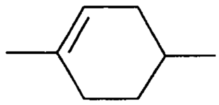 ,

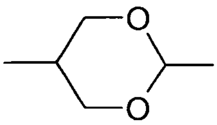 , 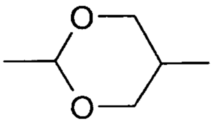 , 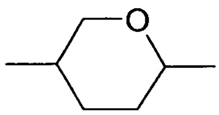 ,

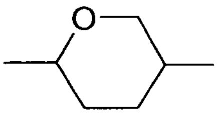 , 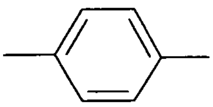 , 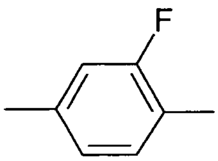 ,

oder

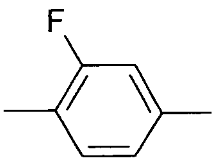 ,

bevorzugt

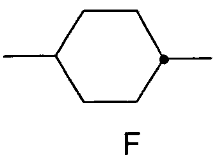 , 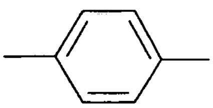 , 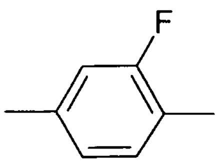 ,

oder

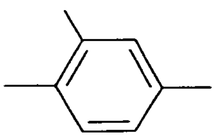 ,

bevorzugt

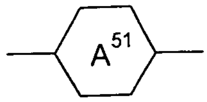 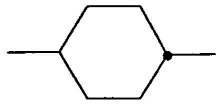,

und, wenn vorhanden,

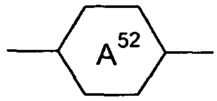

bevorzugt

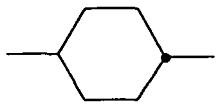,

$Z^{51}$ bis $Z^{53}$ jeweils unabhängig voneinander-$CH_2$-$CH_2$-, -$CH_2$-O-, -CH=CH-, -C≡C-, -COO- oder eine Einfachbindung, bevorzugt -$CH_2$-$CH_2$-, -$CH_2$-O- oder eine Einfachbindung und besonders bevorzugt eine Einfachbindung,

i und j jeweils unabhängig voneinander 0 oder 1,

(i + j) bevorzugt 0 oder 1,

bedeuten,
enthält.

Bevorzugt sind die folgenden Ausführungsformen

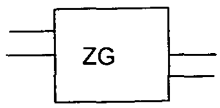

bedeutet

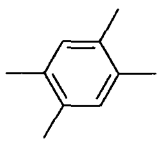

(Benzol-1,2,4,5-tetrayl) oder -$CH_2$-(CH-)-$[CH_2]_q$-(CH-)-$CH_2$-oder
>CH-$[CH_2]_p$-CH<, (mit p ∈ {0, 1, 2, 3, 4, 5 bis 18} und q ∈ {0, 1, 2, 3, bis 16}) oder

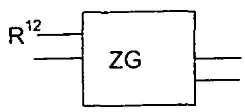

bedeutet
>CH-$[CH_2]_p$-$CH_2$- (mit p ∈ {0, 1, 2, 3, 4, 5 to 18}) oder

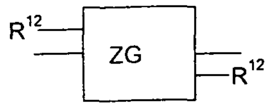

bedeutet

$-CH_2-[CH_2]_p-CH_2-$ (mit p ∈ {0, 1, 2, 3, 4, 5 to 18}), Propan-1,2-diyl, Butan-1,2-diyl, Ethan-1,2-diyl,

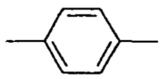
(1,4-Phenylen),

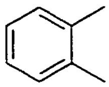
(1,2-Phenylen)

oder

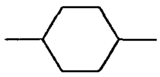
(1,4-Cyclohexylen).

In der vorliegenden Anmeldung umfassen die Elemente alle ihre jeweiligen Isotope. Insbesondere können in den Verbindungen ein oder mehrere H durch D ersetzt sein und dieses ist in einigen Ausführungsformen auch besonders bevorzugt. Ein entsprechend hoher Deuterierungsgrad der entsprechenden Verbindungen ermöglicht z.B. eine Detektion und Wiedererkennung der Verbindungen. Dieses ist insbesondere bei den Verbindungen der Formel I in einigen Fällen sehr hilfreich.

In der vorliegenden Anmeldung bedeutet

| | |
|---|---|
| Alkyl | besonders bevorzugt geradkettiges Alkyl, insbesondere $CH_3$-, $C_2H_5$-, *n*-$C_3H_7$, *n*-$C_4H_9$- oder *n*-$C_5H_{11}$-, und |
| Alkenyl | besonders bevorzugt $CH_2$=CH-, *E*-$CH_3$-CH=CH-, $CH_2$=CH-$CH_2$-$CH_2$-, *E*-$CH_3$-CH=CH-$CH_2$-$CH_2$- oder *E*-(*n*-$C_3H_7$)-CH=CH-. |

Bevorzugt enthalten die flüssigkristallinen Medien gemäß der vorliegenden Anmeldung insgesamt 1 ppm bis 1.000 ppm, bevorzugt 1 ppm bis 500 ppm, noch stärker bevorzugt 1 bis 250 ppm, bevorzugt bis 200 ppm, und, ganz besonders bevorzugt, 1 ppm bis 100 ppm an Verbindungen der Formel I.

Bevorzugt beträgt die Konzentration der Verbindungen der Formel I in den erfindungsgemäßen Medien 90 ppm oder weniger, besonders bevorzugt 50 ppm oder weniger. Ganz besonders bevorzugt beträgt die Konzentration der Verbindungen der Formel I in den erfindungsgemäßen Medien 10 ppm oder mehr bis 80 ppm oder weniger.

In einer bevorzugten Ausführungsform der vorliegenden Erfindung bedeutet bei den Verbindungen der Formel I

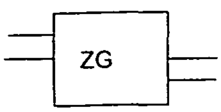

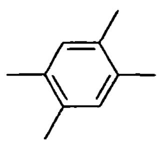
(Benzol-1,2,4,5-tetrayl)

oder

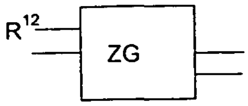 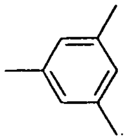 (Benzol-1,3,5-triyl)

oder

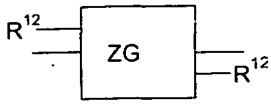

$-(CH_2-)_2$, $-(CH_2-)_4$, $-(CH_2-)_6$, $-(CH_2-)_8$, Propan-1,2-diyl, Butan-1,2-diyl, Ethan-1,2-diyl,

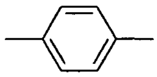 (1,4-Phenylen),

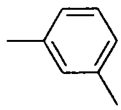 (1,3-Phenylen),

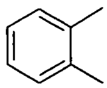 (1,2-Phenylen)

oder

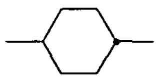 (*trans*-1,4-Cyclohexylen)

und/oder

| | |
|---|---|
| $-[Z^{11}-]_r-[Z^{12}-]_s$ | bei jedem Auftreten unabhängig voneinander -O-, -(C=O)-O- oder -O-(C=O)-, $-(N-R^{14})-$ oder eine Einfachbindung, bevorzugt -O- oder -(C=O)-O- oder -O-(C=O)-, und/oder |
| $R^{11}$ | $-O^{\bullet}$, OH oder $O-R^{13}$, bevorzugt: $-O^{\bullet}$, $-O-CH(-CH_3)_2$, $-O-CH(-CH_3)(-CH_2)_3-CH_3$, $-O-CH(-C_2H_5)(-CH_2)_3-CH_3$, |

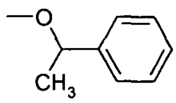 oder 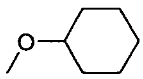

und/oder

| | |
|---|---|
| $R^{12}$, | wenn vorhanden, Alkyl oder Alkoxy, und/oder |
| $R^{13}$, | *iso*-Propyl oder 3-Heptyl, Acetophenyl oder Cyclohexyl. |

In einer bevorzugten Ausführungsform der vorliegenden Erfindung bedeutet bei den Verbindungen der Formel I die Gruppierung

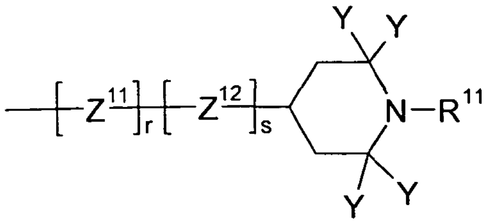
,

bei jedem Auftreten unabhängig voneinander,

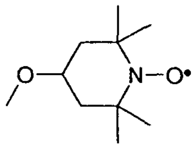
,
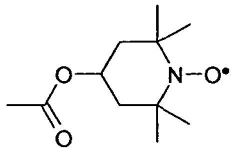
,

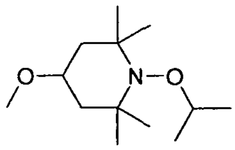
,
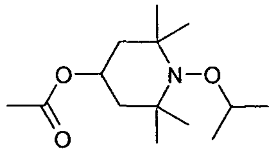
,

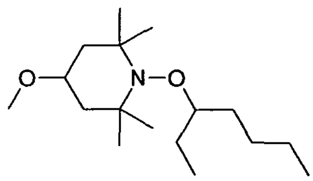
,
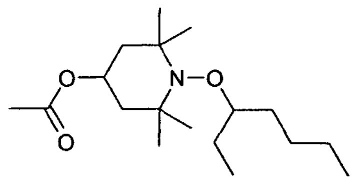
,

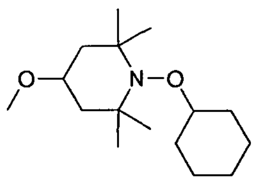
,
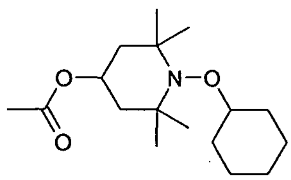
,

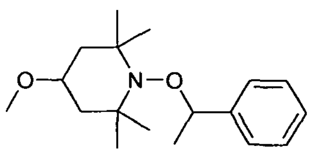
oder
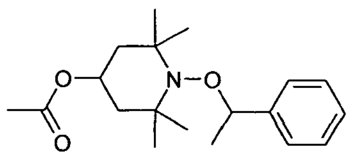
,

bevorzugt

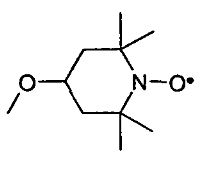
oder
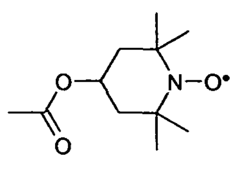
.

In einer besonders bevorzugten Ausführungsform der vorliegenden Erfindung haben bei den Verbindungen

der Formel I alle vorhandenen Gruppierungen

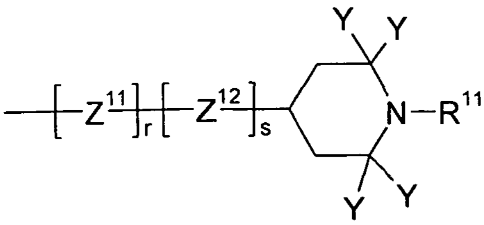

dieselbe Bedeutung.

Diese Verbindungen eignen sich hervorragend als Stabilisatoren in Flüssigkristallmischungen. Insbesondere stabilisieren sie die VHR der Mischungen gegen UV-Belastung.

In einer bevorzugten Ausführungsform der vorliegenden Erfindung enthalten die erfindungsgemäßen Medien jeweils eine oder mehrere Verbindungen der Formel I ausgewählt aus der Gruppe der Verbindungen der Formeln I-1 bis I-9, bevorzugt ausgewählt aus der Gruppe der Verbindungen der Formeln I-1 bis I-4,

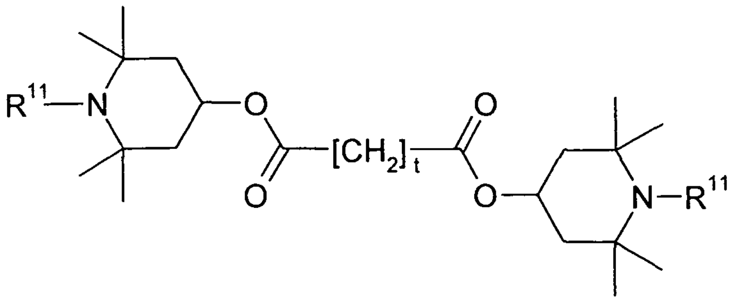

I-1

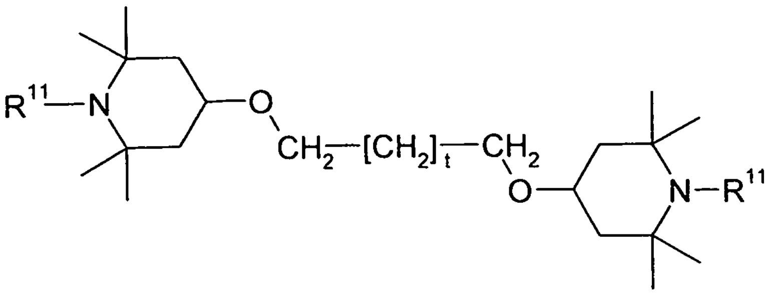

I-2

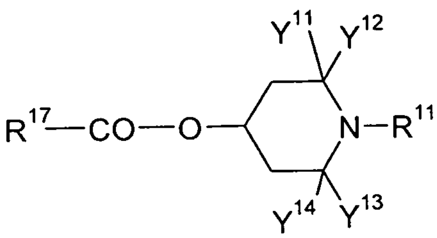

I-3

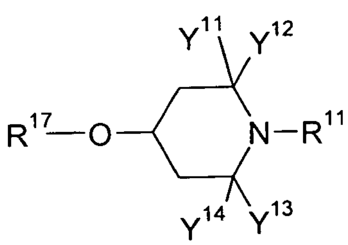

I-4

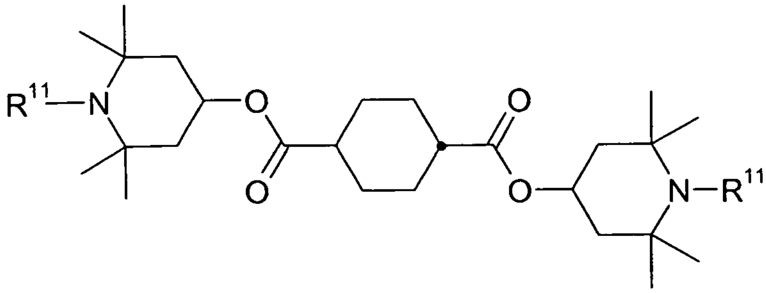

I-5

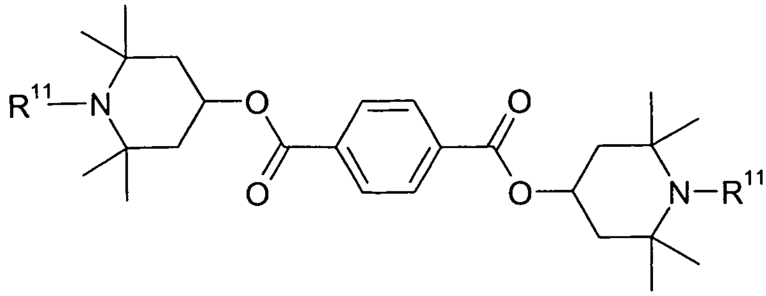

I-6

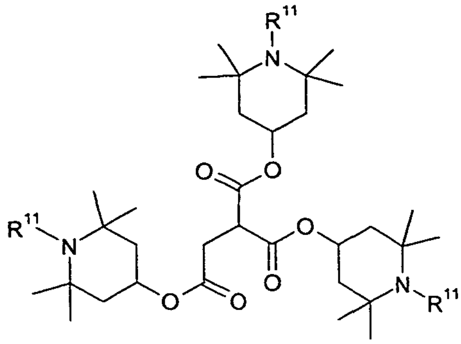

I-7

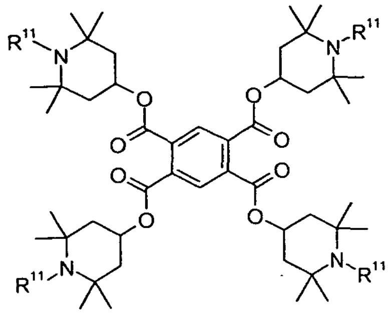

I-8

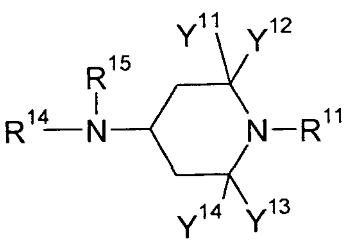

I-9

worin die Parameter die oben unter Formel I angegebenen Bedeutungen haben und

t eine ganze Zahl von 1 bis12

$R^{17}$ eine geradkettige oder verzweigte Alkylkette mit 1-12 C-Atomen in der eine $-CH_2-$ -Gruppe oder mehrere $-CH_2-$-Gruppen durch -O- oder -C(=O)- ersetzt sein können, jedoch nicht zwei benachbarte $-CH_2-$ -Gruppen durch -O- ersetzt sein können oder ein aromatischer oder heteroaromatischer Kohlenwasserstoffrest in denen ein H-Atom oder mehrere H-Atome durch $OR^{14}$, $N(R^{14})(R^{15})$ oder $R^{16}$ ersetzt sein können,

bedeuten.

In einer noch stärker bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-1a-1 bis I-8a-1

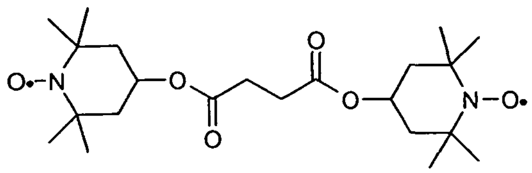

I-1a-1

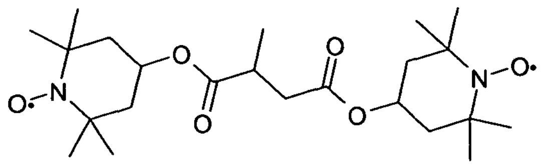

I-1a-2

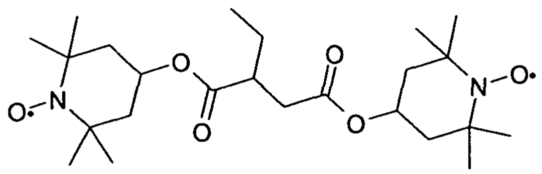

I-1a-3

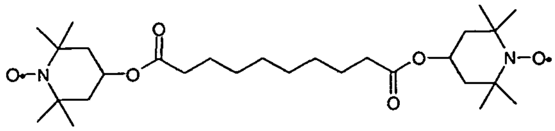

I-1a-4

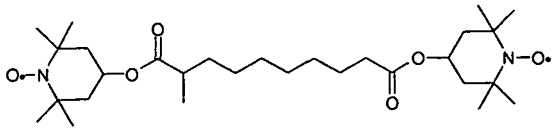

I-1a-5

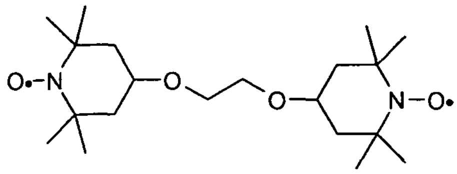

I-2a-1

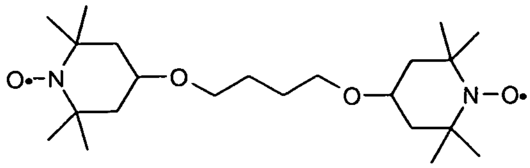

I-2a-2

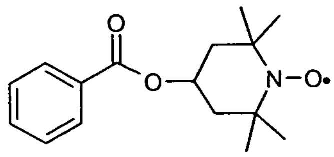

I-3a-1

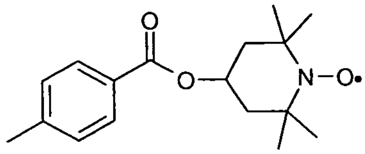

I-3a-2

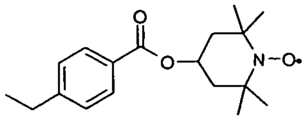

I-3a-3

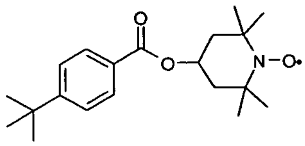

I-3a-4

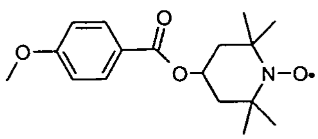

I-3a-5

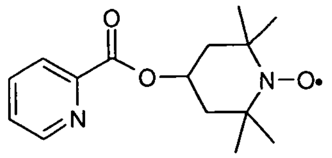

I-3a-6

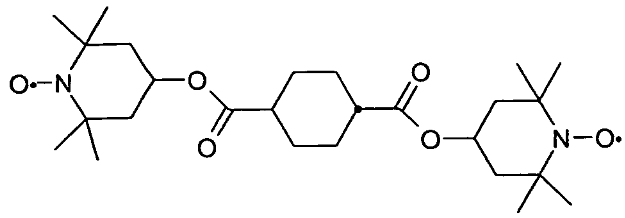

I-5a-1

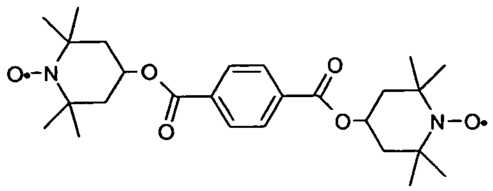

I-6a-1

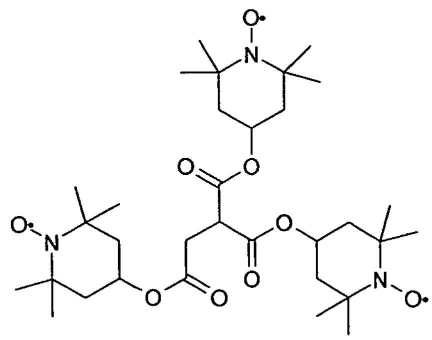

I-7a-1

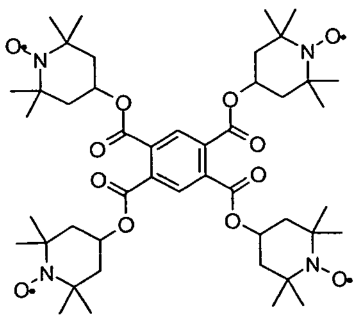

I-8a-1

In einer noch stärker bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-2a-1 und I-2a-2

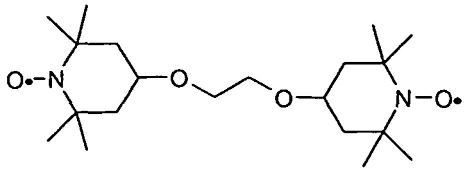

I-2a-1

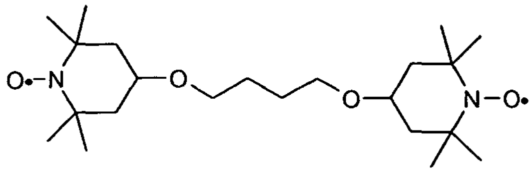

I-2a-2

In einer alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-1b-1 und I-1b-2

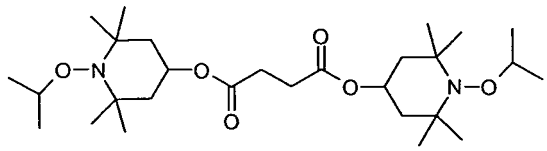

I-1b-1

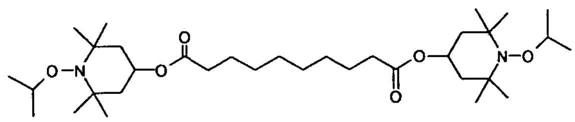

I-1b-2

In einer alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-1c-1 und I-1c-2

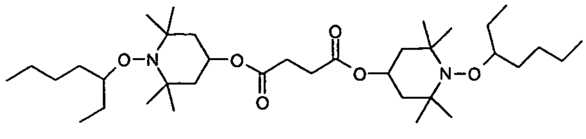

I-1c-1

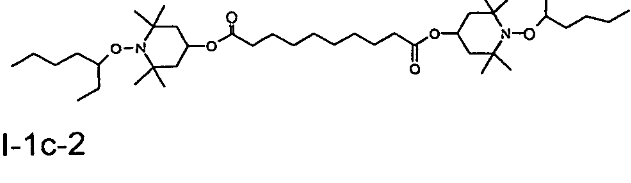

I-1c-2

In einer weiteren alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-1d-1 bis I-1d-4

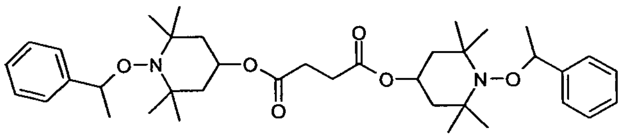

I-1d-1

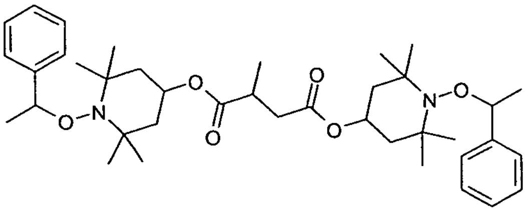

I-1d-2

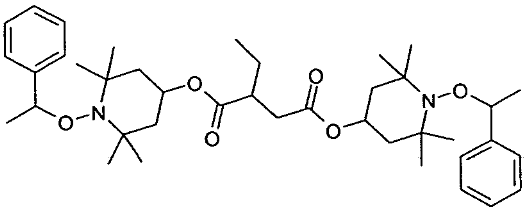

I-1d-3

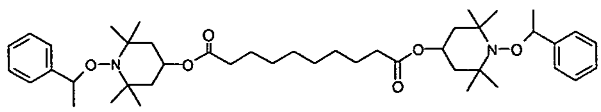

I-1d-4

In einer weiteren alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-3d-1 bis I-3d-8

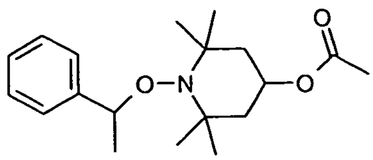

I-3d-1

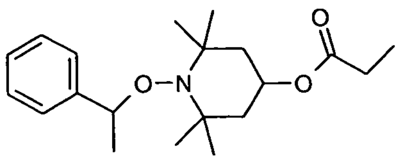

I-3d-2

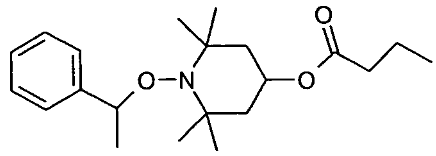

I-3d-3

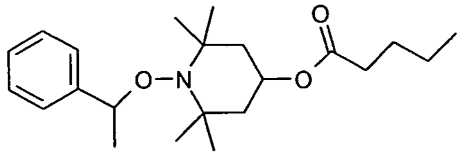

I-3d-4

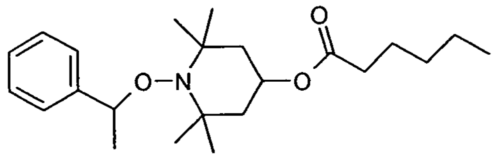

I-3d-5

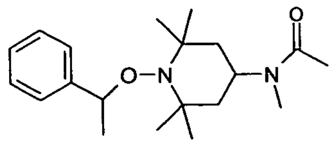

I-3d-6

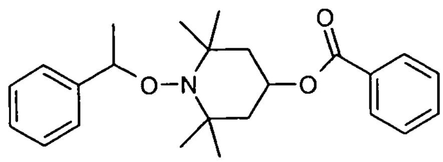

I-3d-7

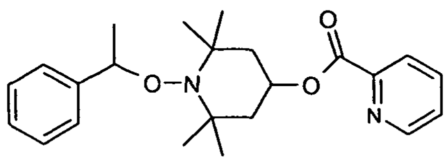

I-3d-8

In einer weiteren alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-4d-1 und I-4d-2

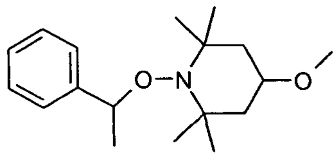

I-4d-1

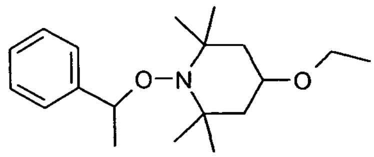

I-4d-2

In einer weiteren alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-1e-1 und I-1e-2

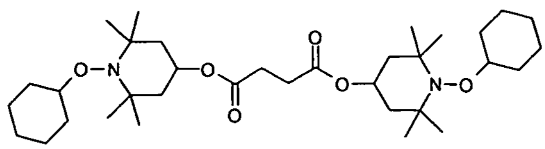

I-1e-1

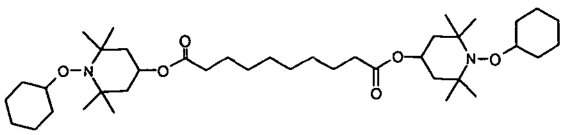

I-1e-2

In einer weiteren alternativen, bevorzugten Ausführungsform der vorliegenden Erfindung enthalten die erfindungsgemäßen Medien jeweils eine oder mehrere Verbindungen der Formel I ausgewählt aus der Gruppe der folgenden Verbindungen, der Formeln I-5e-1 bis I-8e-1

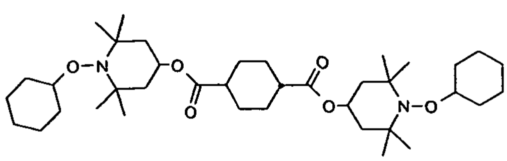

I-5e-1

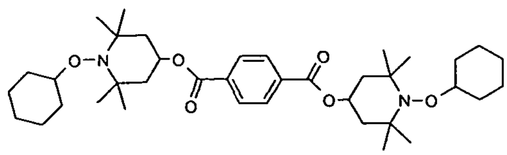

I-6e-1

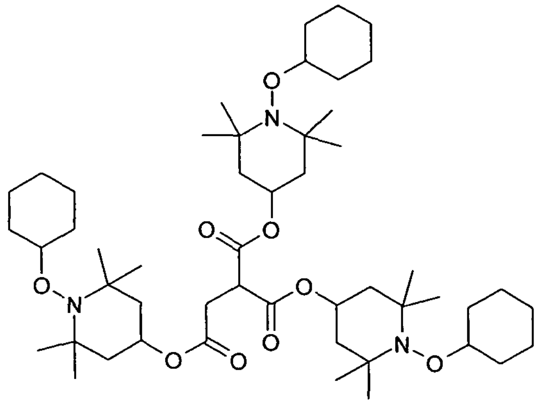

I-7e-1

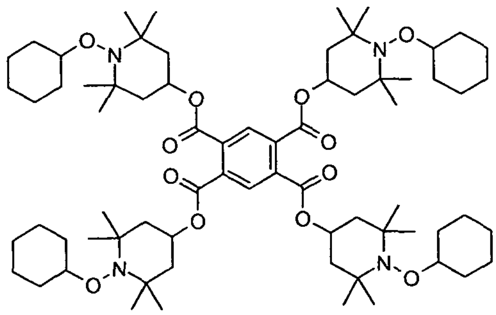

I-8e-1

Vorzugsweise enthalten die Medien gemäß der vorliegenden Erfindung zusätzlich zu den Verbindungen der Formel I, bzw. deren bevorzugter Unterformeln, eine oder mehrere dielektrisch neutrale Verbindungen der Formel II in einer Gesamtkonzentration im Bereich von 5 % oder mehr bis 90 % oder weniger, bevorzugt von 10 % oder mehr bis 80 % oder weniger, besonders bevorzugt von 20 % oder mehr bis 70 % oder weniger.

In einer bevorzugten Ausführungsform der vorliegenden Erfindung enthalten die erfindungsgemäßen Medien jeweils eine oder mehrere Verbindungen der Formel II ausgewählt aus der Gruppe der Verbindungen der Formeln II-1 bis II-4

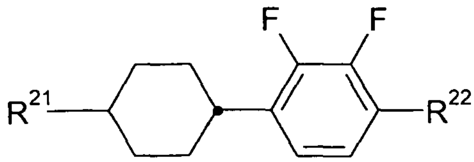

II-1

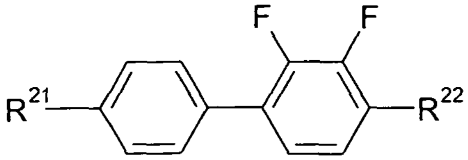

II-2

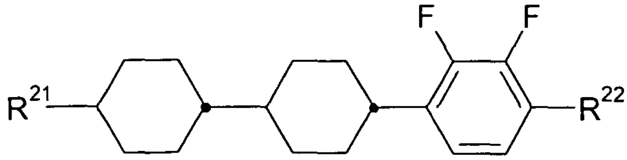

II-3

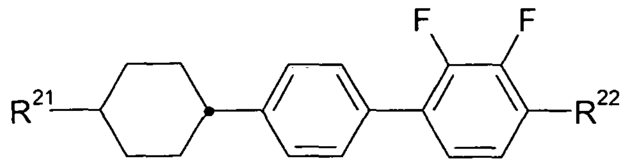

II-4

worin die Parameter die jeweiligen oben bei Formel II gegebenen Bedeutungen haben, und bevorzugt

$R^{21}$ Vinyl, 1-*E*-Propenyl, But-4-en-1-yl, Pent-1-en-1-yl oder Pent-3-en-1-yl und

$R^{22}$ bevorzugt einen unsubstituierten Alkylrest mit 1 bis 7 C-Atomen, bevorzugt mit 2 bis 5 C-Atomen oder, bevorzugt, einen unsubstituierten Alkoxyrest mit 1 bis 6 C-Atomen, besonders bevorzugt mit 2 oder 4 C-Atomen,

bedeuten.

In einer bevorzugten Ausführungsform der vorliegenden Erfindung enthalten die erfindungsgemäßen Medien jeweils eine oder mehrere Verbindungen der Formel II-1 ausgewählt aus der Gruppe der folgenden Verbindungen

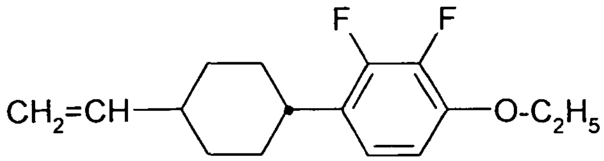

II-1a

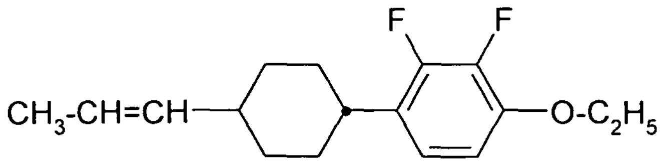

II-1b

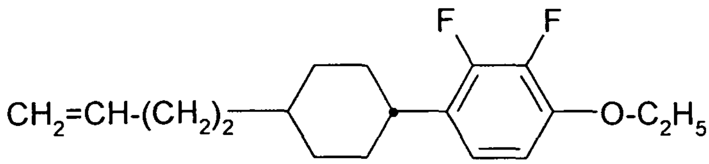

II-1c

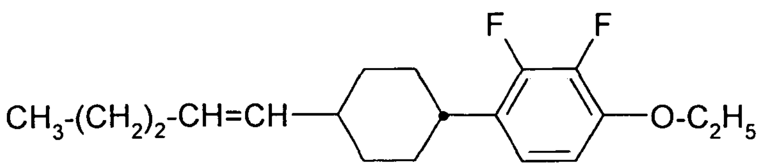

II-1d

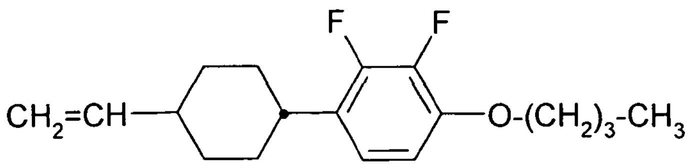

II-1e

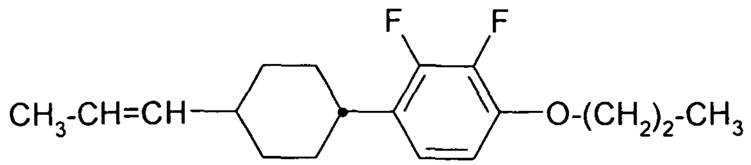

II-1f

In einer bevorzugten Ausführungsform der vorliegenden Erfindung enthalten die erfindungsgemäßen Medien jeweils eine oder mehrere Verbindungen der Formel II-2 ausgewählt aus der Gruppe der folgenden Verbindungen

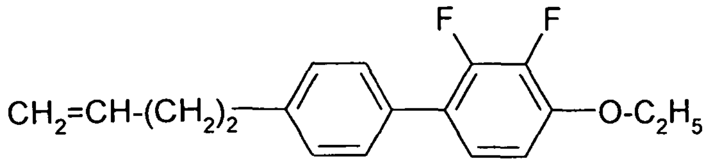

II-2a

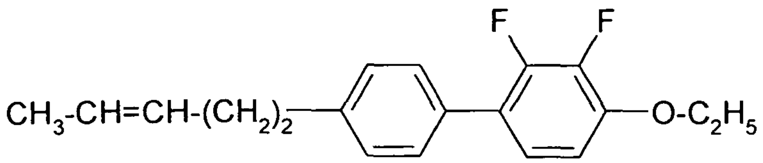

II-2b

In einer bevorzugten Ausführungsform der vorliegenden Erfindung enthalten die erfindungsgemäßen Medien jeweils eine oder mehrere Verbindungen der Formel II-3 ausgewählt aus der Gruppe der folgenden Verbindungen

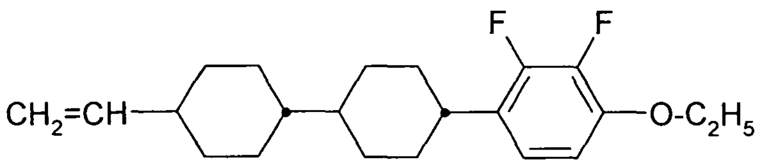

II-3a

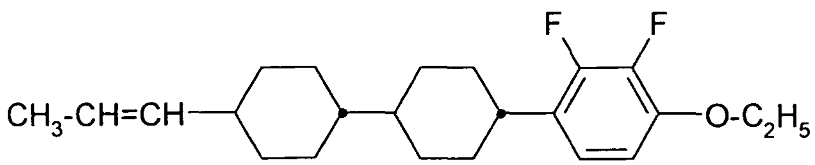

II-3b

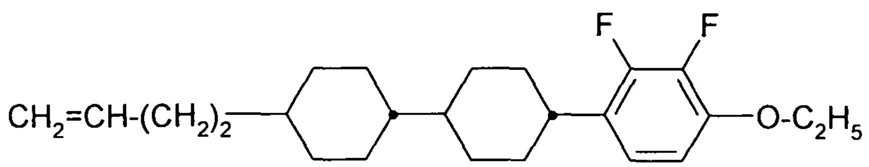

II-3c

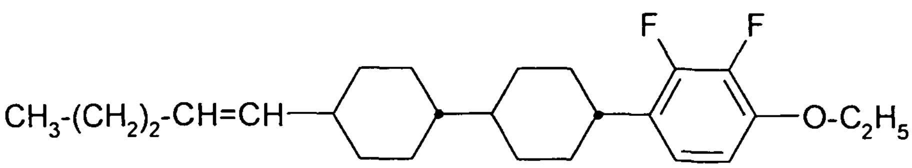

II-3d

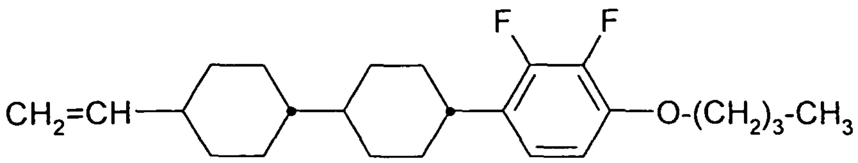

II-3e

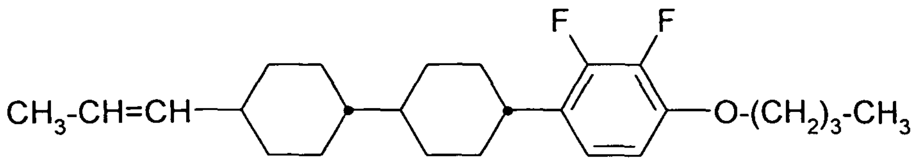

II-3f

In einer bevorzugten Ausführungsform der vorliegenden Erfindung enthalten die erfindungsgemäßen Medien jeweils eine oder mehrere Verbindungen der Formel II-4 ausgewählt aus der Gruppe der folgenden Verbindungen

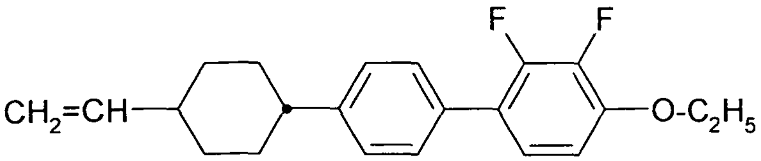

II-4a

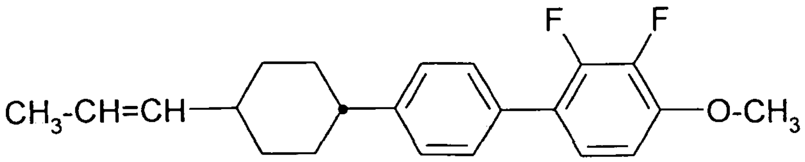

II-4b

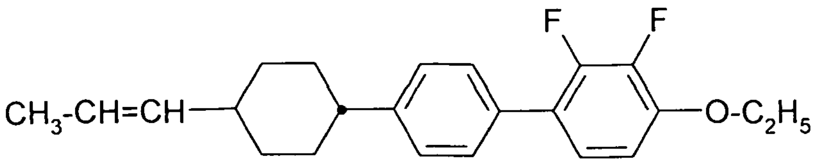

II-4c

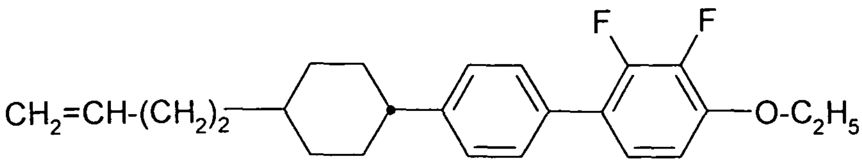

II-4d

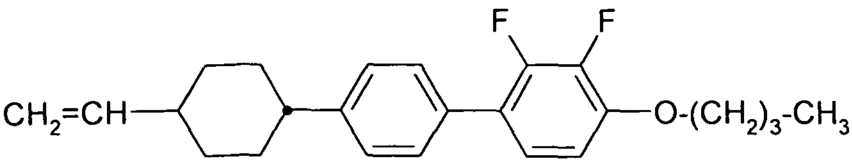

II-4e

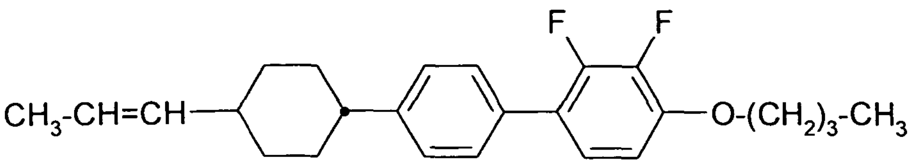

II-4f

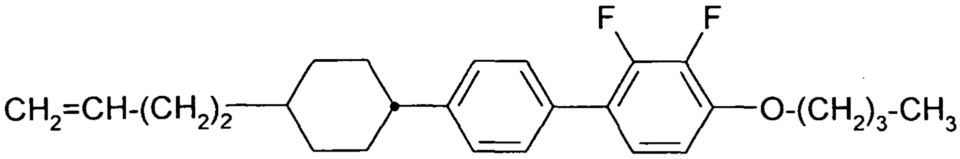

II-4g

Vorzugsweise enthält das erfindungsgemäße Medium eine oder mehrere Verbindungen ausgewählt aus der Gruppe der Formeln III-1 bis III-4 in einer Gesamtkonzentration im Bereich von 10 % oder mehr bis 80 % oder weniger, bevorzugt von 15 % oder mehr bis 70 % oder weniger, besonders bevorzugt von 20 % oder mehr bis 60 % oder weniger.

Insbesondere bevorzugt enthält das erfindungsgemäße Medium

eine oder mehrere Verbindungen der Formel II in einer Gesamtkonzentration im Bereich von 5 % oder mehr bis 50 % oder weniger, bevorzugt im Bereich von 10 % oder mehr bis 40 % oder weniger, und/oder

eine oder mehrere Verbindungen der Formel III-1 in einer Gesamtkonzentration im Bereich von 5 % oder mehr bis 30 % oder weniger und/oder

eine oder mehrere Verbindungen der Formel III-2 in einer Gesamtkonzentration im Bereich von 3 % oder mehr bis 30 % oder weniger und/oder

eine oder mehrere Verbindungen der Formel III-3 in einer Gesamtkonzentration im Bereich von 5 % oder mehr bis 30 % oder weniger und/oder

eine oder mehrere Verbindungen der Formel III-4 in einer Gesamtkonzentration im Bereich von 1 % oder mehr bis 30 % oder weniger.

In einer bevorzugten Ausführungsform der vorliegenden Erfindung enthalten die erfindungsgemäßen Medien eine oder mehrere Verbindungen der Formel III-1, bevorzugt eine oder mehrere Verbindungen ausgewählt aus der Gruppe der Verbindungen der Formeln III-1-1 und III-1-2

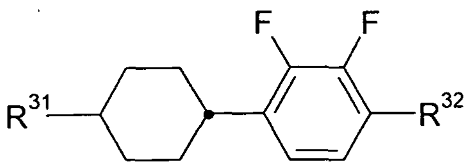

III-1-1

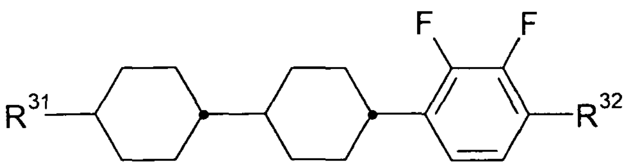

III-1-2

worin die Parameter die oben bei Formel III-1 gegeben Bedeutung haben und bevorzugt

$R^{31}$ einen Alkylrest mit 2 bis 5 C-Atomen, bevorzugt mit 3 bis 5 C-Atomen, und

$R^{32}$ einen Alkyl- oder Alkoxyrest mit 2 bis 5 C-Atomen, bevorzugt einen Alkoxyrest mit 2 bis 4 C-Atomen, oder ein Alkenyloxyrest mit 2 bis 6, bevorzugt bis 4 C-Atomen.

bedeuten.

In einer bevorzugten Ausführungsform der vorliegenden Erfindung enthalten die erfindungsgemäßen Medien eine oder mehrere Verbindungen der Formel III-2, bevorzugt eine oder mehrere Verbindungen ausgewählt aus der Gruppe der Verbindungen der Formeln III-2-1 und III-2-2

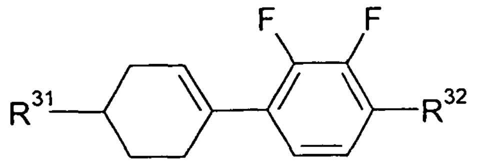

III-2-1

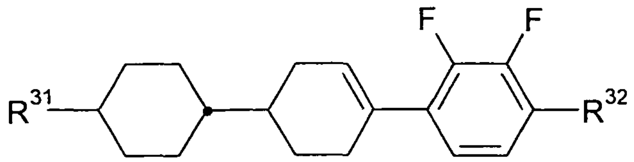

III-2-2

worin die Parameter die oben bei Formel III-2 gegeben Bedeutung haben und bevorzugt

$R^{31}$ einen Alkylrest mit 2 bis 5 C-Atomen, bevorzugt mit 3 bis 5 C-Atomen, und

$R^{32}$ einen Alkyl- oder Alkoxyrest mit 2 bis 5 C-Atomen, bevorzugt einen Alkoxyrest mit 2 bis 4 C-Atomen oder ein Alkenyloxyrest mit 2 bis6, bevorzugt bis 4 C-Atomen,

bedeuten.

In einer besonders bevorzugten Ausführungsform der vorliegenden Erfindung enthalten die erfindungsgemäßen Medien eine oder mehrere Verbindungen der Formel III-3, bevorzugt eine oder mehrere Verbindungen ausgewählt aus der Gruppe der Verbindungen der Formeln III-3-1 und III-3-2, ganz besonders bevorzugt der Formel III-3-2,

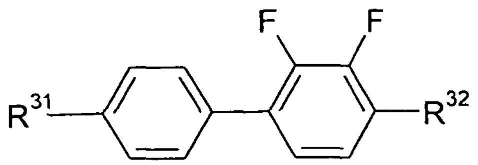

III-3-1

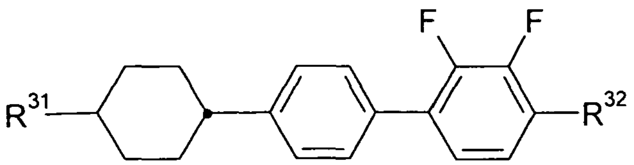

III-3-2

worin die Parameter die oben bei Formel III-3 gegeben Bedeutung haben und bevorzugt

$R^{31}$ einen Alkylrest mit 2 bis 5 C-Atomen, bevorzugt mit 3 bis 5 C-Atomen, und

$R^{32}$ einen Alkyl- oder Alkoxyrest mit 2 bis 5 C-Atomen, bevorzugt ein Alkoxyrest mit 2 bis 4 C-Atomen oder ein Alkenyloxyrest mit 2 bis 6, bevorzugt bis 4 C-Atomen,

bedeuten.

In einer weiteren bevorzugten Ausführungsform enthält das Medium eine oder mehrere Verbindungen der Formel III-4, bevorzugt der Formel III-4-a,

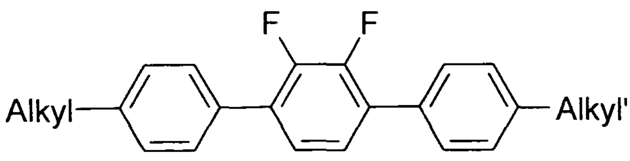

III-4-a

worin

Alkyl und Alkyl’ unabhängig voneinander Alkyl mit 1 bis 7 C-Atomen, bevorzugt mit 2 bis 5 C-Atomen,

bedeuten.

In einer weiteren bevorzugten Ausführungsform enthält das Medium eine oder mehrere Verbindungen der Formel IV

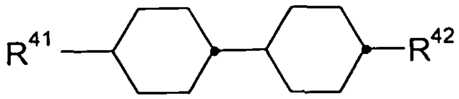

IV

worin

$R^{41}$ einen unsubstituierten Alkylrest mit 1 bis 7 C-Atomen oder einen unsubstituierten Alkenylrest mit 2 bis 7 C-Atomen, bevorzugt einen n-Alkylrest, besonders bevorzugt mit 2, 3, 4 oder 5 C-Atomen, und

$R^{42}$ einen unsubstituierten Alkylrest mit 1 bis 7 C-Atomen oder einen unsubstituierten Alkoxyrest mit 1 bis 6 C-Atomen, beide bevorzugt mit 2 bis 5 C-Atomen, einen unsubstituierten Alkenylrest mit 2 bis 7 C-Atomen, bevorzugt mit 2, 3 oder 4 C-Atomen, stärker bevorzugt einen Vinylrest oder einen 1-Propenylrest und insbesondere einen Vinylrest

bedeuten.

In einer besonders bevorzugten Ausführungsform enthält das Medium eine oder mehrere Verbindungen der Formel IV, ausgewählt aus der Gruppe der Verbindungen der Formeln IV-1 bis IV-4, bevorzugt ausgewählt aus der Gruppe der Verbindungen der Formeln IV-1 und IV-2,

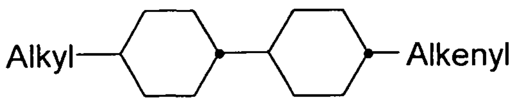

IV-1

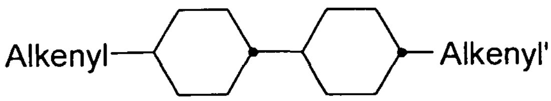

IV-2

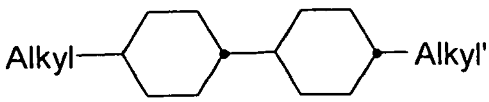

IV-3

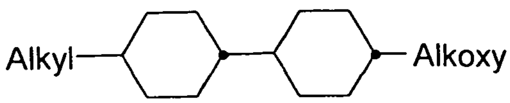

IV-4

worin

Alkyl und Alkyl’ unabhängig voneinander Alkyl mit 1 bis 7 C-Atomen, bevorzugt mit 2 bis 5 C-Atomen,

Alkenyl einen Alkenylrest mit 2 bis 5 C-Atomen, bevorzugt mit 2 bis 4 C-Atomen, besonders bevorzugt 2 C-Atomen,

Alkenyl’ einen Alkenylrest mit 2 bis 5 C-Atomen, bevorzugt mit 2 bis 4 C-Atomen, besonders bevorzugt mit 2 bis 3 C-Atomen, und

Alkoxy Alkoxy mit 1 bis 5 C-Atomen, bevorzugt mit 2 bis 4 C-Atomen,

bedeuten.

In einer besonders bevorzugten Ausführungsform enthalten die erfindungsgemäßen Medien enthalten eine oder mehrere Verbindungen der Formel IV-1 und/oder eine oder mehrere Verbindungen der Formel IV-2.

In einer weiteren bevorzugten Ausführungsform enthält das Medium eine oder mehrere Verbindungen der Formel V

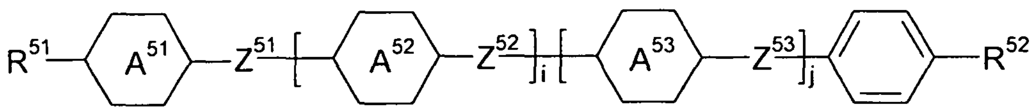

V

worin

$R^{51}$ und $R^{52}$ unabhängig voneinander eine der für $R^{21}$ und $R^{22}$ gegebenen Bedeutung haben und bevorzugt Alkyl mit 1 bis 7 C-Atomen, bevorzugt *n*-Alkyl, besonders bevorzugt n-Alkyl mit 1 bis 5 C-Atomen, Alkoxy mit 1 bis 7 C-Atomen, bevorzugt *n*-Alkoxy, besonders bevorzugt *n*-Alkoxy mit 2 bis 5 C-Atomen, Alkoxyalkyl, Alkenyl oder Alkenyloxy mit 2 bis 7 C-Atomen, bevorzugt mit 2 bis 4 C-Atomen, bevorzugt Alkenyloxy,

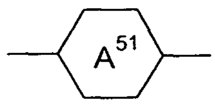

bis

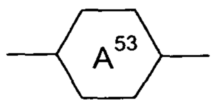

,

soweit vorhanden, jeweils unabhängig voneinander

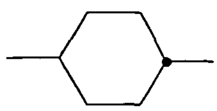

,

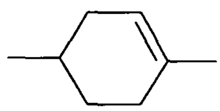

,

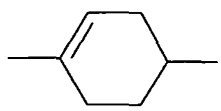

,

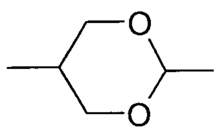

,

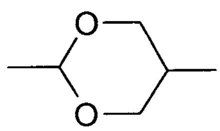

,

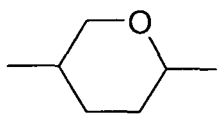

,

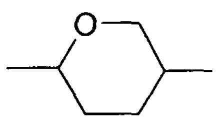

,

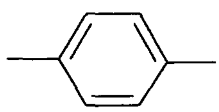

,

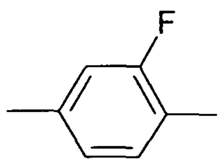

,

oder

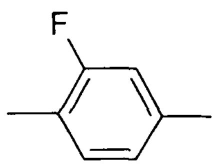

,

bevorzugt

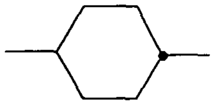 , 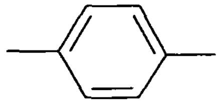 , 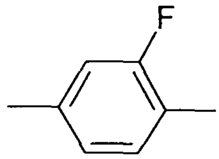 ,

oder

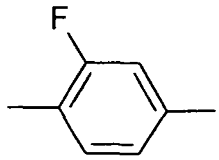 ,

bevorzugt

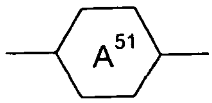 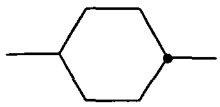 ,

und, wenn vorhanden,

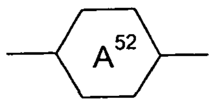

bevorzugt

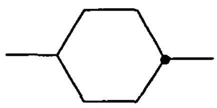 ,

| | |
|---|---|
| $Z^{51}$ bis $Z^{53}$ | jeweils unabhängig voneinander $-CH_2-CH_2-$, $-CH_2-O-$, -CH=CH-, -C≡C-, -COO- oder eine Einfachbindung, bevorzugt $-CH_2-CH_2-$, $-CH_2-O-$ oder eine Einfachbindung und besonders bevorzugt eine Einfachbindung, |
| i und j | jeweils unabhängig voneinander 0 oder 1, |
| (i + j) | bevorzugt 0 oder 1, |

bedeuten.

Bevorzugt enthalten die erfindungsgemäßen Medien die folgenden Verbindungen in den angegebenen Gesamtkonzentrationen

| | |
|---|---|
| 5 - 60 | Gew.-% einer oder mehrerer Verbindungen ausgewählt aus der Gruppe der Verbindungen der Formel II und/oder |
| 10 - 60 | Gew.-% einer oder mehrerer Verbindungen ausgewählt aus der Gruppe der Verbindungen der Formeln III-1 bis III-4 und/oder |
| 10 - 60 | Gew.-% einer oder mehrerer Verbindungen der Formeln IV und/oder V, |

wobei der Gesamtgehalt aller Verbindungen in dem Medium 100 % beträgt.

In einer besonders bevorzugten Ausführungsform enthalten die erfindungsgemäßen Medien eine oder mehrere Verbindungen ausgewählt aus der Gruppe der Verbindungen der Formeln OH-1 bis OH-6

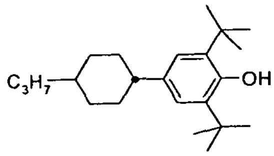

OH-1

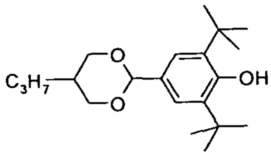

OH-2

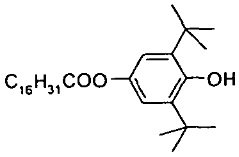

OH-3

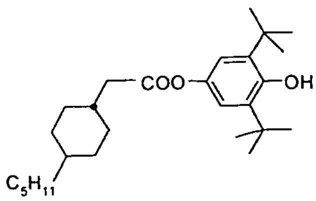

OH-4

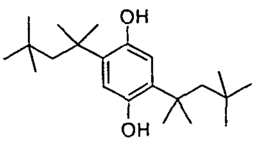

OH-5

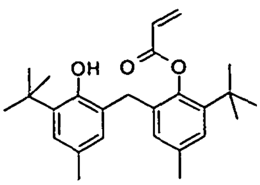

OH-6

Diese Verbindungen eignen sich hervorragend zur Stabilisierung der Medien gegen thermische Belastungen.

In einer anderen bevorzugten Ausführungsform der vorliegenden Erfindung, in der die erfindungsgemäßen Medien insbesondere eine oder mehrere Verbindungen Formel I enthalten, in denen $R^{11}$, bzw. mindestens einer von $R^{11}$, $O^{\bullet}$ bedeutet, können diese Medien auch ausreichende Stabilität aufweisen, wenn sie keine Phenolverbindung, insbesondere ausgewählt aus der Gruppe der Verbindungen der Formeln OH-1 bis OH-6, enthalten.

In einer weiteren bevorzugten Ausführungsform der vorliegenden Erfindung enthalten die erfindungsgemäßen Medien mindestens jeweils eine oder mehrere Verbindungen der Formel I bei denen die Gruppen $R^{11}$ der einen Verbindungen der Formel I eine andere Bedeutung haben als bei den anderen Verbindungen der Formel I.

Gegenstand der vorliegenden Erfindung sind auch elektrooptische Anzeigen oder elektrooptische Komponenten, die erfindungsgemäße flüssigkristalline Medien enthalten. Bevorzugt sind elektrooptische Anzeigen die auf dem VA- oder dem ECB-Effekt basieren und insbesondere solche, die mittels einer Aktivmatrix-Adressierungsvorrichtung angesteuert werden.

Dementsprechend ist die Verwendung eines erfindungsgemäßen flüssigkristallinen Mediums in einer elektrooptischen Anzeige oder in einer elektrooptischen Komponente ebenfalls Gegenstand der vorliegenden Erfindung, ebenso wie ein Verfahren zur Herstellung der erfindungsgemäßen flüssigkristallinen Medien, dadurch gekennzeichnet, dass eine oder mehrere Verbindungen der Formel I mit einer oder mehreren Verbindungen, die eine oder mehrere Verbindungen der Formel II enthalten, bevorzugt mit eine oder mehrere Verbindungen der Teilformel II-1 und/oder II-2 und/oder II-3 und/oder II-4, besonders bevorzugt eine oder mehrere Verbindungen aus zwei oder mehr, bevorzugt aus drei oder mehr, verschiedener dieser Formel und ganz besonders bevorzugt aus allen vier dieser Formeln II-1, II-2, II-3 und II-4, mit einer oder mehreren weiteren Verbindungen, bevorzugt ausgewählt aus der Gruppe der Verbindungen der Formeln III-1 bis III-4 und IV und/oder V, gemischt wird.

In einer weiteren bevorzugten Ausführungsform enthält das Medium eine oder mehrere Verbindungen der Formel IV, ausgewählt aus der Gruppe der Verbindungen der Formeln IV-3 und IV-4,

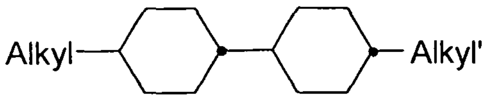
IV-3

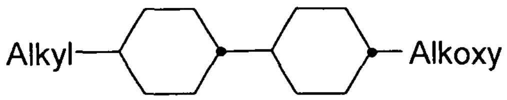
IV-4

worin

| | |
|---|---|
| Alkyl und Alkyl' | unabhängig voneinander Alkyl mit 1 bis 7 C-Atomen, bevorzugt mit 2 bis 5 C-Atomen |
| Alkoxy | Alkoxy mit 1 bis 5 C-Atomen, bevorzugt mit 2 bis 4 C-Atomen. |

In einer weiteren bevorzugten Ausführungsform enthält das Medium eine oder mehrere Verbindungen ausgewählt der Formel V ausgewählt aus der Gruppe der Verbindungen der Formeln V-1 bis V-7 und VI-1 bis VI-3, bevorzugt ausgewählt aus der Gruppe der Verbindungen der Formeln V-1 bis V-5,

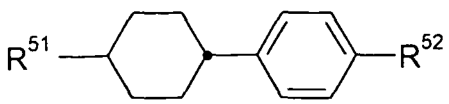
V-1

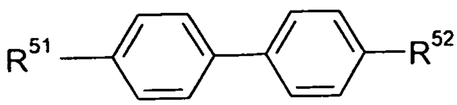
V-2

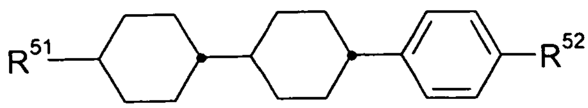
V-3

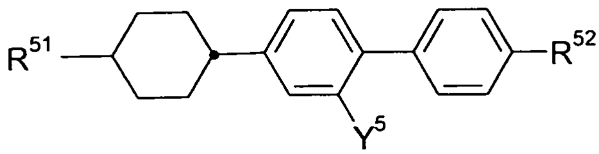
V-4

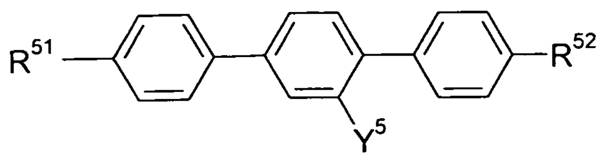
V-5

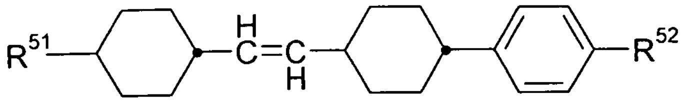
V-6

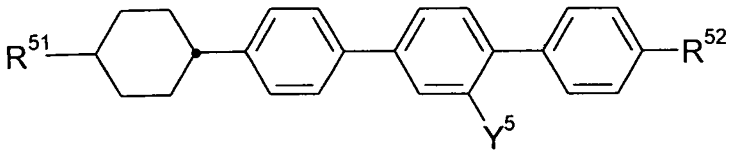
V-7

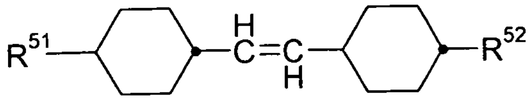
VI-1

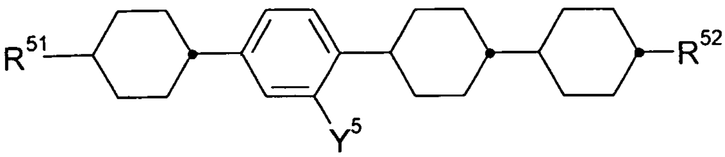
V1-2

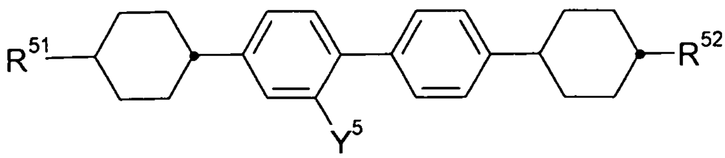
VI-3

worin die Parameter die oben unter Formel V gegebenen Bedeutungen haben und

$Y^5$ H oder F bedeutet und bevorzugt

$R^{51}$ Alkyl mit 1 bis 7 C-Atomen oder Alkenyl mit 2 bis 7 C-Atomen und

$R^{52}$ Alkyl mit 1 bis 7 C-Atomen, Alkenyl mit 2 bis 7 C-Atomen oder Alkoxy mit 1 bis 6 C-Atomen, bevorzugt Alkyl oder Alkenyl, besonders bevorzugt Alkenyl,

bedeuten.

In einer weiteren bevorzugten Ausführungsform enthält das Medium eine oder mehrere Verbindungen der Formel V-1, ausgewählt aus der Gruppe der Verbindungen der Formeln V-1a und V-1b, bevorzugt der Formel V-1b,

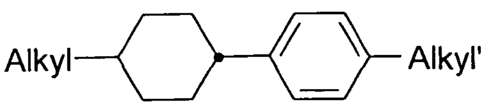
V-1a

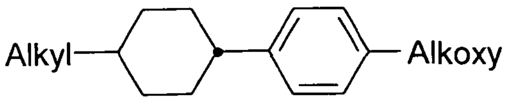
V-1b

worin

Alkyl und Alkyl' unabhängig voneinander Alkyl mit 1 bis 7 C-Atomen, bevorzugt mit 2 bis 5 C-Atomen,

Alkoxy Alkoxy mit 1 bis 5 C-Atomen, bevorzugt mit 2 bis 4 C-Atomen.

In einer weiteren bevorzugten Ausführungsform enthält das Medium eine oder mehrere Verbindungen der

Formel V-3, ausgewählt aus der Gruppe der Verbindungen der Formeln V-3a und V-3b,

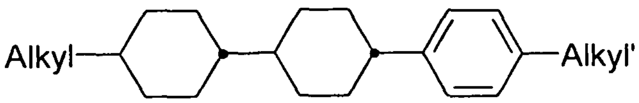

V-3a

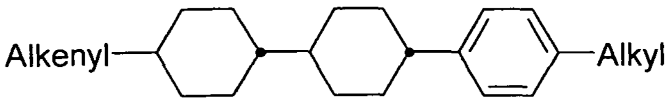

V-3b

worin

Alkyl und Alkyl' unabhängig voneinander Alkyl mit 1 bis7 C-Atomen, bevorzugt mit 2 bis 5 C-Atomen und

Alkenyl Alkenyl mit 2 bis 7 C-Atomen, bevorzugt mit 2 bis 5 C-Atomen bedeuten.

In einer weiteren bevorzugten Ausführungsform enthält das Medium eine oder mehrere Verbindungen der Formel V-4 ausgewählt aus der Gruppe der Verbindungen der Formeln V-4a und V-4b,

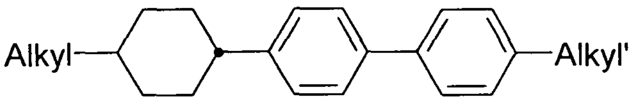

V-4a

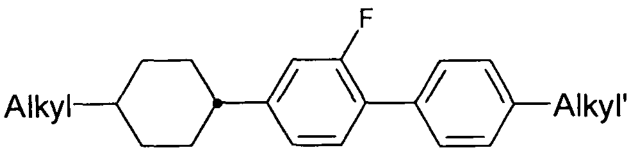

V-4b

worin

Alkyl und Alkyl' unabhängig voneinander Alkyl mit 1 bis 7 C-Atomen, bevorzugt mit 2 bis 5 C-Atomen.

Außerdem betrifft die vorliegenden Erfindung ein Verfahren zur Stabilisierung eines flüssigkristallinen Mediums, das eine oder mehrere Verbindungen der Formel II, gegebenenfalls eine oder mehrere Verbindungen ausgewählt aus der Gruppe der Verbindungen der Formeln III-1 bis III-4 enthält und/oder eine oder mehrere Verbindungen der Formel IV und/oder eine oder mehrere Verbindungen der Formel V, dadurch gekennzeichnet, dass dem Medium eine oder mehrere Verbindungen der Formel I zugesetzt werden.

Die Flüssigkristallmedien gemäß der vorliegenden Erfindung können eine oder mehrere chirale Verbindungen enthalten.

In einer besonders bevorzugten Ausführungsformen der vorliegenden Erfindung enthalten die flüssigkristallinen Medien eine oder mehrere Verbindungen der Formel

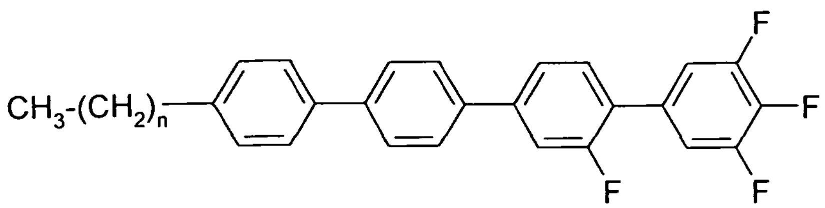

worin n 0, 1, 2, 3, 4, 5 oder6, bevorzugt 2 oder 4, besonders bevorzugt 2, bedeutet, bevorzugt in einer Konzentration vom 0,1 bis 5 % besonders bevorzugt vom 0,2 bis 1 %.

Besonders bevorzugte Ausführungsformen der vorliegenden Erfindung erfüllen eine oder mehrere der folgenden Bedingungen,

wobei die Akronyme (Abkürzungen) in den Tabellen A bis C erläutert und in Tabelle D durch Beispiele illustriert sind.

i. Das flüssigkristalline Medium hat eine Doppelbrechung von 0,060 oder mehr, besonders bevorzugt von 0,070 oder mehr.

ii. Das flüssigkristalline Medium hat eine Doppelbrechung von 0,130 oder weniger, besonders bevorzugt von 0,120 oder weniger.

iii. Das flüssigkristalline Medium hat eine Doppelbrechung im Bereich von 0,090 oder mehr bis 0,120 oder weniger.

iv. Das flüssigkristalline Medium hat eine negative dielektrische Anisotropie mit einem Betrag von 2,0 oder mehr, besonders bevorzugt von 3,0 oder mehr.

v. Das flüssigkristalline Medium hat eine negative dielektrische Anisotropie mit einem Betrag von 5,5 oder weniger, besonders bevorzugt von 4.0 oder weniger.

vi. Das flüssigkristalline Medium hat eine negative dielektrische Anisotropie mit einem Betrag im Bereich von 2,5 oder mehr bis 3,8 oder weniger.

vii. Das flüssigkristalline Medium enthält eine oder mehrere besonders bevorzugte Verbindungen der Formeln IV ausgewählt aus den nachfolgend genannten Teilformeln:

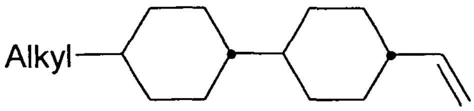

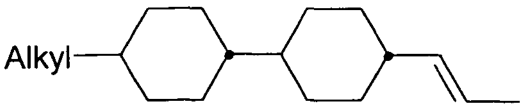

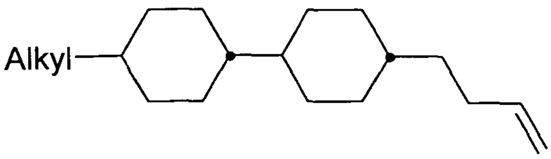

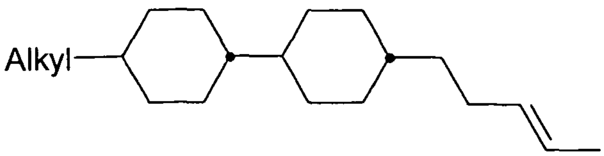

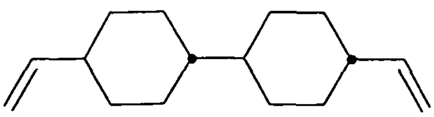

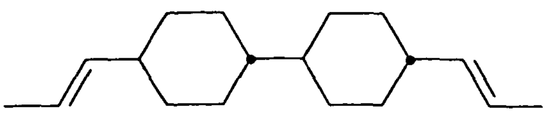

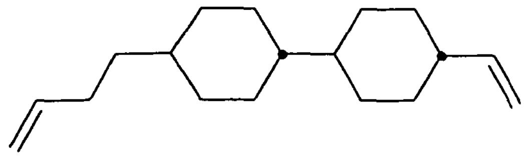

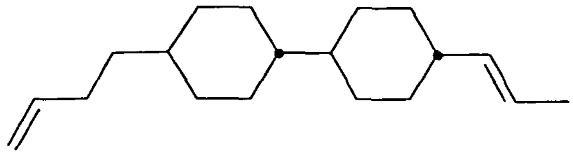

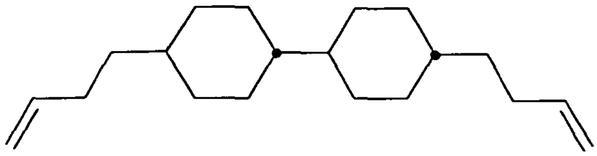

worin Alkyl die oben gegebene Bedeutung besitzt und bevorzugt, jeweils unabhängig voneinander Alkyl mit 1 bis 6, bevorzugt mit 2 bis 5 C-Atomen und besonders bevorzugt *n*-Alkyl, bedeutet.

viii. Die Gesamtkonzentration der Verbindungen der Formel IV im Gesamtgemisch beträgt 25 % oder mehr, bevorzugt 30 % oder mehr und liegt bevorzugt im Bereich von 25 % oder mehr bis 49 % oder weniger, besonders bevorzugt im Bereich von 29 % oder mehr bis 47 % oder weniger, und ganz besonders bevorzugt im Bereich von 37 % oder mehr bis 44 % oder weniger.

ix. Das flüssigkristalline Medium enthält eine oder mehrere Verbindungen der Formel IV ausgewählt aus der Gruppe der Verbindungen folgenden Formeln: CC-n-V und/oder CC-n-Vm, insbesondere bevorzugt CC-3-V, bevorzugt in einer Konzentration von bis zu 50 % oder weniger, besonders bevorzugt bis zu 42 % oder weniger, und optional zusätzlich CC-3-V1, bevorzugt in einer Konzentration von bis zu 15 % oder weniger, und/oder CC-4-V, bevorzugt in einer Konzentration von bis zu 20 % oder weniger, besonders bevorzugt bis zu 10 % oder weniger.

x. Die Gesamtkonzentration der Verbindungen der Formel CC-3-V im Gesamtgemisch beträgt 20 % oder mehr, bevorzugt 25 % oder mehr.

xi. Der Anteil an Verbindungen der Formeln III-1 bis III-4 im Gesamtgemisch beträgt 50 % oder mehr und bevorzugt 75 % oder weniger.

xii. Das flüssigkristalline Medium besteht im Wesentlichen aus Verbindungen der Formeln I, II, III-1 bis III-4, IV und V, bevorzugt aus Verbindungen der Formeln I, II und III-1 bis III-4.

xiii. Das flüssigkristalline Medium enthält eine oder mehrere Verbindungen der Formel IV, bevorzugt der Formeln IV-1 und/oder IV-2, vorzugsweise in einer Gesamtkonzentration von 20 % oder mehr, insbesondere von 25 % oder mehr, und ganz besonders bevorzugt von 30 % oder mehr bis 45 % oder weniger.

Ein weiterer Gegenstand der Erfindung ist eine elektrooptische Anzeige mit einer Aktivmatrix-Adressierung basierend auf dem VA- bzw. ECB-Effekt, dadurch gekennzeichnet, dass sie als Dielektrikum ein flüssigkristallines Medium gemäß der vorliegenden Erfindung enthält.

Vorzugsweise weist die Flüssigkristallmischung einen nematischen Phasenbereich mit einer Breite von mindestens 80 Grad und eine Fließviskosität $\nu_{20}$ von maximal 30 $mm^2 \cdot s^{-1}$ bei 20 °C auf.

Die erfindungsgemäße Flüssigkristallmischung weist ein $\Delta\varepsilon$ von -0,5 bis -8,0, insbesondere von -1,5 bis -6,0 auf, und ganz besonders bevorzugt von -2,0 bis -5,0 auf, wobei $\Delta\varepsilon$ die dielektrische Anisotropie bedeutet.

Die Rotationsviskosität $\gamma_1$ ist vorzugsweise 120 mPa·s oder weniger, insbesondere 100 mPa·s oder weniger.

Die erfindungsgemäßen Mischungen sind für alle VA-TFT-Anwendungen geeignet, wie z.B. VAN, MVA, (S)-PVA und ASV. Weiterhin sind sie für IPS (In plan switching)-, FFS (Fringe field switching)- und PALC-Anwendungen mit negativem $\Delta\varepsilon$ geeignet.

Die nematischen Flüssigkristallmischungen in den erfindungsgemäßen Anzeigen enthalten in der Regel zwei Komponenten A und B, die ihrerseits aus einer oder mehreren Einzelverbindungen bestehen.

Die erfindungsgemäßen flüssigkristallinen Medien enthalten bevorzugt 4 bis 15, insbesondere 5 bis 12, und besonders bevorzugt 10 oder weniger, Verbindungen. Diese sind vorzugsweise ausgewählt aus der Gruppe der Verbindungen der Formeln I, II und III-1 bis III-4, und/oder IV und/oder V.

Die erfindungsgemäßen flüssigkristallinen Medien können optional auch mehr als 18 Verbindungen enthalten. In diesem Fall enthalten sie vorzugsweise 18 bis 25 Verbindungen.

Neben Verbindungen der Formeln I bis V können auch noch andere Bestandteile zugegen sein, z. B. in einer Menge von bis zu 45 %, vorzugsweise jedoch bis zu 35 %, insbesondere bis zu 10 %, der Gesamtmischung.

Optional können die erfindungsgemäßen Medien auch eine dielektrisch positive Komponente enthalten, deren Gesamtkonzentration bevorzugt 10 % oder weniger bezogen auf das gesamte Medium beträgt.

In einer bevorzugten Ausführungsform enthalten die erfindungsgemäßen Flüssigkristallmedien insgesamt bezogen auf die Gesamtmischung
10 ppm oder mehr bis 1000 ppm oder weniger, bevorzugt 50 ppm oder mehr bis 500 ppm oder weniger, besonders bevorzugt 100 ppm oder mehr bis 400 ppm oder weniger und ganz besonders bevorzugt 150 ppm oder mehr bis 300 ppm oder weniger, der Verbindung der Formel I.

20% oder mehr bis 60 % oder weniger, bevorzugt 25 % oder mehr bis 50 % oder weniger, besonders bevorzugt 30 % oder mehr bis 45 % oder weniger, an Verbindungen der Formel II und
50 % oder mehr bis 70 % oder weniger an Verbindungen der Formeln III-1 bis III-4.

In einer bevorzugten Ausführungsform enthalten die erfindungsgemäßen Flüssigkristallmedien Verbindungen ausgewählt aus der Gruppe der Verbindungen der Formeln I, II, III-1 bis III-4, IV und V, bevorzugt ausgewählt aus der Gruppe der Verbindungen der Formeln I, II und III-1 bis III-4, bevorzugt bestehen sie überwiegend, besonders bevorzugt im wesentlichen und ganz besonders bevorzugt nahezu vollständig aus den Verbindungen der genannten Formeln.

Die erfindungsgemäßen Flüssigkristallmedien weisen bevorzugt eine nematische Phase von jeweils mindestens von -20°C oder weniger bis 70°C oder mehr, besonders bevorzugt von -30°C oder weniger bis 80°C oder mehr, ganz besonders bevorzugt von -40°C oder weniger bis 85°C oder mehr und am allermeisten bevorzugt von -40°C oder weniger bis 90°C oder mehr auf.

Hierbei bedeutet der Begriff "eine nematische Phase aufweisen" einerseits, dass bei tiefen Temperaturen bei der entsprechenden Temperatur keine smektische Phase und keine Kristallisation beobachtet wird und andererseits, dass beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen einer der elektrooptischen Anwendung entsprechenden Schichtdicke für mindestens 100 Stunden überprüft. Wenn die Lagerstabilität bei einer Temperatur von -20°C in einer entsprechenden Testzelle 1.000 h oder mehr beträgt, wird das Medium als bei dieser Temperatur stabil bezeichnet. Bei Temperaturen von -30°C bzw. -40°C betragen die entsprechenden Zeiten 500 h bzw. 250 h. Bei hohen Temperaturen wird der Klärpunkt nach üblichen Methoden in Kapillaren gemessen.

In einer bevorzugten Ausführungsform sind die erfindungsgemäßen Flüssigkristallmedien durch Werte der optischen Anisotropien im mittleren bis niedrigen Bereich gekennzeichnet. Die Werte der Doppelbrechung liegen bevorzugt im Bereich von 0,065 oder mehr bis 0,130 oder weniger, besonders bevorzugt im Bereich von 0,080 oder mehr bis 0,120 oder weniger und ganz besonders bevorzugt im Bereich von 0,085 oder mehr bis 0,110 oder weniger.

In dieser Ausführungsform haben die erfindungsgemäßen Flüssigkristallmedien eine negative dielektrische Anisotropie und weisen relativ hohe Werte des Betrags der dielektrischen Anisotropie ($|\Delta\varepsilon|$) auf, die bevorzugt im Bereich von 2,7 oder mehr bis 5,3 oder weniger, bevorzugt bis 4,5 oder weniger, bevorzugt von 2,9 oder mehr bis 4,5 oder weniger, besonders bevorzugt von 3,0 oder mehr bis 4,0 oder weniger und ganz besonders bevorzugt von 3,5 oder mehr bis 3,9 oder weniger, liegen.

Die erfindungsgemäßen Flüssigkristallmedien weisen relativ kleine Werte für die Schwellenspannung ($V_0$) im Bereich von 1,7 V oder mehr bis 2,5 V oder weniger, bevorzugt von 1,8 V oder mehr bis 2,4 V oder weniger, besonders bevorzugt von 1,9 V oder mehr bis 2,3 V oder weniger und ganz besonders bevorzugt von 1,95 V oder mehr bis 2,1 V oder weniger, auf.

In einer weiteren bevorzugten Ausführungsform weisen die erfindungsgemäßen Flüssigkristallmedien bevorzugt relativ niedrige Werte der mittleren dielektrischen Anisotropie ($\varepsilon_{av.} \equiv (\varepsilon_{\parallel} + 2\varepsilon_{\perp})/3$) auf, die bevorzugt im Bereich von 5,0 oder mehr bis 7,0 oder weniger, bevorzugt von 5,4 oder mehr bis 6,5 oder weniger, noch mehr bevorzugt von 5,5 oder mehr bis 6,3 oder weniger, besonders bevorzugt von 5,6 oder mehr bis 6,1 oder weniger und ganz besonders bevorzugt von 5,7 oder mehr bis 5,9 oder weniger, liegen.

Außerdem weisen die erfindungsgemäßen Flüssigkristallmedien hohe Werte für die VHR in Flüssigkristallzellen auf.

Diese sind in frisch gefüllten Zellen bei 20°C in den Zellen größer oder gleich 95 %, bevorzugt größer oder gleich 97 %, besonders bevorzugt größer oder gleich 98 % und ganz besonders bevorzugt größer oder gleich 99 % und nach 5 Minuten im Ofen bei 100°C in den Zellen größer oder gleich 90 %, bevorzugt größer oder gleich 93 %, besonders bevorzugt größer oder gleich 96 % und ganz besonders bevorzugt größer oder gleich 98%.

In der Regel weisen dabei Flüssigkristallmedien mit einer geringen Ansteuerspannung bzw. Schwellenspannung eine geringere VHR auf als solche mit einer größeren Ansteuerspannung bzw. Schwellenspannung und umgekehrt.

Diese bevorzugten Werte für die einzelnen physikalischen Eigenschaften werden von den erfindungsgemäßen Medien bevorzugt auch jeweils miteinander kombiniert eingehalten.

In der vorliegenden Anmeldung bedeutet der Begriff "Verbindungen", auch geschrieben als "Verbindung(en)", sofern nicht explizit anders angegeben, sowohl eine als auch mehrere Verbindungen.

Die einzelnen Verbindungen werden, sofern nichts anderes angegeben, in den Mischungen in Konzentrationen generell jeweils von 1 % oder mehr bis 30 % oder weniger, bevorzugt von 2 % oder mehr bis 30 % oder weniger und besonders bevorzugt von 3 % oder mehr bis 16 % oder weniger eingesetzt.

In einer bevorzugten Ausführungsform enthalten die erfindungsgemäßen flüssigkristallinen Medien
die Verbindung der Formel I,
eine oder mehrere Verbindungen der Formel IV, bevorzug ausgewählt aus der Gruppe der Verbindungen der Formeln CC-n-V und CC-n-Vm, bevorzugt CC-3-V, CC-3-V1, CC-4-V und CC-5-V, besonders bevorzugt ausgewählt aus der Gruppe der Verbindungen CC-3-V, CC-3-V1 und CC-4-V, ganz besonders bevorzugt der Verbindung CC-3-V und gegebenenfalls zusätzlich der Verbindung CC-4-V und/oder CC-3-V1,
eine oder mehrere Verbindungen der Formel III-1-1, bevorzugt der Formel CY-n-Om, ausgewählt aus der Gruppe der Verbindungen der Formeln CY-3-O2, CY-3-O4, CY-5-O2 und CY-5-O4,
eine oder mehrere Verbindungen der Formel III-1-2, bevorzugt ausgewählt aus der Gruppe der Verbindungen der Formel CCY-n-m und CCY-n-Om, bevorzugt der Formel CCY-n-Om, bevorzugt ausgewählt aus der Gruppe der Verbindungen der Formeln CCY-3-O2, CCY-2-O2, CCY-3-O1, CCY-3-O3, CCY-4-O2, CCY-3-O2 und CCY-5-O2,
optional, bevorzugt obligatorisch, eine oder mehrere Verbindungen der Formel III-2-2, bevorzugt der Formel CLY-n-Om, bevorzugt ausgewählt aus der Gruppe der Verbindungen der Formeln CLY-2-O4, CLY-3-O2, CLY-3-O3,
eine oder mehrere Verbindungen der Formel III-3-2, bevorzugt der Formel CPY-n-Om, bevorzugt ausgewählt aus der Gruppe der Verbindungen der Formeln CPY-2-O2 und CPY-3-O2, CPY-4-O2 und CPY-5-O2,
eine oder mehrere Verbindungen der Formel III-4, bevorzugt der Formel PYP-n-m, bevorzugt ausgewählt aus der Gruppe der Verbindungen der Formeln PYP-2-3 und PYP-2-4.

Für die vorliegende Erfindung bedeutet im Zusammenhang mit der Angabe der Bestandteile der Zusammensetzungen, wenn nicht im Einzelfall anders angegeben:

- "enthalten": die Konzentration der betreffenden Bestandteile in der Zusammensetzung beträgt bevorzugt 5 % oder mehr, besonders bevorzugt 10 % oder mehr, ganz besonders bevorzugt 20 % oder mehr,

- "überwiegend bestehen aus": die Konzentration der betreffenden Bestandteile in der Zusammensetzung beträgt bevorzugt 50 % oder mehr, besonders bevorzugt 55 % oder mehr und ganz besonders bevorzugt 60 % oder mehr,

- "im wesentlichen bestehen aus": die Konzentration der betreffenden Bestandteile in der Zusammensetzung beträgt bevorzugt 80 % oder mehr, besonders bevorzugt 90 % oder mehr und ganz besonders bevorzugt 95 % oder mehr und

- "nahezu vollständig bestehen aus": die Konzentration der betreffenden Bestandteile in der Zusammensetzung beträgt bevorzugt 98 % oder mehr, besonders bevorzugt 99 % oder mehr und ganz besonders bevorzugt 100,0 %.

Dies gilt sowohl für die Medien als Zusammensetzungen mit ihren Bestandteilen, die Komponenten und Verbindungen sein können, als auch für die Komponenten mit ihren Bestandteilen, den Verbindungen. Lediglich in Bezug auf die Konzentration einer einzelnen Verbindung im Verhältnis zum gesamten Medium bedeutet der Begriff enthalten: die Konzentration der betreffenden Verbindung beträgt bevorzugt 1 % oder mehr, besonders bevorzugt 2 % oder mehr, ganz besonders bevorzugt 4 % oder mehr.

Für die vorliegende Erfindung bedeutet "≤" kleiner oder gleich, bevorzugt kleiner und "≥" größer oder gleich, bevorzugt größer.

Für die vorliegende Erfindung bedeuten

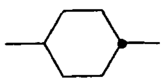 , 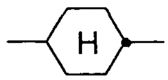 und 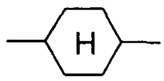

trans-1,4-Cyclohexylen und

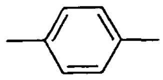

und

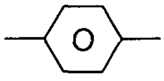

1,4-Phenylen.

Für die vorliegende Erfindung bedeuten die Begriffe "dielektrisch positive Verbindungen" solche Verbindungen mit einem $\Delta\varepsilon > 1,5$, "dielektrisch neutrale Verbindungen" solche mit $-1,5 \leq \Delta\varepsilon \leq 1,5$ und "dielektrisch negative" Verbindungen solche mit $\Delta\varepsilon < -1,5$. Hierbei wird die dielektrische Anisotropie der Verbindungen bestimmt, indem 10 % der Verbindungen in einem flüssigkristallinen Host gelöst werden und von der resultierenden Mischung die Kapazität in mindestens jeweils einer Testzelle mit 20 $\mu$m Schichtdicke mit homeotroper und mit homogener Oberflächenorientierung bei 1 kHz bestimmt wird. Die Messspannung beträgt typischerweise 0,5 V bis 1,0 V, sie ist jedoch stets niedriger als die kapazitive Schwelle der jeweiligen untersuchten Flüssigkristallmischung.

Als Hostmischung für dielektrisch positive und dielektrisch neutrale Verbindungen wird ZLI-4792 und für dielektrisch negative Verbindungen ZLI-2857, beide von Merck KGaA, Deutschland, verwendet. Aus der Änderung der Dielektrizitätskonstante der Hostmischung nach Zugabe der zu untersuchenden Verbindung und Extrapolation auf 100 % der eingesetzten Verbindung werden die Werte für die jeweiligen zu untersuchenden Verbindungen erhalten. Die zu untersuchende Verbindung wird zu 10 % in der Hostmischung gelöst. Wenn die Löslichkeit der Substanz hierzu zu gering ist, wird die Konzentration schrittweise solange halbiert, bis die Untersuchung bei der gewünschten Temperatur erfolgen kann.

Die erfindungsgemäßen Flüssigkristallmedien können bei Bedarf auch weitere Zusatzstoffe wie z. B. Stabilisatoren und/oder pleochroitische Farbstoffe und/oder chirale Dotierstoffe in den üblichen Mengen enthalten. Die eingesetzte Menge dieser Zusatzstoffe beträgt bevorzugt insgesamt 0 % oder mehr bis 10 % oder weniger bezogen auf die Menge der gesamten Mischung, besonders bevorzugt 0,1 % oder mehr bis 6 % oder weniger. Die Konzentration der einzelnen eingesetzten Verbindungen beträgt bevorzugt 0,1 % oder mehr bis 3 % oder weniger. Die Konzentration dieser und ähnlicher Zusatzstoffe wird bei der Angabe der Konzentrationen sowie der Konzentrationsbereiche der Flüssigkristallverbindungen in den Flüssigkristallmedien in der Regel nicht berücksichtigt.

In einer bevorzugten Ausführungsform enthalten die erfindungsgemäßen Flüssigkristallmedien einen Polymervorläufer, der eine oder mehrere reaktive Verbindungen, bevorzugt reaktive Mesogene und bei Bedarf auch weitere Zusatzstoffe wie z. B. Polymerisationsinitiatoren und/oder Polymerisationsmoderatore in den üblichen Mengen enthalten. Die eingesetzte Menge dieser Zusatzstoffe beträgt insgesamt 0 % oder mehr bis 10 % oder weniger bezogen auf die Menge der gesamten Mischung bevorzugt 0,1 % oder mehr bis 2 % oder weniger. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 oder mehr bis 30 oder weniger, besonders bevorzugt aus 6 oder mehr bis 20 oder weniger und ganz besonders bevorzugt aus 10 oder mehr bis 16 oder weniger Verbindungen, die auf herkömmliche Weise gemischt werden. In der Regel wird die gewünschte Menge der in geringerer Menge verwendeten Komponenten in den Komponenten gelöst, die den Hauptbestandteil der Mischung ausmachenden. Dies erfolgt zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z. B. unter Verwendung von Vormischungen oder aus einem so genannten "Multi Bottle System" herzustellen.

Die erfindungsgemäßen Mischungen zeigen sehr breite nematische Phasenbereiche mit Klärpunkten 65°C oder mehr, sehr günstige Werte für die kapazitive Schwelle, relativ hohe Werte für die Holding Ratio und gleichzeitig sehr gute Tieftemperaturstabilitäten bei -30°C und -40°C. Weiterhin zeichnen sich die erfindungsgemäßen Mischungen durch niedrige Rotationsviskositäten $\gamma_1$ aus.

Es versteht sich für den Fachmann von selbst, dass die erfindungsgemäßen Medien für die Verwendung in VA-, IPS-, FFS- oder PALC-Anzeigen auch Verbindungen enthalten kann, worin beispielsweise H, N, O, Cl, F durch die entsprechenden Isotope ersetzt sind.

Der Aufbau der erfindungsgemäßen Flüssigkristallanzeigen entspricht der üblichen Geometrie, wie sie z. B. in EP-OS 0 240 379, beschrieben wird.

Mittels geeigneter Zusatzstoffe können die erfindungsgemäßen Flüssigkristallphasen derart modifiziert werden, dass sie in jeder bisher bekannt gewordenen Art von z. B. ECB-, VAN-, IPS-, GH- oder ASM-VA-LCD-Anzeige einsetzbar sind.

In der nachfolgenden Tabelle E werden mögliche Dotierstoffe angegeben, die den erfindungsgemäßen Mischungen zugesetzt werden können. Sofern die Mischungen einen oder mehrere Dotierstoffe enthalten, wird er in Mengen von 0,01 % bis 4 %, vorzugsweise 0,1 % bis 1,0 %, eingesetzt.

Stabilisatoren, die beispielsweise den erfindungsgemäßen Mischungen zugesetzt werden können, vorzugsweise in Mengen von 0,01 % bis 6 %, insbesondere 0,1 % bis 3 %, werden nachfolgend in Tabelle F genannt.

Alle Konzentrationen sind für die Zwecke der vorliegenden Erfindung, soweit nicht explizit anders vermerkt, in Massenprozent angegeben und beziehen sich auf die entsprechende Mischung oder Mischungskomponente, soweit nicht explizit anders angegeben.

Alle angegebenen Werte für Temperaturen in der vorliegenden Anmeldung, wie z. B. der Schmelzpunkt T(C,N), der Übergang von der smektischen (S) zur nematischen (N) Phase T(S,N) und der Klärpunkt T(N,I), sind in Grad Celsius (°C) und alle Temperaturdifferenzen entsprechend Differenzgrad (° oder Grad) angegeben, sofern nicht explizit anders angegeben.

Der Begriff "Schwellenspannung" bezieht sich für die vorliegende Erfindung auf die kapazitive Schwelle ($V_0$), auch Freedericksz-Schwelle genannt, sofern nicht explizit anders angegeben.

Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20°C und $\Delta n$ wird bei 589 nm und $\Delta\varepsilon$ bei 1 kHz bestimmt, sofern nicht jeweils explizit anders angegeben.

Die elektrooptischen Eigenschaften, z. B. die Schwellenspannung ($V_0$) (kapazitive Messung) werden, ebenso wie das Schaltverhalten, in bei der Firma Merck Japan hergestellten Testzellen bestimmt. Die Messzellen haben Substrate aus Natriumglas (Sodalime Glas) und sind in einer ECB- bzw. VA-Konfiguration mit Polyimidorientierungsschichten (SE-1211 mit Verdünner **26 (Mischungsverhältnis 1:1) beide der Firma Nissan Chemicals, Japan), die senkrecht zueinander gerieben sind und die eine homöotrope Orientierung der Flüssigkristalle bewirken, ausgeführt. Die Fläche der durchsichtigen, nahezu quadratischen Elektroden aus ITO beträgt $1 cm^2$.

Die verwendeten Flüssigkristallmischungen sind, wenn nicht anders angegeben, nicht mit einem chiralen Dotierstoff versetzt, sie eignen sich aber auch besonders für Anwendungen, in denen eine solche Dotierung erforderlich ist.

Die VHR wird in bei der Firma Merck Japan hergestellten Testzellen bestimmt. Die Messzellen haben Substrate aus Natriumglas (Sodalime Glas) und sind mit Polyimidorientierungsschichten (AL-3046 der Firma Japan Synthetic Rubber, Japan) mit einer Schichtdicke von 50 nm, die senkrecht zueinander gerieben sind ausgeführt. Die Schichtdicke beträgt einheitlich 6,0 $\mu$m. Die Fläche der durchsichtigen Elektroden aus ITO beträgt $1 cm^2$.

Die VHR wird bei 20°C ($VHR_{20}$) und nach 5 Minuten im Ofen bei 100°C ($VHR_{100}$) in einem kommerziell erhältlichen Gerät der Firma Autronic Melchers, Deutschland bestimmt. Die verwendete Spannung hat eine Frequenz von 60 Hz.

Die Genauigkeit der Messwerte der VHR hängt vom jeweiligen Wert der VHR ab. Dabei nimmt die Genauigkeit mit geringer werdenden Werten ab. Die bei Werten in den verschiedenen Größenbereichen in der Regel beobachten Abweichungen sind in ihrer Größenordnung in der folgenden Tabelle zusammen gestellt.

| VHR-Bereich | | Abweichung (relativ) |
|---|---|---|
| VHR-Werte | | $\Delta_G$VHR/VHR/% |
| von | bis | ca. |
| 99,6 % | 100 % | +/- 0,1 |
| 99,0 % | 99,6 % | +/- 0,2 |
| 98 % | 99 % | +/- 0,3 |
| 95 % | 98 % | +/- 0,5 |
| 90% | 95 % | +/- 1 |
| 80 % | 90 % | +/- 2 |
| 60 % | 80 % | +/- 4 |
| 40 % | 60 % | +/- 8 |
| 20 % | 40 % | +/- 10 |
| 10 % | 20 % | +/- 20 |

Die Stabilität gegen Bestrahlung mit UV wird in einem "Suntest CPS" einem kommerziellen Gerät der Firma Heraeus, Deutschland untersucht. Dabei werden die versiegelten Testzellen 2,0 Stunden ohne zusätzliche thermische Belastung bestrahlt. Die Bestrahlungsleistung im Wellenlängebereich von 300 nm bis 800 nm beträgt 765 $W/m^2$ V. Es wird ein UV "cut-off" Filter mit einer Kantenwellenlänge von 310 nm verwendet um den sogenannten Fensterglasmodus (Englisch: "window glass mode") zu simulieren. Bei jeder Versuchsserie werden für jede Bedingung mindestens vier

Testzellen untersucht und die jeweiligen Ergebnisse werden als Mittelwerte der entsprechenden einzelnen Messungen angegeben.

Die üblicherweise durch die Belastung, z.B. durch Bestrahlung mit UV durch eine LCD-Hintergrundbeleuchtung, verursachte Abnahme der Voltage Holding Ratio (ΔVHR) wird nach der folgenden Gleichung (1) bestimmt:

$$\Delta VHR(t) = VHR(t) - VHR(t=0) \qquad (1).$$

Eine weitere Kenngröße, die neben der VHR die Leitfähigkeit der Flüssigkristallmischungen charakterisieren kann, ist die Ionendichte ("ion density"). Hohe Werte der Ionendichte führen oft zur Entstehung von Displayfehler wie Imagesticking und Flackern. Die Ionendichte wird bevorzugt in Testzellen bestimmt, die bei Merck Japan[Ltd.] hergestellten werden. Die Testzellen haben Substrate aus Natriumglas (Sodalime Glas) und sind mit Polyimidorientierungsschichten (AL-3046 der Firma Japan Synthetic Rubber, Japan) mit einer Schichtdicke des Polyimids von 40 nm ausgeführt. Die Schichtdicke der Flüssigkristallmischung beträgt einheitlich 5,8 μm. Die Fläche der kreisförmigen, durchsichtigen Elektroden aus ITO, die zusätzlich mit einem "guard-ring" ausgestattet sind, beträgt 1 $cm^2$. Die Genauigkeit der Messmethode beträgt ca. ± 15 %. Die Zellen werden vor der Befüllung mit der betreffenden Flüssigkristallmischung über Nacht in einem Ofen bei 120°C getrocknet.

Die Ionendichte wird mit einem kommerziell erhältlichen Gerät der Firma TOYO, Japan gemessen. Bei der Messmethode handelt es sich im Wesentlichen um eine zur Cyclovoltametrie analogen Messmethode, wie in M. Inoue, "Recent measurement of Liquid Crystal Material Characterisitcs", Proceedings IDW 2006, LCT-7-1,647 beschrieben. Hierbei wird eine angelegte Gleichspannung gemäß eines vorgegebenen Dreiecksprofils zwischen einem positiven bzw. negativen Maximalwert variiert. Ein komplettes Durchlaufen des Profils bildet somit einen Messzyklus. Ist die angelegte Spannung groß genug, so dass sich die Ionen im Feld zur jeweiligen Elektrode bewegen können, entsteht durch Entladung der Ionen ein Ionenstrom. Die übertragene Ladungsmenge liegt hierbei typischerweise im Bereich von einigen pC bis zu wenigen nC. Dieses macht eine hochempfindliche Detektion notwendig, die durch das oben genannte Gerät gewährleistet ist. Die Resultate werden in einer Strom/Spannungs-Kurve dargestellt. Der Ionenstrom ist hierbei durch Auftreten eines Peaks bei Spannungen, die kleiner sind als die Schwellspannung der Flüssigkristallmischung, zu erkennen. Durch Integration der Peakfläche erhält man den Wert für die Ionendichte der untersuchten Mischung. Pro Mischung werden vier Testzellen gemessen. Die Wiederholfrequenz der Dreiecksspannung beträgt 0,033 Hz, die Messtemperatur 60°C, die Maximalspannung ± 3 V bis ± 10 V je nach der Größe der dielektrischen Anisotropie der betreffenden Mischung.

Die Rotationsviskosität wird mit der Methode des rotierenden Permanentmagneten und die Fließviskosität in einem modifizierten Ubbelohde-Viskosimeter bestimmt. Für die Flüssigkristallmischungen ZLI-2293, ZLI-4792 und MLC-6608, alle Produkte der Firma Merck KGaA, Darmstadt, Deutschland, betragen die bei 20°C bestimmten Werte der Rotationsviskosität 161 mPa·s, 133 mPa·s bzw. 186 mPa·s und die der Fließviskosität (ν) 21 $mm^2 \cdot s^{-1}$, 14 $mm^2 \cdot s^{-1}$ bzw. 27 $mm^2 \cdot s^{-1}$.

Es werden, wenn nicht explizit anders angegeben, die folgenden Symbole verwendet:

| | |
|---|---|
| $V_o$ | Schwellenspannung, kapazitiv [V] bei 20°C, |
| $n_e$ | außerordentlicher Brechungsindex gemessen bei 20°C und 589 nm, |
| $n_o$ | ordentlicher Brechungsindex gemessen bei 20°C und 589 nm, |
| $\Delta n$ | optische Anisotropie gemessen bei 20°C und 589 nm, |
| $\varepsilon_\perp$ | dielektrische Suszeptibilität senkrecht zum Direktor bei 20°C und 1 kHz, |
| $\varepsilon_\parallel$ | dielektrische Suszeptibilität parallel zum Direktor bei 20°C und 1 kHz, |
| $\Delta\varepsilon$ | dielektrische Anisotropie bei 20°C und 1 kHz, cp. bzw. T(N,I) Klärpunkt [°C], |
| $\nu$ | Fließviskosität gemessen bei 20°C [$mm^2 \cdot s^{-1}$], |
| $\gamma_1$ | Rotationsviskosität gemessen bei 20°C [mPa·s], |
| $K_1$ | elastische Konstante, "splay"-Deformation bei 20°C [pN], |
| $K_2$ | elastische Konstante, "twist"-Deformation bei 20°C [pN], |
| $K_3$ | elastische Konstante, "bend"-Deformation bei 20°C [pN] und |
| LTS | Tieftemperaturstabilität der Phase ("low temperature stability"), bestimmt in Testzellen, |
| VHR | Spannungshaltevermögen ("voltage holding ratio"), |
| ΔVHR | Abnahme der Voltage Holding Ratio, |
| $S_{rel}$ | relative Stabilität der VHR. |

Die folgenden Beispiele erläutern die vorliegende Erfindung ohne sie zu begrenzen. Sie zeigen dem Fachmann jedoch bevorzugte Mischungskonzepte mit bevorzugt einzusetzenden Verbindungen und deren jeweiligen Konzentrationen sowie deren Kombinationen miteinander. Außerdem illustrieren die Beispiele, welche Eigenschaften und Eigenschaftskombinationen zugänglich sind.

Für die vorliegende Erfindung und in den folgenden Beispielen sind die Strukturen der Flüssigkristallverbindungen durch Acronyme angegeben, wobei die Transformation in chemische Formeln gemäß folgender Tabellen A bis C erfolgt. Alle Reste $C_nH_{2n+1}$, $C_mH_{2m+1}$ und $C_lH_{2l+1}$ bzw. $C_nH_{2n}$, $C_mH_{2m}$ und $C_lH_{2l}$ sind geradkettige Alkylreste bzw. Alkylenreste jeweils mit n, m bzw. l C-Atomen. In Tabelle A sind die Ringelemente der Kerne der Verbindung codiert, in Tabelle B sind die Brückenglieder aufgelistet und in Tabelle C sind die Bedeutungen der Symbole für die linken bzw. rechten Endgruppen der Moleküle aufgelistet. Die Akronyme werden aus den Codes für die Ringelemente mit optionalen Verknüpfungsgruppen, gefolgt von einem ersten Bindestrich und den Codes für die linke Endgruppe, sowie einem zweiten Bindestrich und den Codes für die rechts Endgruppe, zusammengesetzt. In Tabelle D sind Beispielstrukturen von Verbindungen mit ihren jeweiligen Abkürzungen zusammengestellt.

**Tabelle A: Ringelemente**

| | | | |
|---|---|---|---|
| **C** | 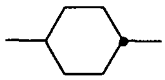 | | |
| **D** | 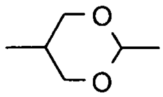 | **DI** | 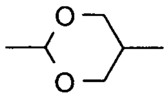 |
| **A** | 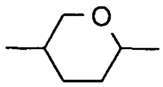 | **AI** | 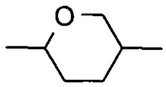 |
| **P** | 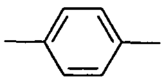 | | |
| **G** | 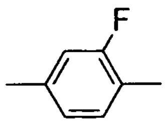 | **GI** | 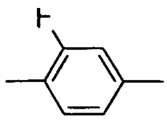 |
| **U** | 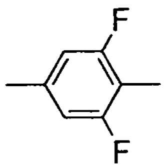 | **UI** | 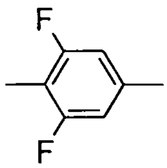 |
| **Y** | 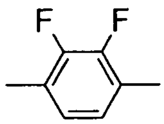 | | |
| **P(F,Cl)** | 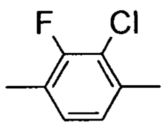 | **P(Cl,F)** | 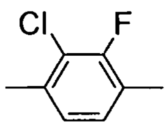 |
| **np** | 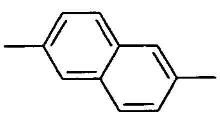 | | |
| **n3f** | 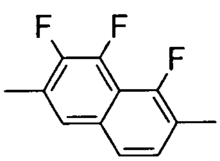 | **n3fl** | 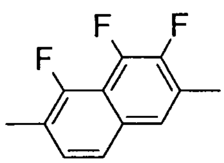 |
| **th** | 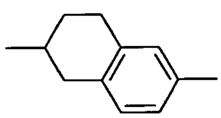 | **thl** | 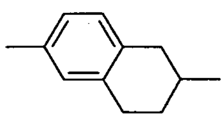 |

(fortgesetzt)

| | | | |
|---|---|---|---|
| **tH2f** | 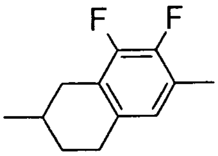 | **tH2fl** | 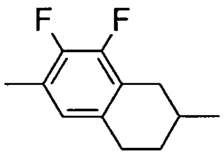 |
| **o2f** | 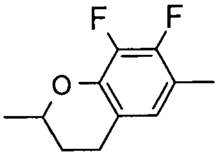 | **o2fl** | 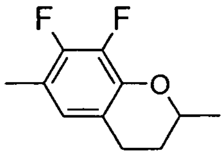 |
| **dh** | 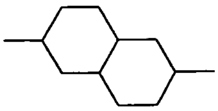 | | |
| **K** | 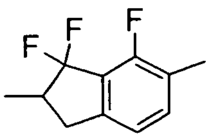 | **Kl** | 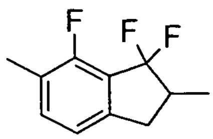 |
| **L** | 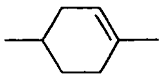 | **Ll** | 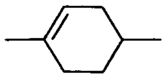 |
| **F** | 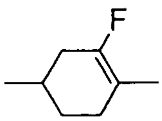 | **Fl** | 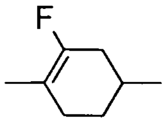 |

**Tabelle B: Brückenglieder**

| | | | |
|---|---|---|---|
| **E** | $-CH_2-CH_2-$ | | |
| **V** | -CH=CH- | | |
| **T** | $-C\equiv C-$ | | |
| **W** | $-CF_2-CF_2-$ | | |
| **B** | -CF=CF- | | |
| **Z** | -CO-O- | **ZI** | -O-CO- |
| **X** | -CF=CH- | **XI** | -CH=CF- |
| **O** | $-CH_2-O-$ | **OI** | $-O-CH_2-$ |
| **Q** | $-CF_2-O-$ | **QI** | $-O-CF_2-$ |

**Tabelle C: Endgruppen**

| Links einzelstehend oder in Kombination | | Rechts einzelstehend oder in Kombination | |
|---|---|---|---|
| **-n-** | $C_nH_{2n+1}-$ | **-n** | $-C_nH_{2n+1}$ |
| **-nO-** | $C_nH_{2n+1}-O-$ | **-nO** | $-O- C_nH_{2n+1}$ |
| **-V-** | $CH_2=CH-$ | **-V** | $-CH=CH_2$ |
| **-nV-** | $C_nH_{2n+1}-CH=CH-$ | **-nV** | $-C_nH_{2n}-CH=CH_2$ |
| **-Vn-** | $CH_2=CH- C_nH_{2n}-$ | **-Vn** | $-CH=CH-C_nH_{2n+1}$ |
| **-nVm-** | $C_nH_{2n+1}-CH=CH-C_mH_{2m}-$ | **-nVm** | $- C_nH_{2n}-CH=CH-C_mH_{2m+1}$ |
| **-N-** | $N\equiv C-$ | **-N** | $-C\equiv N$ |
| **-S-** | S=C=N- | **-S** | -N=C=S |
| **-F-** | F- | **-F** | -F |
| **-CL-** | Cl- | **-CL** | -Cl |

(fortgesetzt)

| Links einzelstehend oder in Kombination | | Rechts einzelstehend oder in Kombination | |
|---|---|---|---|
| **-M-** | $CFH_2$- | **-M** | -$CFH_2$ |
| **-D-** | $CF_2H$- | **-D** | -$CF_2H$ |
| **-T-** | $CF_3$- | **-T** | -$CF_3$ |
| **-MO-** | $CFH_2O$ - | **-OM** | -$OCFH_2$ |
| **-DO-** | $CF_2HO$ - | **-OD** | -$OCF_2H$ |
| **-TO-** | $CF_3O$ - | **-OT** | -$OCF_3$ |
| **-A-** | H-C≡C- | **-A** | -C≡C-H |
| **-nA-** | $C_nH_{2n+1}$-C≡C- | **-An** | -C≡C-$C_nH_{2n+1}$ |
| **-NA-** | N≡C-C≡C- | **-AN** | -C≡C-C≡N |

| Links nur in Kombination | | Rechts nur in Kombination | |
|---|---|---|---|
| **-...n...-** | -$C_nH_{2n}$- | **-...n...** | -$C_nH_{2n}$- |
| **-...M...-** | -CFH- | **-...M...** | -CFH- |
| **-...D...-** | -$CF_2$- | **-...D...** | -$CF_2$- |
| **-...V...-** | -CH=CH- | **-...V...** | -CH=CH- |
| **-...Z...-** | -CO-O- | **-...Z...** | -CO-O- |
| **-...ZI...-** | -O-CO- | **-...ZI...** | -O-CO- |
| **-...K...-** | -CO- | **-...K...** | -CO- |
| **-...W...-** | -CF=CF- | **-...W...** | -CF=CF- |

worin n und m jeweils ganze Zahlen und die drei Punkte "..." Platzhalter für andere Abkürzungen aus dieser Tabelle sind.

Vorzugsweise enthalten die erfindungsgemäßen Mischungen neben den Verbindungen der Formeln I eine oder mehrere Verbindungen der nachfolgend genannten Verbindungen.

Folgende Abkürzungen werden verwendet:

(n, m und l unabhängig voneinander jeweils eine ganze Zahl, bevorzugt 1 bis 6)

**Tabelle D**

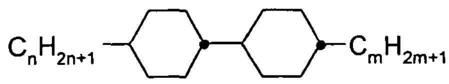

**CC-n-m**

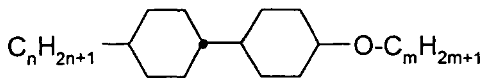

**CC-n-Om**

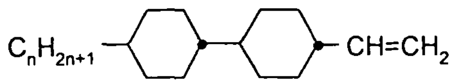

**CC-n-V**

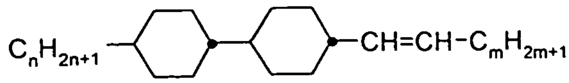

**CC-n-Vm**

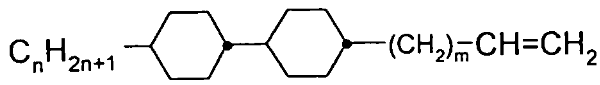

**CC-n-mV**

(fortgesetzt)

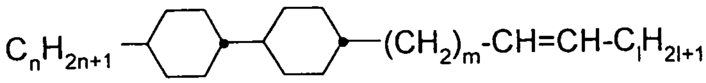

**CC-n-mVI**

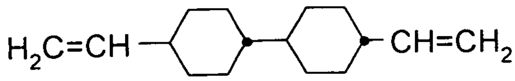

**CC-V-V**

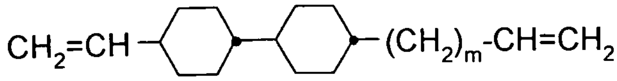

**CC-V-mV**

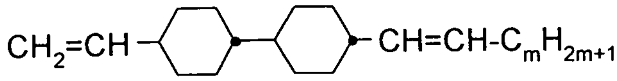

**CC-V-Vm**

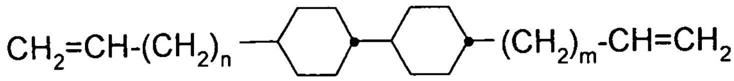

**CC-Vn-mV**

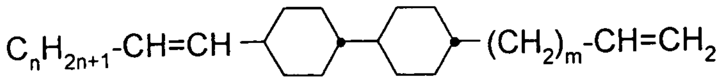

**CC-nV-mV**

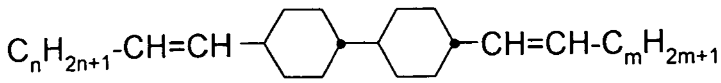

**CC-nV-Vm**

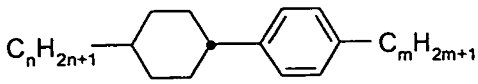

**CP-n-m**

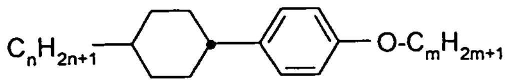

**CP-n-Om**

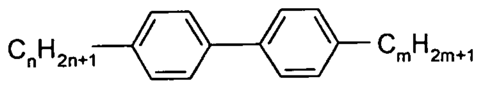

**PP-n-m**

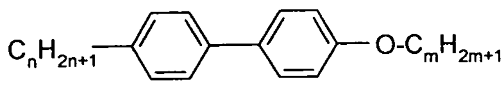

**PP-n-Om**

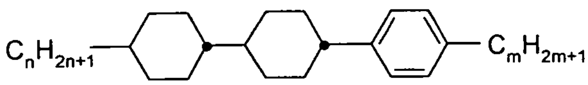

**CCP-n-m**

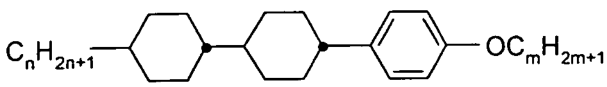

**CCP-n-Om**

(fortgesetzt)

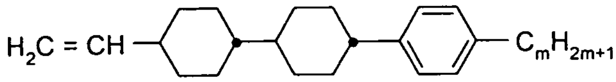

**CCP-V-m**

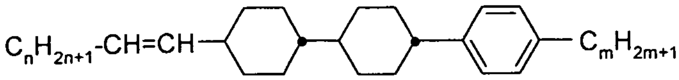

**CCP-nV-m**

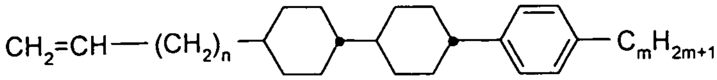

**CCP-Vn-m**

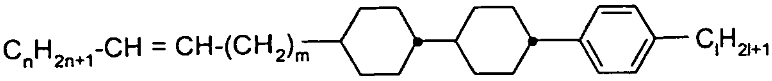

**CCP-nVm-l**

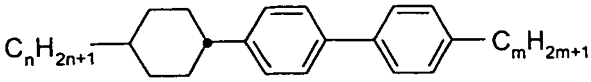

**CPP-n-m**

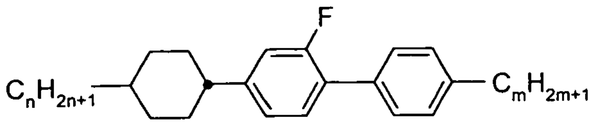

**CGP-n-m**

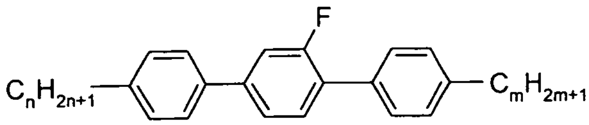

**PGP-n-m**

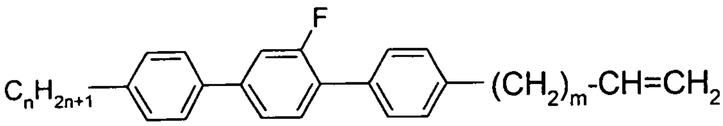

**PGP-n-mV**

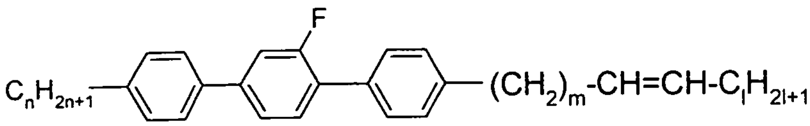

**PGP-n-mVl**

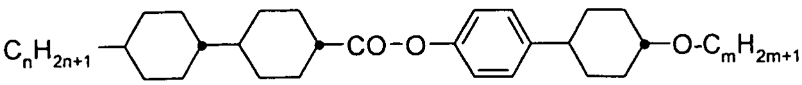

**CCZPC-n-m**

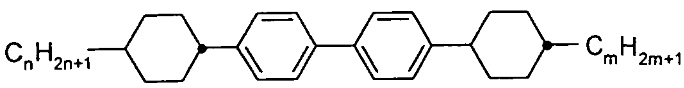

**CPPC-n-m**

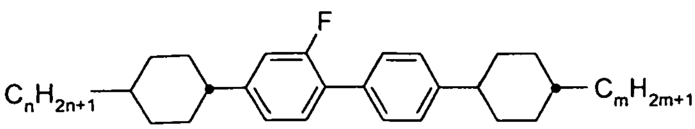

(fortgesetzt)

**CGPC-n-m**

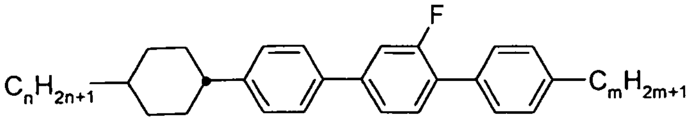

**CPGP-n-m**

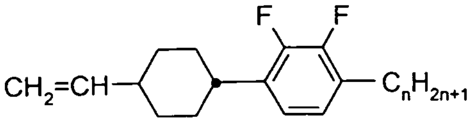

**CY-V-n**

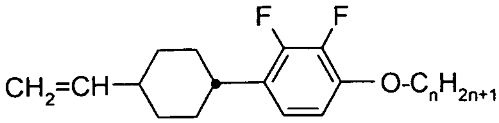

**CY-V-On**

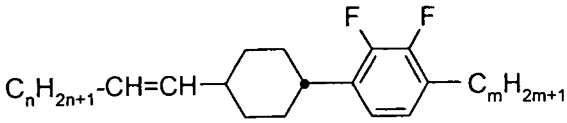

**CY-nV-m**

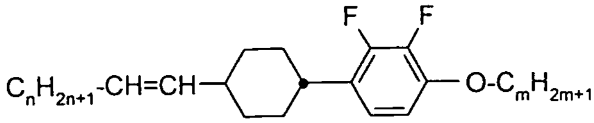

**CY-nV-Om**

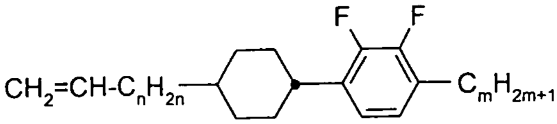

**CY-Vn-m**

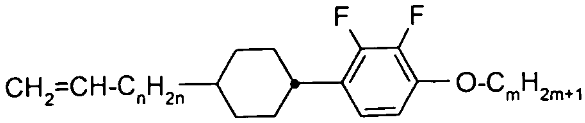

**CY-Vn-Om**

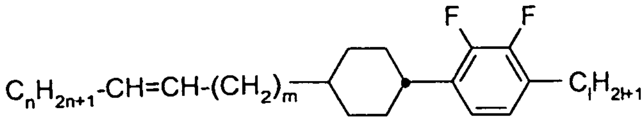

**CY-nVm-l**

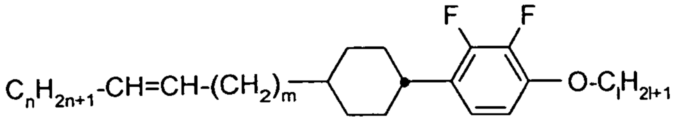

**CY-nVm-Ol**

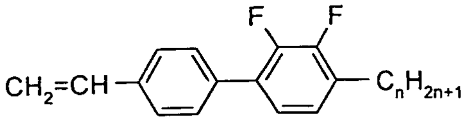

**PY-V-n**

(fortgesetzt)

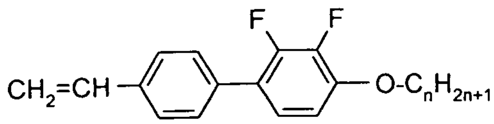

**PY-V-On**

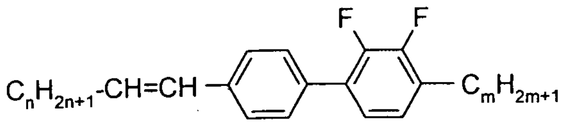

**PY-nV-m**

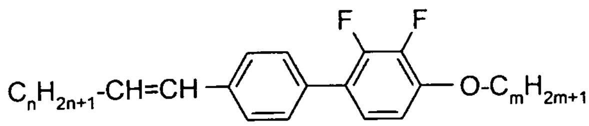

**PY-nV-Om**

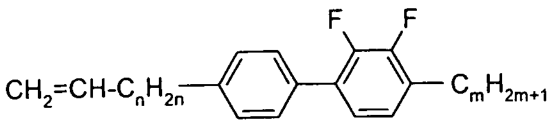

**PY-Vn-m**

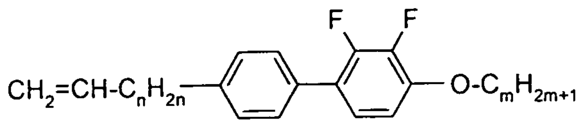

**PY-Vn-Om**

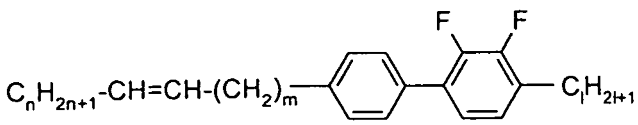

**PY-nVm-l**

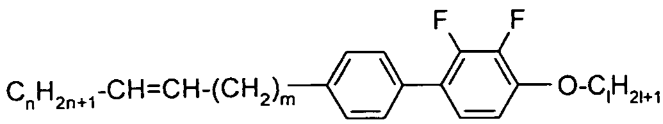

**PY-nVm-Ol**

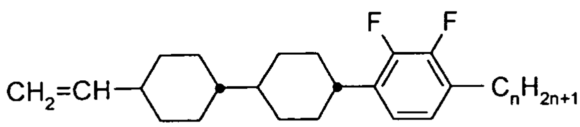

**CCY-V-n**

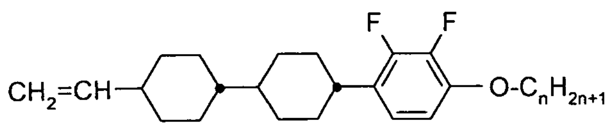

**CCY-V-On**

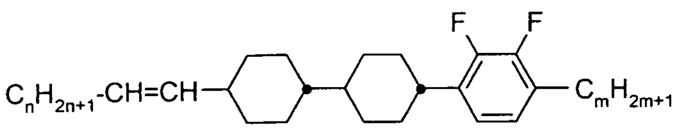

**CCY-nV-m**

(fortgesetzt)

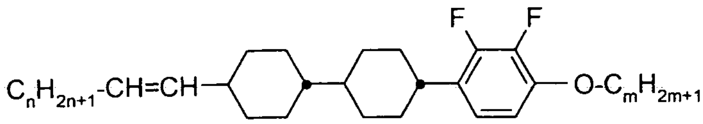

**CCY-nV-Om**

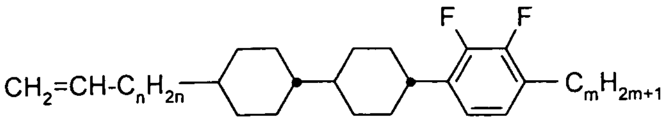

**CCY-Vn-m**

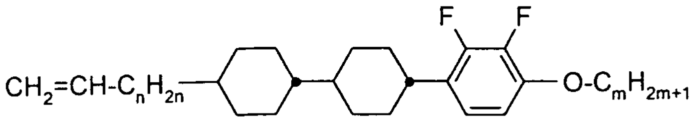

**CCY-Vn-Om**

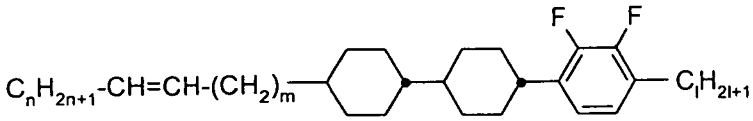

**CCY-nVm-l**

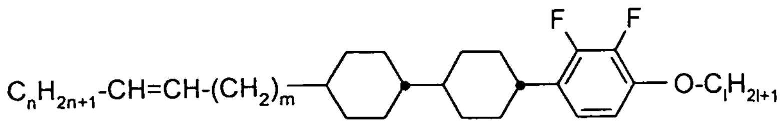

**CCY-nVm-Ol**

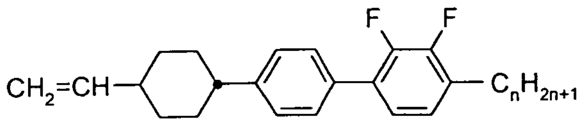

**CPY-V-n**

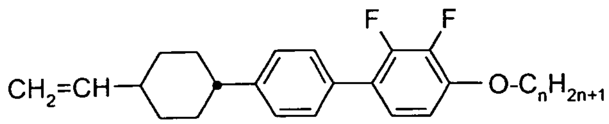

**CPY-V-On**

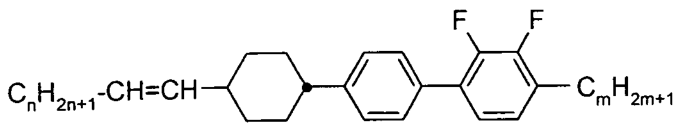

**CPY-nV-m**

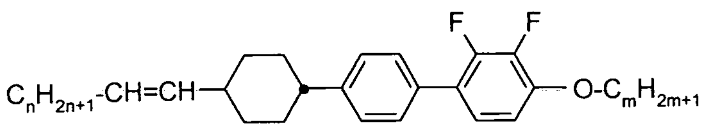

**CPY-nV-Om**

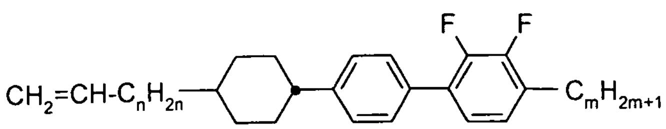

**CPY-Vn-m**

(fortgesetzt)

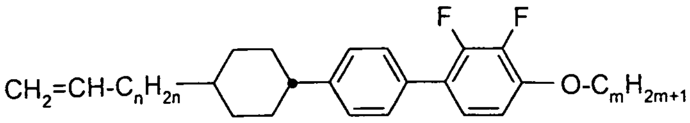

**CPY-Vn-Om**

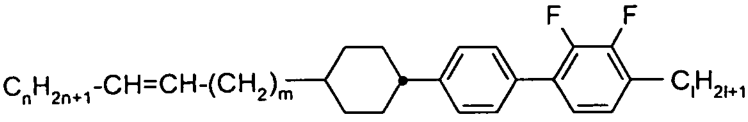

**CPY-nVm-l**

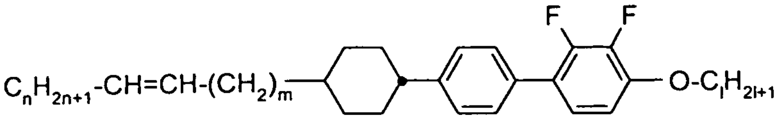

**CPY-nVm-Ol**

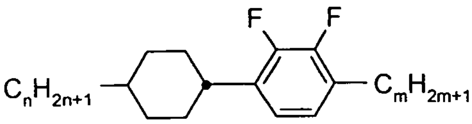

**CY-n-m**

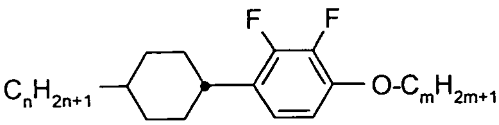

**CY-n-Om**

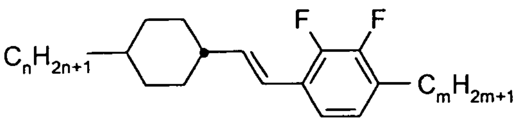

**CVY-n-m**

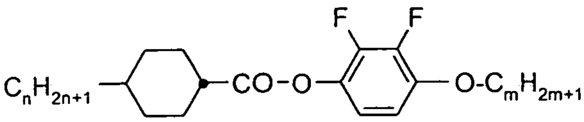

**CZY-n-Om**

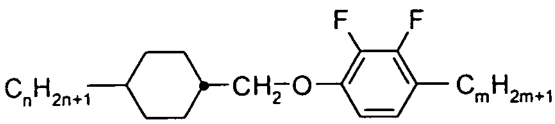

**COY-n-m**

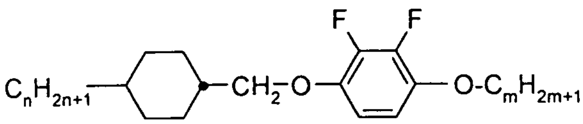

**COY-n-Om**

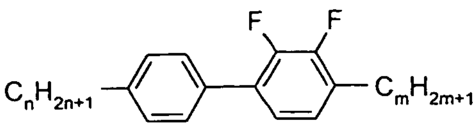

**PY-n-m**

(fortgesetzt)

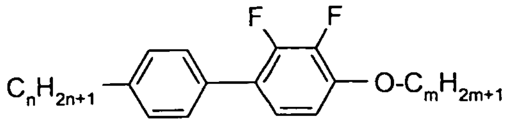

**PY-n-Om**

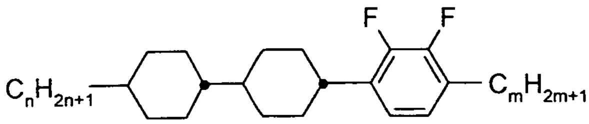

**CCY-n-m**

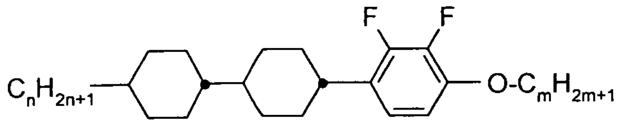

**CCY-n-Om**

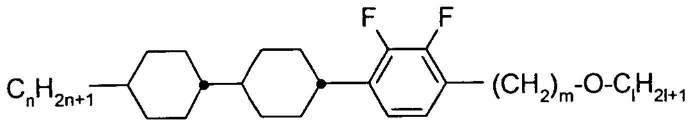

**CCY-n-mOl**

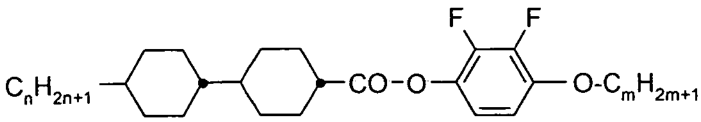

**CCZY-n-Om**

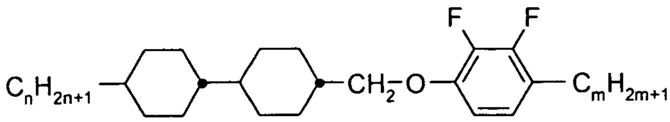

**CCOY-n-m**

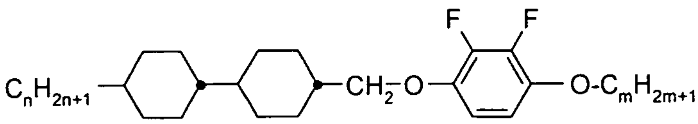

**CCOY-n-Om**

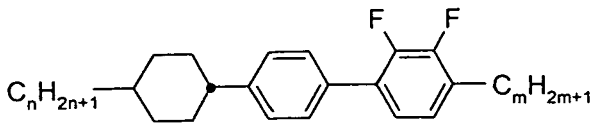

**CPY-n-m**

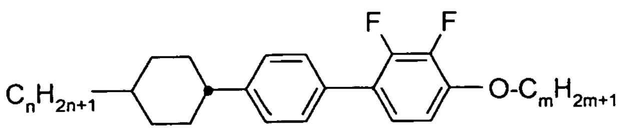

**CPY-n-Om**

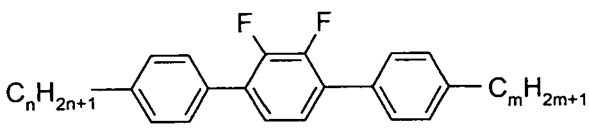

**PYP-n-m**

(fortgesetzt)

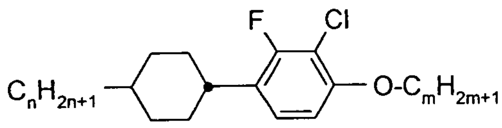

**CP(F,Cl)-n-Om**

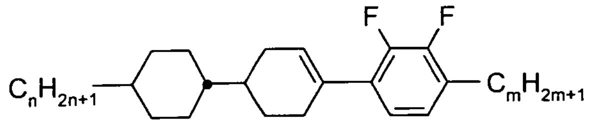

**CLY-n-m**

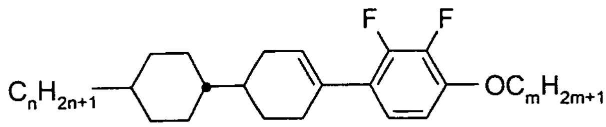

**CLY-n-Om**

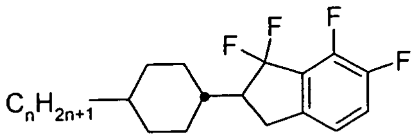

**CK-n-F**

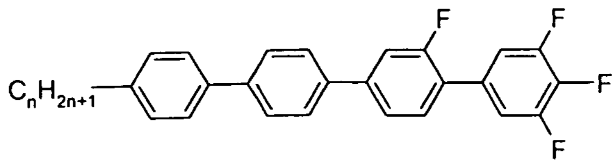

**PPGU-n-F**

In der Tabelle E werden chirale Dotierstoffe genannt, die bevorzugt in den erfindungsgemäßen Mischungen eingesetzt werden.

**Tabelle E**

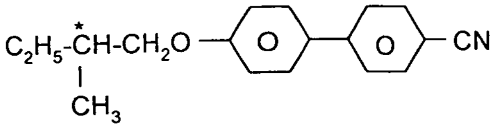

**C 15**

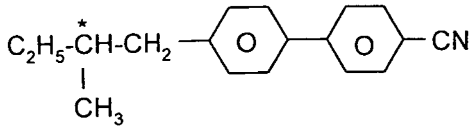

**CB 15**

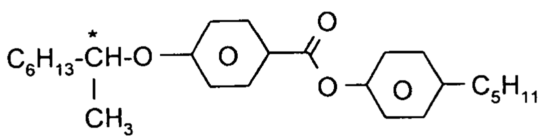

**CM 21**

(fortgesetzt)

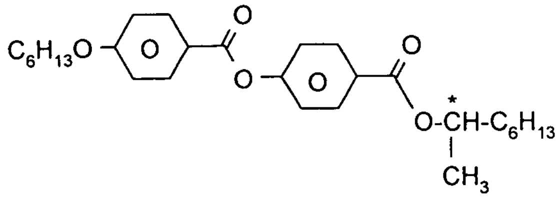

**R S-811 / S-811**

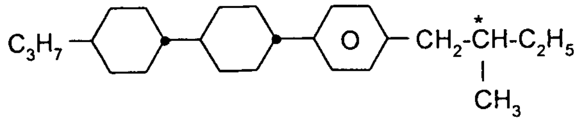

**CM 44**

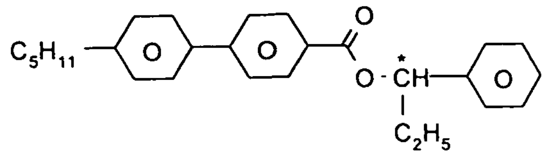

**CM 45**

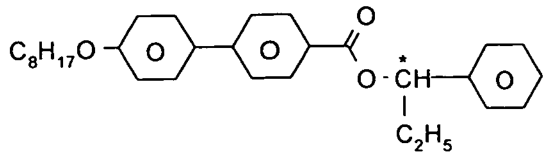

**CM 47**

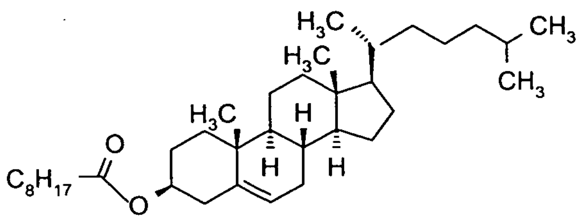

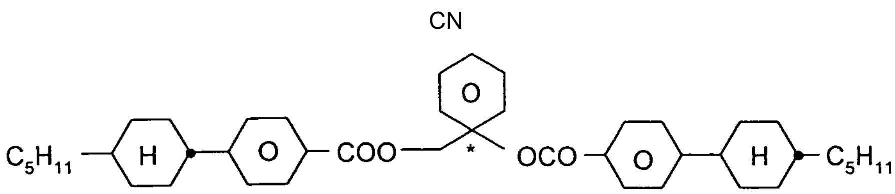

**R-10111 / S-1011**

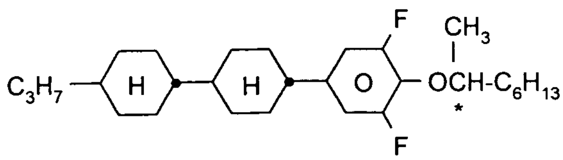

**R-2011 / S-2011**

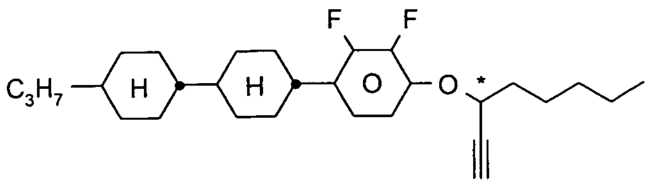

**R-3011 / S-3011**

(fortgesetzt)

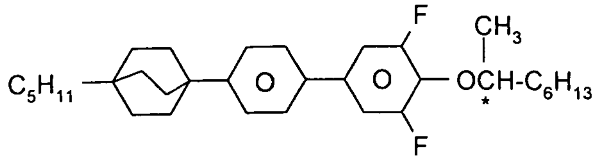

**R-4011 / S-4011**

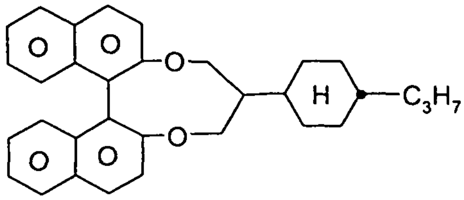

**R-5011 / S-5011**

In einer bevorzugten Ausführungsform der vorliegenden Erfindung enthalten die erfindungsgemäßen Medien eine oder mehrere Verbindungen, ausgewählt aus der Gruppe der Verbindungen der Tabelle E.

In der Tabelle F werden Stabilisatoren genannt, die bevorzugt zusätzlich zu den Verbindungen der Formel I in den erfindungsgemäßen Mischungen eingesetzt werden können. Der Parameter n bedeutet hier eine ganze Zahl im Bereich von 1 bis 12. Insbesondere die gezeigten Phenolderivate sind als zusätzliche Stabilisatoren einsetzbar, da sie als Antioxidantien wirken.

## Tabelle F

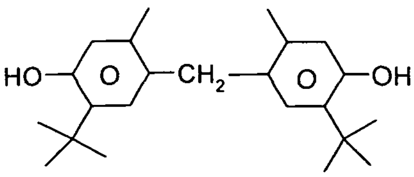

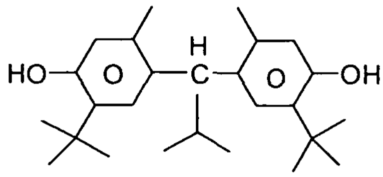

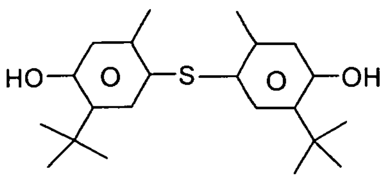

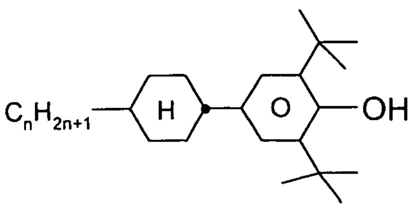

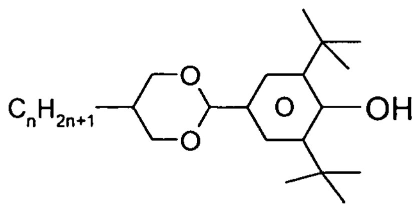

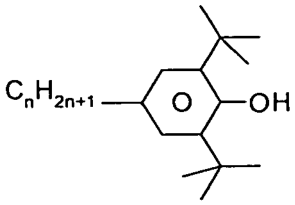

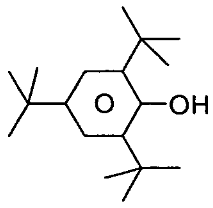

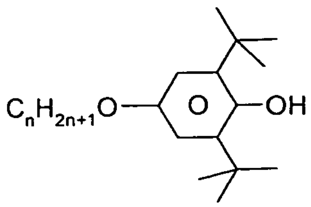

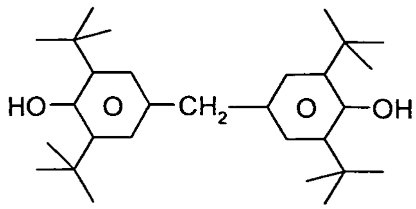

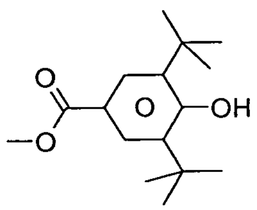

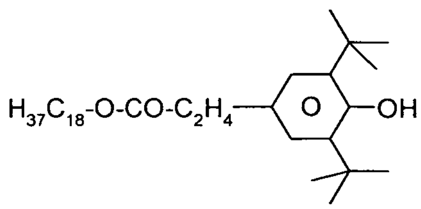

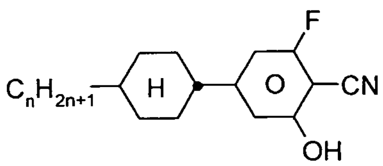

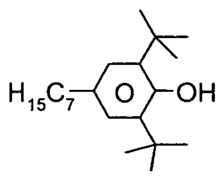

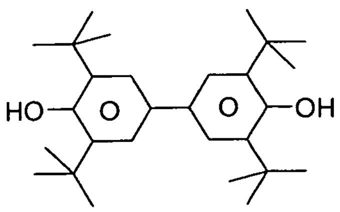

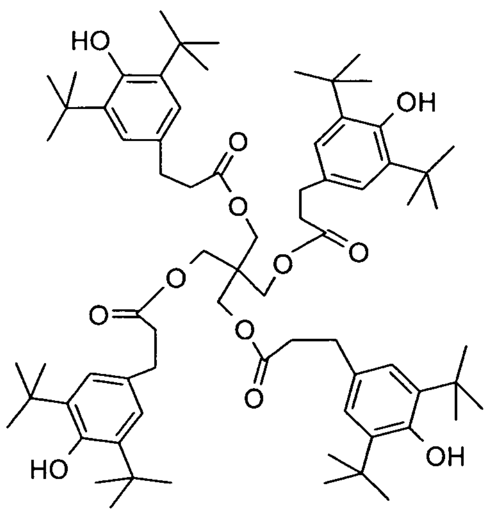

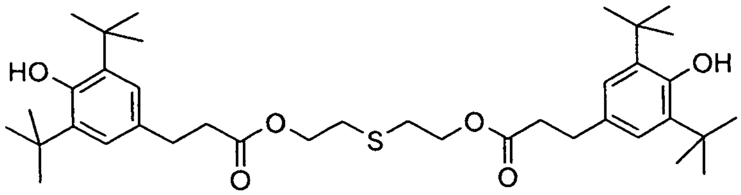

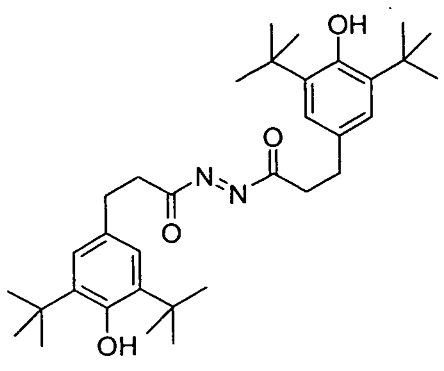

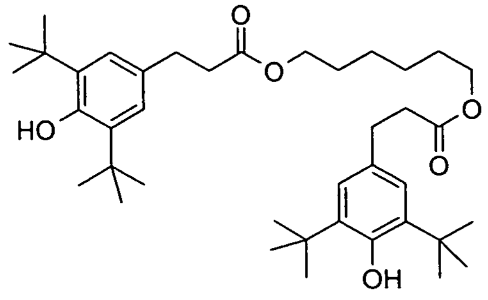

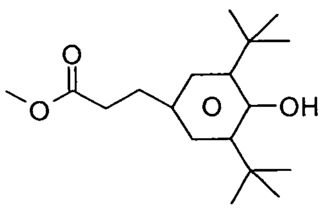

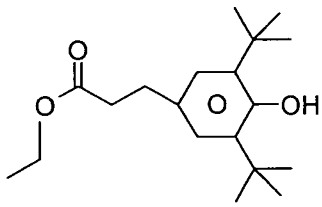

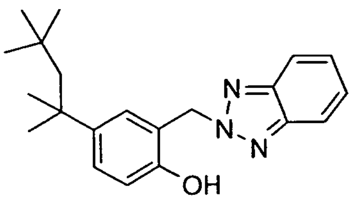

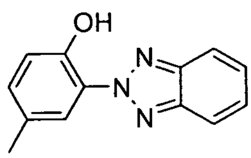

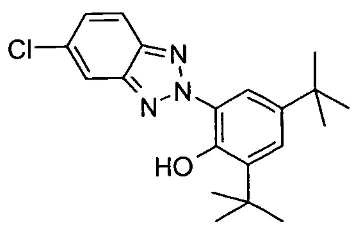

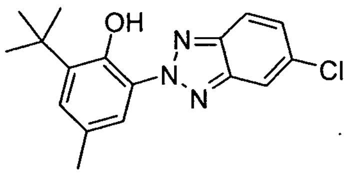

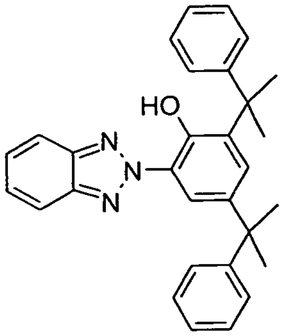

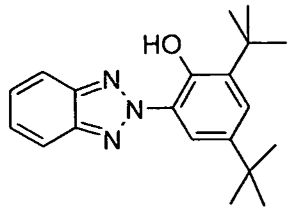

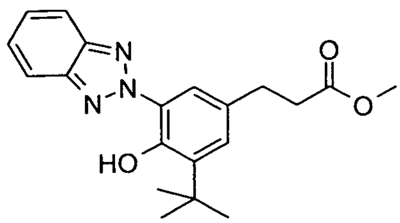

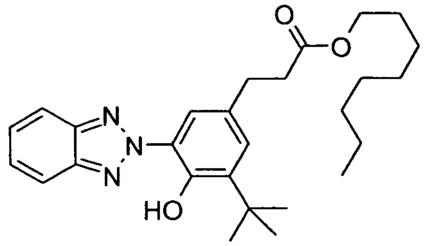

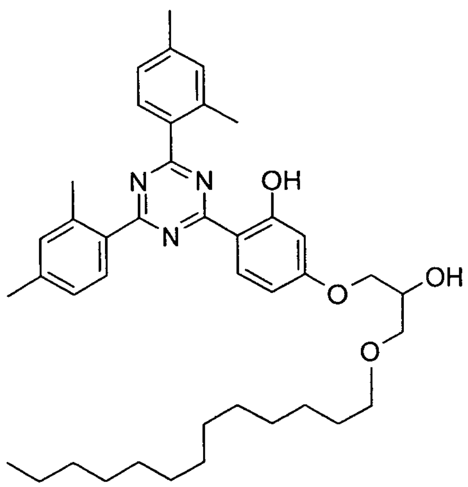

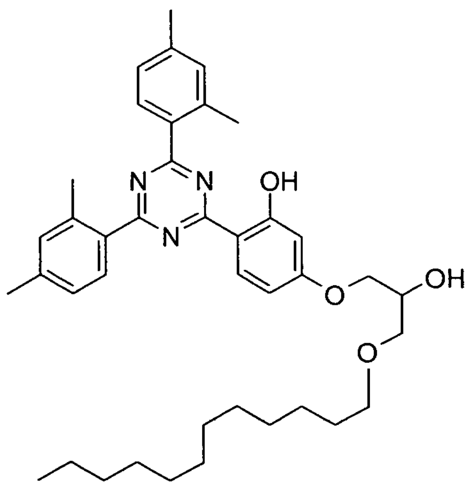

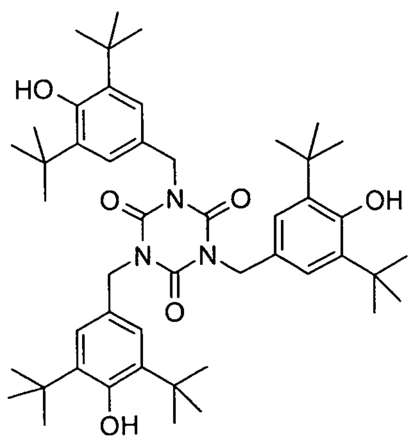

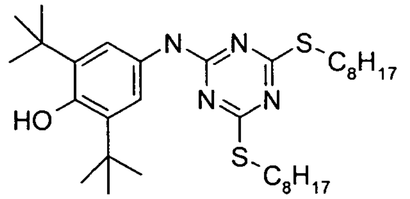

In einer bevorzugten Ausführungsform der vorliegenden Erfindung enthalten die erfindungsgemäßen Medien eine oder mehrere Verbindungen, ausgewählt aus der Gruppe der Verbindungen der Tabelle F, insbesondere eine oder mehrere Verbindungen ausgewählt aus der Gruppe der Verbindungen der beiden Formeln

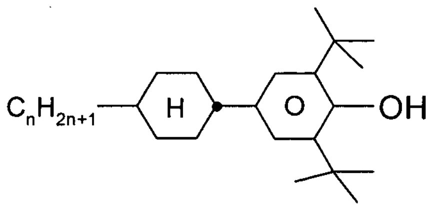

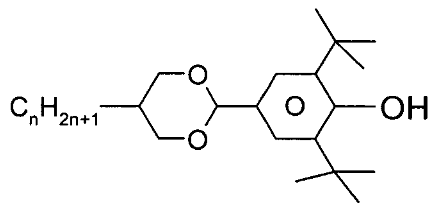

Beispiele

Die folgenden Beispiele erläutern die vorliegende Erfindung, ohne sie in irgendeiner Weise zu beschränken. Aus den physikalischen Eigenschaften wird dem Fachmann jedoch deutlich, welche Eigenschaften zu erzielen sind und in welchen Bereichen sie modifizierbar sind. Insbesondere ist also die Kombination der verschiedenen Eigenschaften, die vorzugsweise erreicht werden können, für den Fachmann gut definiert.

Substanzbeispiele

Die folgenden Substanzen sind bevorzugte Substanzen der Nummern 1 bis 19, 44 und 45 der Formel I gemäß der vorliegenden Anmeldung, bzw. gemäß der vorliegenden Anmeldung bevorzugt einzusetzende Substanzen der Formel I, die übrigen Substanzen sind Vergleichsverbindungen.

1

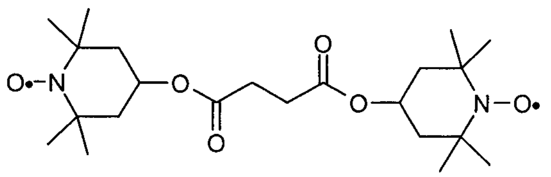

2

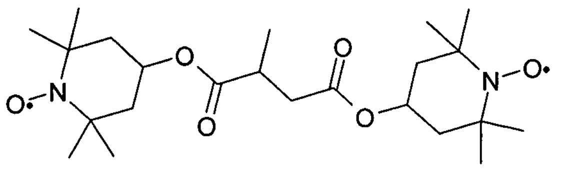

3

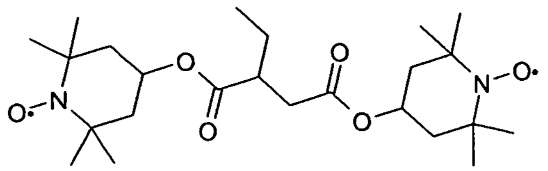

4

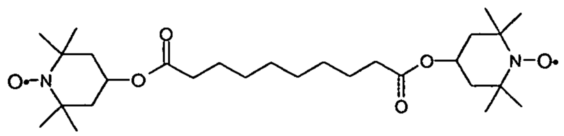

5

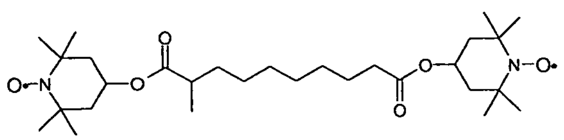

6

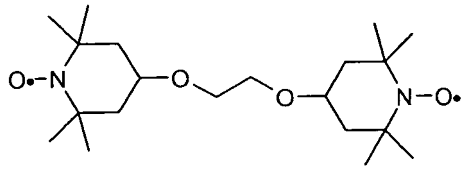

7

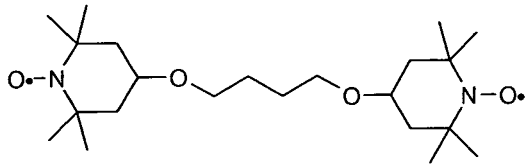

8

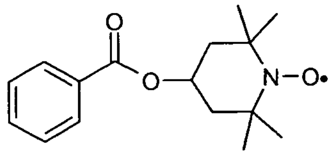

9

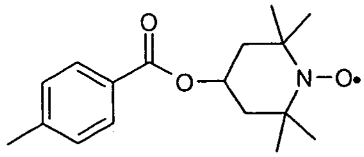

10

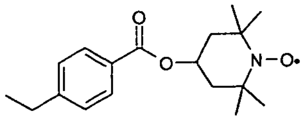

11

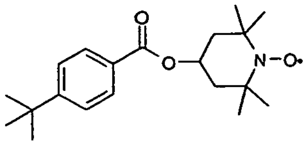

12

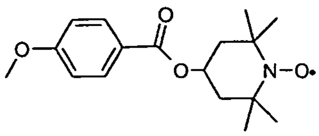

13

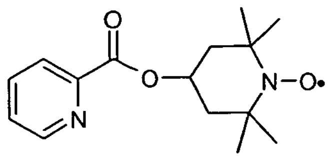

14

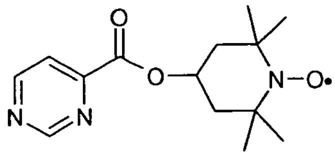

15

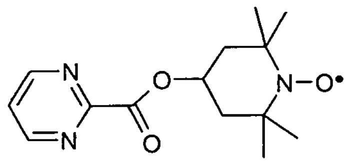

16

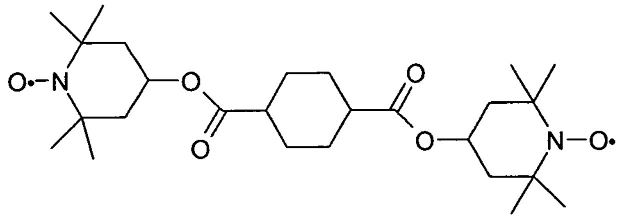

17

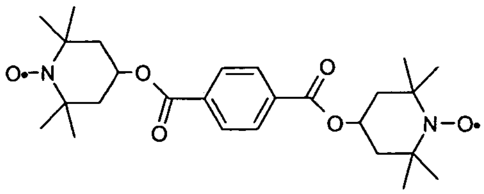

18

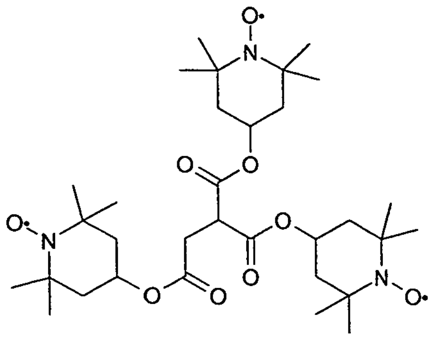

19

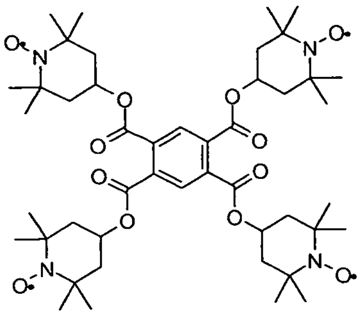

20

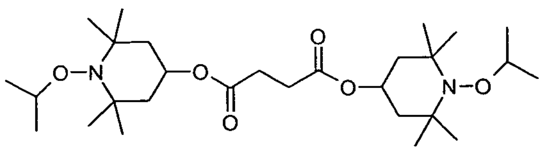

21

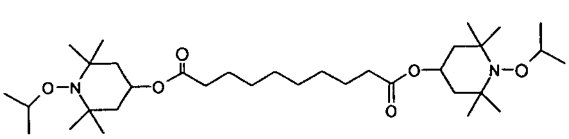

22 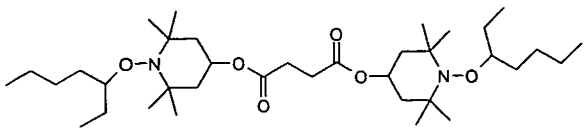

23 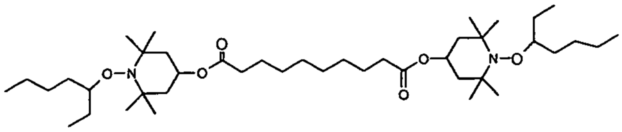

24 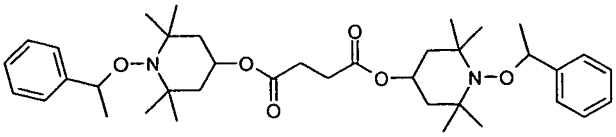

25 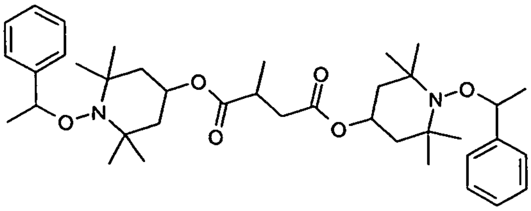

26 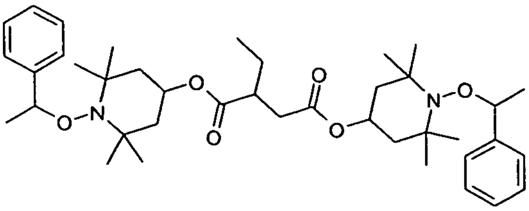

27 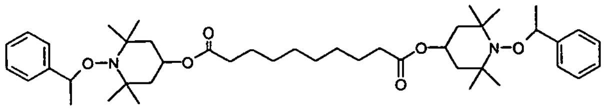

28 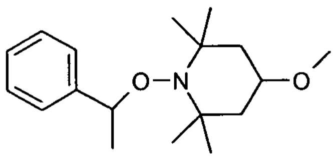

29 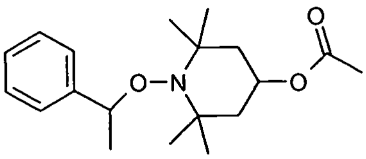

30 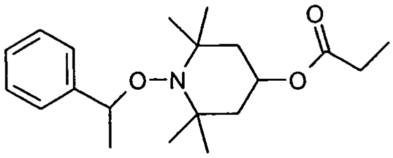

31 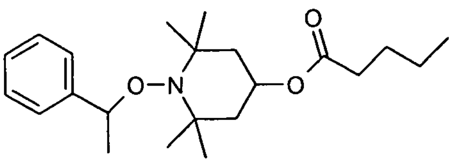

32 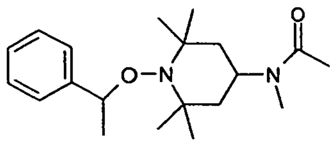

33 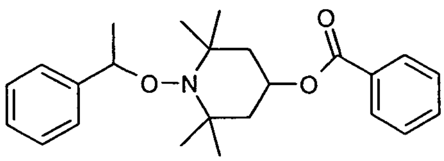

34 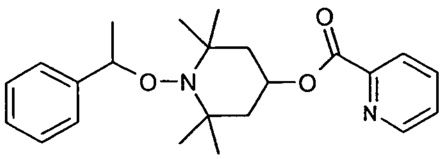

35 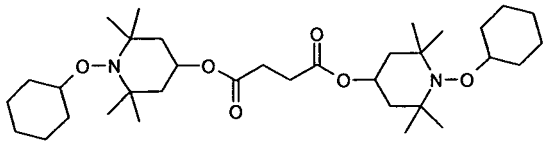

36 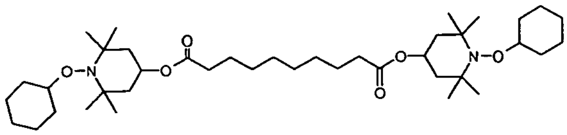

37 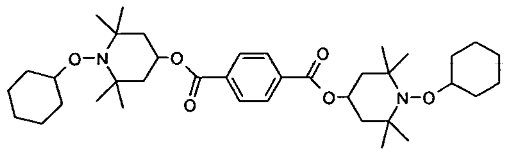

38 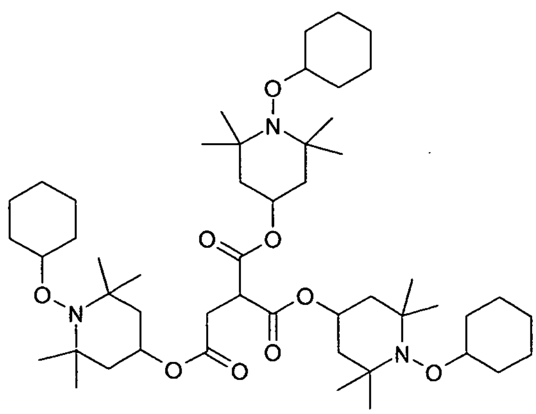

39

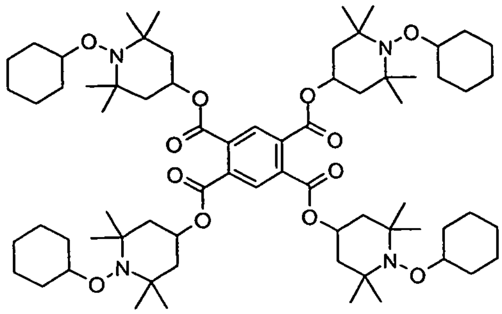

40

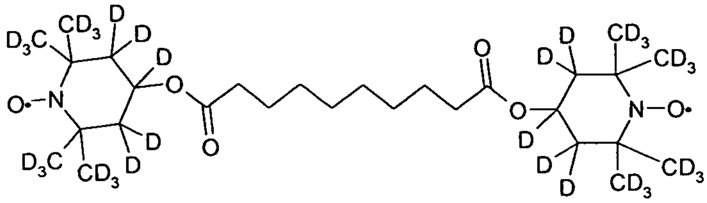

41

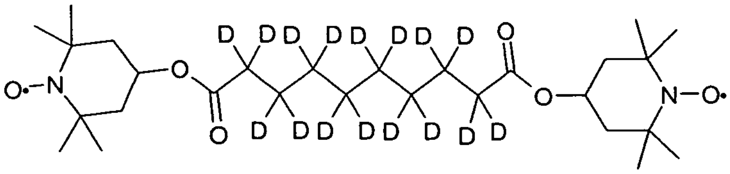

42

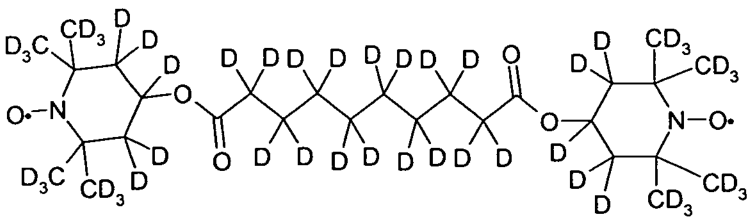

43

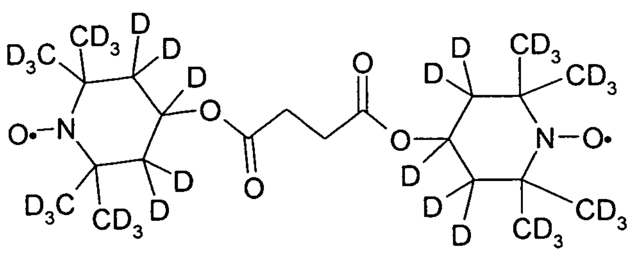

44

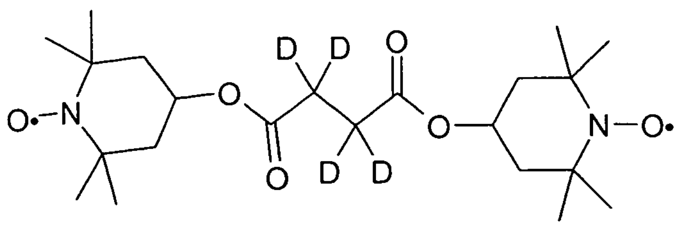

45

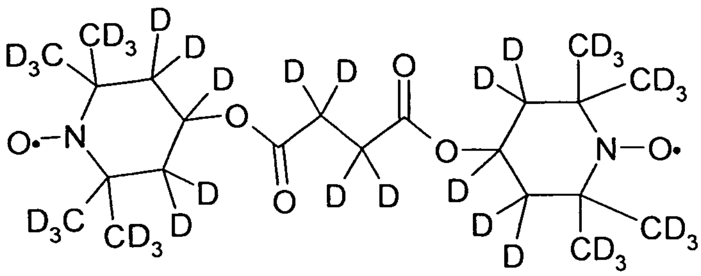

46

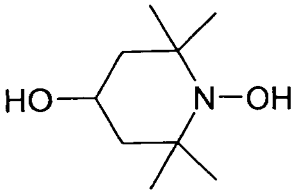

47

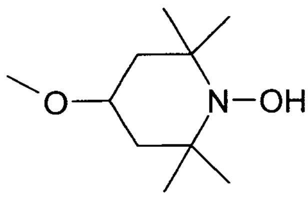

48

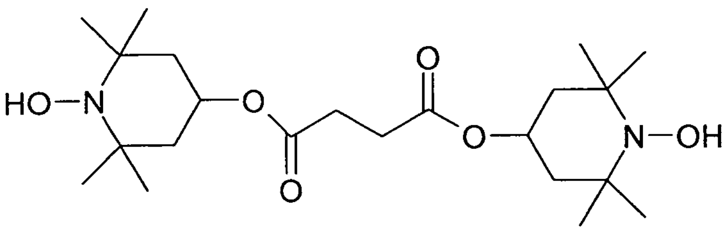

49

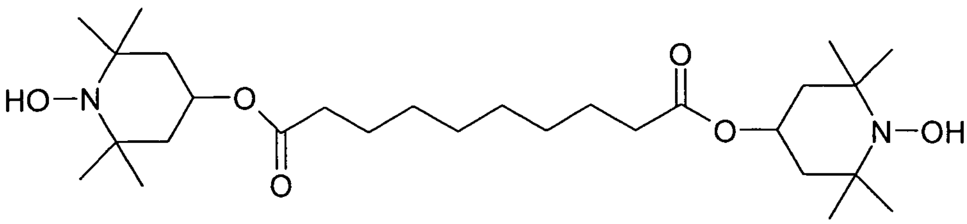

50

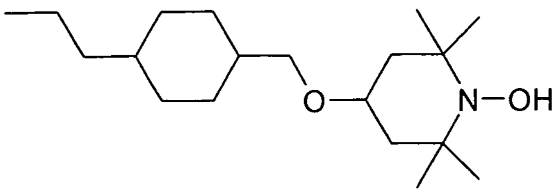

51

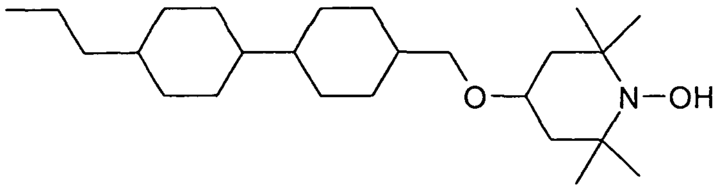

52

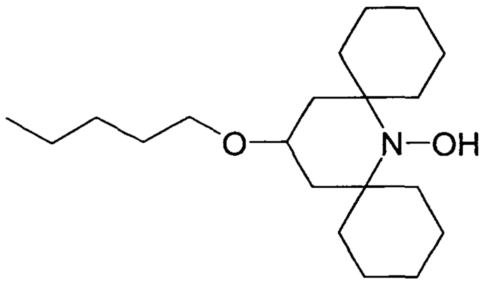

Synthesebeispiel 1: Synthese von Bis(2,2,6,6-tetramethyl-4-piperidyl)-N,N'-dioxylsuccinat (Substanzbeispiel 1)

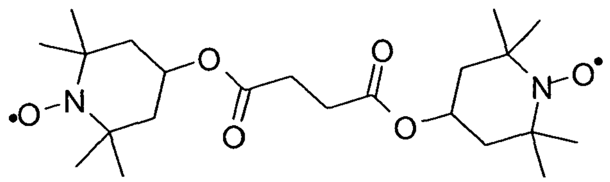

2,15g (12,26 mmol) 4-Hydroxy-2,2,6,6-tetramethylpiperidin-1-oxyl, 40 mg (0,33 mmol) 4-(Dimethylamino)-pyridin und 1 ml (12,4 mmol) getrocknetes Pyridin werden in 20 ml trockenem Dichlormethan vorgelegt. Anschließend wird aktiviertes Molsieb 4 Angström zugegeben und 90 min bei Raumtemperatur (kurz RT; ca. 22°C) gerührt. Die Reaktionslösung wird auf eine Temperatur im Bereich von 7 bis 10°C gekühlt und langsam mit 0,71 ml (6,13 mmol) Bernsteinsäuredichlorid versetzt und wird für 18 h bei RT gerührt. Die Reaktionslösung wird mit ausreichend ges. $NaHCO_3$-Lösung und Dichlormethan versetzt und die organische Phase abgetrennt. Es wird mit Wasser und ges. NaCl-Lösung gewaschen, über $Na_2SO_4$ getrocknet, filtriert und eingeengt. Das Rohprodukt wird über Kieselgel mit Dichlormethan / Methyl-tertbutylether (95:5) gereinigt und man erhält das Produkt als weißen Feststoff mit einer Reinheit von > 99.5 %.

Synthesebeispiel 2: Synthese von Bis(2,2,6,6-tetramethylpiperidin-1-oxyl-4-yl)decandiat (Substanzbeispiel 4)

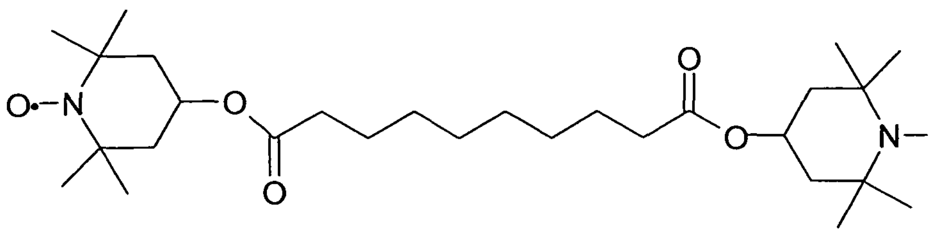

Es werden 28.5 g (166 mmol) 4-Hydroxy-2,2,6,6-tetramethylpiperidin-1-Oxyl (freies Radikal) und 250 mg (2.05 mmol) 4-(Dimethylamino)-pyridin in 300 ml entgastem Dichlormethan gelöst und mit 50.0 ml (361 mmol) Triethylamin versetzt. Es wird nachentgast und auf 0°C gekühlt und es werden 10 g (41.4 mmol) Sebacinsäure-chlorid gelöst in 100 ml entgastem Dichlormethan bei 0-5°C zugetropft und für 18 h bei Raumtemperatur gerührt. Nach vollständigem Umsatz wird unter Eiskühlung mit Wasser und HCl (pH = 4 - 5) versetzt und für 30 min. nachgerührt. Die organische Phase wird abgetrennt und die Wasserphase wird mit Dichlormethan nachextrahiert und die vereinigten Phasen mit gesättigter NaCl-Lösung gewaschen und über $Na_2SO_4$ getrocknet, filtriert und eingeengt. Man erhält 24.4 g einer roten Flüssigkeit,

die zusammen über 100 g $Al_2O_3$ basisch und 500 g Kieselgel mit Dichlormethan / Methyltertbuthylether (95 / 5) gefrittet werden. Man erhält orangene Kristalle, die in entgastem Acetonitril bei 50°C gelöst und bei -25°C kristallisiert werden. Man erhält das Produkt als orangene Kristalle mit einer HPLC-Reinheit von 99.9%.

Synthesebeispiel 3: Synthese von Bis(2,2,6,6-tetramethyl-4-piperidyl)-N,N'-dioxylbutandiol (Substanzbeispiel 7)

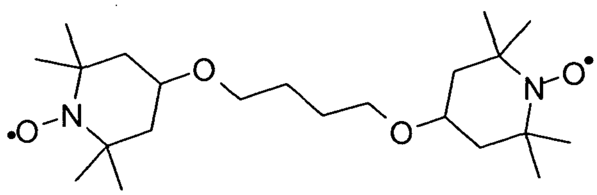

15,0g (60 %-ig in Mineralöl, 375 mmol) NaH werden unter Schutzgas mit ausreichend Pentan versetzt und absetzen gelassen. Der Pentan-überstand wird abpipetiert und vorsichtig unter Kühlung mit Isopropanol gequencht. Das gewaschene NaH wird nun vorsichtig mit 100 ml THF versetzt. Die Reaktionsmischung wird auf 55°C erhitzt und tropfenweise vorsichtig mit einer Lösung aus 50,0 g (284 mmol) 4-Hydroxy-2,2,6,6-tetramethylpiperidin-1-oxyl in 400 ml THF versetzt. Der entstehende Wasserstoff wird direkt abgeführt. Es wird nach vollständiger Zugabe der Lösung über Nacht (16 h) bei 60°C nachgerührt. Die Reaktionsmischung wird anschließend auf 5°C gekühlt und portionsweise mit 1,4-Butandioldimethylsulfonat versetzt. Anschließend wird die Mischung langsam auf 60°C erhitzt und für 16 h bei dieser Temperatur gerührt. Nach vollständigem Umsatz wird auf RT abgekühlt und unter Kühlung mit 200 ml 6 %-iger Ammoniaklösung in Wasser versetzt und 1 h lang gerührt. Anschließend wird die organische Phase abgetrennt, die wässrige Phase mit Methyl-tertbutylether nachgewaschen, die vereinigten organischen Phasen mit ges. NaCl-Lösung gewaschen, getrocknet und eingeengt. Das Rohprodukt wird über Kieselgel mit Dichlormethan / Methyl-tertbutylether (8:2) gereinigt und aus Acetonitril bei -20°C kristallisiert. Man erhält das Produkt als rosa kristallinen Feststoff mit einer Reinheit von > 99.5 %.

Synthesebeispiel 4: Synthese von Bernsteinsäure bis-[2,2,6,6-tetramethyl-1-(1-phenyl-ethoxy)-piperidin-4-yl]ester (Substanzbeispiel 24)

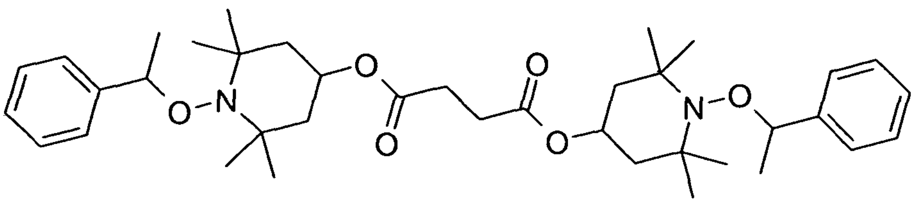

Schritt 4.1: Synthese von 2,2,6,6-Tetramethyl-1-(1-phenyl-ethoxy)-piperidin-4-ol

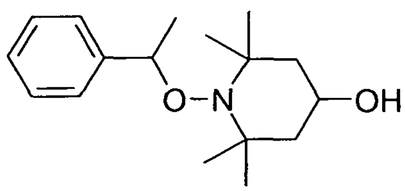

5,0g (29,03 mmol) 4-Hydroxy-2,2,6,6-tetramethylpiperidin-1-oxyl, 7,80 g (58,1 mmol) 2-Phenyl-propionaldehyd und 100,6 mg (1.02 mmol) Kupfer(I)chlorid werden in 20 ml *tert*-Butanol vorgelegt. Dann werden 6,45 ml (58,06 mmol) 35 %-ige Wasserstoffperoxidlösung vorsichtig und langsam zugetropft so, dass die Innentemperatur 30°C nicht überschreitet. Es wird daher während des Zutropfens mittels Eiskühlung gekühlt. Bei der Reaktion entsteht Sauerstoff, der

bei zu schneller Zugabe und zu hoher Temperatur spontan in großem Umfang frei würde. Nach vollständiger Zugabe wird die Reaktionslösung für weitere 16 h bei RT nachgerührt und anschließend mit ausreichend Wasser / Methyltertbutylether versetzt und die organische Phase abgetrennt. Die organische Phase wird mit 10 %- iger Ascorbinsäure peroxidfrei gewaschen und der Peroxidgehalt überprüft. Es wird anschließend mit 10 %-iger NaOH-Lösung, Wasser und ges. NaCl-Lösung gewaschen, über $Na_2SO_4$ getrocknet, filtriert und eingeengt. Das erhaltene Rohprodukt wird mit Heptan / Methyltertbutylether (1:1) über Kieselgel gereinigt und man erhält das Produkt als farblose Kristalle.

Schritt 4.2: Synthese von Bernsteinsäure bis-[2,2,6,6-tetramethyl-1-(1-phenyl-ethoxy)-piperidin-4-yl]ester

1.52g (5.5 mmol) des Produkts der vorherigen Stufe, der Verbindung 2,2,6,6-Tetramethyl-1-(1-phenyl-ethoxy)-piperidin-4-ol, 15,3 mg (0,125 mmol) Dimethylaminopyridin und 1,02 ml (12,6 mmol) getrocknetes Pyridin werden in 10 ml Dichlormethan vorgelegt und auf eine Temperatur im Bereich von 7 bis 10°C gekühlt. Dann werden 0,255 ml (2,199 mmol) Bernsteinsäuredichlorid als solches zugetropft und gegebenenfalls nachdosiert, wenn noch Hydroxyverbindung vorhanden ist. Die Reaktionsmischung wird nach vollständigem Umsatz mit Dichlormethan direkt über Kieselgel filtriert und anschließend mit Heptan / Methyltertbuthylether (1:1) und reinem Methyl-tertbutylether eluiert. Das erhaltene Produkt wird gelöst in Acetonitril mittels präperativer HPLC (2 Chromolithsäulen mit Acetonitril 50 ml/min) gereinigt. Man erhält das Produkt als gelbes Öl mit einer Reinheit von > 99.9 %.

Synthesebeispiel 5: Synthese von Pentansäure 2,2,6,6-tetramethyl-1-(1-phenyl-ethoxy)-piperidin-4-ylester (Substanzbeispiel 31)

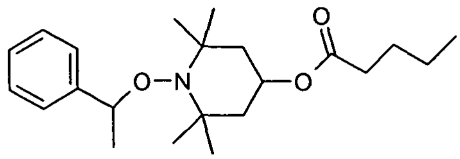

Es werden 2,5g (9,01 mmol) der Verbindung 2,2,6,6-Tetramethyl-1-(1-phenyl-ethoxy)-piperidin-4-ol des Schritts 3.1 und 55,1 mg (0,45 mmol) (4-Dimethylaminopyridin) in 50,0 ml trockenem Dichlormethan gelöst und auf 3°C gekühlt. Es wird bei dieser Temperatur mit 5,47 ml (27,03 mmol) Valeriansäureanhydrid versetzt und 14 h bei Raumtemperatur gerührt. Nach vollständigem Umsatz wird vorsichtig auf Eiswasser gegossen, mit 2N HCl auf pH 6 eingestellt und die organische Phase angetrennt. Die wässrige Phase wird mit Dichlormethan extrahiert und die vereinigten organischen Phasen mit gesättigter NaCl-Lösung, einem Gemisch aus Wasser und Triethylamin (300 : 50 ml) gewaschen und über $MgSO_4$ getrocknet, filtriert und eingeengt. Nach Aufreinigung an Kieselgel mit Heptan / Methyltertbuthylether (9:1) erhält man das Produkt als farbloses Öl.

Synthesebeispiel 6: Synthese von Butandisäure-1,4-bis(1-hydroxy-2,2,6,6-tetramethyl-4-piperidinyl)ester (Substanzbeispiel 49)

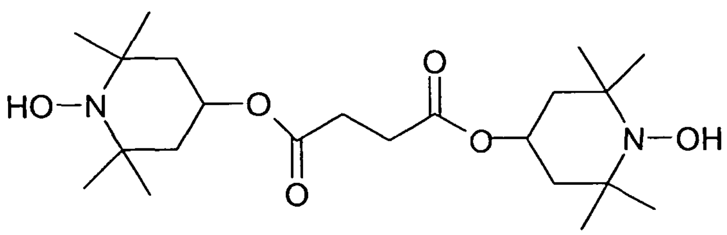

Es werden 40 ml Wasser und 80 ml Dioxan gemischt und mittels Argonstrom sorgfältig entgast. In das Lösungsmittelgemisch werden 2,0 g (4,7 mmol) des freien Radikals des Substanzbeispiels 1 (Synthesebeispiel 1) gelöst und portionsweise mit 4,95 g (28,1 mmol) Ascorbinsäure versetzt. Das Reaktionsgemisch entfärbt sich hierbei und es wird bei 40°C für 18 h unter Schutzgasatmosphäre gerührt. Es wird auf Raumtemperatur abgekühlt und mit 100 ml Wasser versetzt kurz nachgerührt und die anfallenden Kristalle abgesaugt. Das Kristallisat wird in 50 ml entgastem THF

heiß gelöst und die unlöslichen Bestandteile abfiltriert und das Filtrat bei -25°C kristallisiert. Die leicht rosa gefärbten Kristalle werden dann bei Raumtemperatur aus Acetonitril für 18 h ausgerührt und man erhält das Produkt als schwach rosa gefärbte Kristalle mit einer HPLC-Reinheit von 100 %.

Synthesebeispiel 7: Synthese von Decandisäure-1,10-bis(1-hydroxy-2,2,6,6-tetramethyl-4-Piperidinyl)ester (Substanzbeispiel 50)

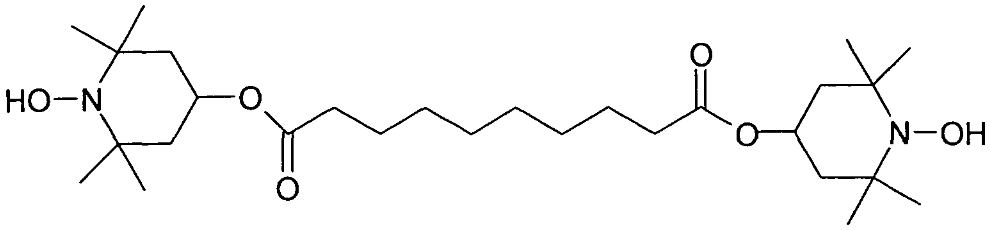

Alle verwendeten Lösungsmittel werden zuvor mittels Argonstrom gründlich entgast. Bei der Aufarbeitung sind Braunglasgeräte zu verwenden. Es wird 1,70 g (3,32 mmol) des freien Radikals des Substanzbeispiels 4 (Synthesebeispiel 2) in 60 ml Dioxan gelöst. Anschließend werden 3,6 g (20 mmol) Ascorbinsäuregelöst in 30 ml Wasser zu der Lösung bei Raumtemperatur zugetropft. Es tritt hierbei eine Entfärbung der Reaktionslösung ein und nach 1 h Rühren bei Raumtemperatur ist die Umsetzung vollständig. Es wird mit 100 mL Dichlormethan extrahiert, die organischen Phase mit Wasser gewaschen und über $Na_2SO_4$ getrocknet, filtriert und eingeengt. Die anfallenden gelben Kristalle werden bei 160°C und $10^{-2}$ mbar für 5 min getrocknet und man erhält ein zähes, langsam kristallisierendes Öl.

Es werden Flüssigkristallmischungen mit den Zusammensetzungen und den Eigenschaften wie in den folgenden Tabellen angegeben hergestellt und untersucht.

Beispiele 1.1.1 bis 1.4.2 und Vergleichsbeispiele 1.1.0 bis 1.4.0:

Die folgende Hostmischung (H-1) wird hergestellt und untersucht.

| Mischung H-1 | | | | | | |
|---|---|---|---|---|---|---|
| Zusammensetzung | | | Physikalische Eigenschaften | | | |
| Verbindung | | Konzentration | T(N, I) | = | 74,5 | °C |
| No. | Abkürzung | /Massen-% | $n_e$(20°C, 589 nm) | = | 1,5628 | |
| 1 | CY-3-O4 | 12,0 | $\Delta$n(20°C, 589 nm) | = | 0,0845 | |
| 2 | CY-5-O4 | 12,0 | $\varepsilon_\perp$(20°, 1 kHz) | = | 7,5 | |
| 3 | CCY-2-1 | 12,0 | $\Delta\varepsilon$(20°, 1 kHz) | = | -3,9 | |
| 4 | CCY-3-1 | 8,0 | $\gamma_1$(20°C) | = | n.z.b. | mPa·s |
| 5 | CCY-2-O2 | 12,0 | $k_{11}$(20°C) | = | n.z.b. | pN |
| 6 | CCY-3-O2 | 12,0 | $k_{33}$(20°C) | = | n.z.b. | pN |
| 7 | CCY-5-O2 | 8,0 | $V_0$(20°C) | = | n.z.b. | V |
| 8 | CC-3-4 | 8,0 | | | | |
| 10 | CP-5-3 | 16,0 | | | | |
| Σ | | 100,0 | | | | |
| Bemerkung: n.z.b.: noch zu bestimmen | | | | | | |

Die Mischung H-1 wird zunächst in vier Teile geteilt. Jedem der vier Teile wird eine andere Verbindung der Formel II zugesetzt und wie in der folgenden Tabelle gezeigt.

| Mischung | M-1-1 | M-1-2 | M-1-3 | M-1-4 |
|---|---|---|---|---|
| Bestandteil | Konzentration/Massen-% | | | |
| H-1 | 80,0 | 90,0 | 90,0 | 90,0 |
| CY-1V-O2 | 20,0 | 0,0 | 0,0 | 0,0 |
| CPY-1V-O1 | 0,0 | 10,0 | 0,0 | 0,0 |
| CCY-1V-O4 | 0,0 | 0,0 | 10,0 | 0,0 |
| CCY-V2-O2 | 0,0 | 0,0 | 0,0 | 10,0 |
| Σ | 100.0 | 100.0 | 100,0 | 100,0 |

Dann wird jede dieser Mischungen nochmals in drei Teile geteilt. Der erste Teil wird als solcher untersucht. Den zwei weiteren Teilen der Mischung wird alternativ 200 ppm der Verbindung

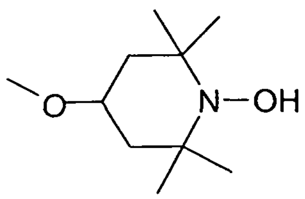

„NOH“

zugesetzt (=> "M-1-n-1"), bzw. 200 ppm der Verbindung des Synthesebeispiels 1: (also des Substanzbeispiels 1)

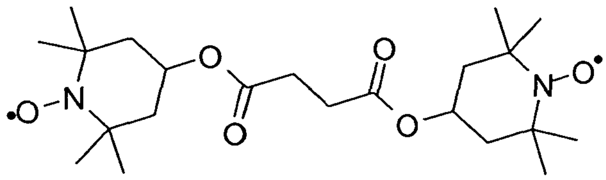

,

zugesetzt (=> "M-1-n-2"). Alle Mischungen werden, wie im weiteren beschrieben, untersucht.

Zunächst wird die Stabilität der Voltage Holding Ratio der Mischungen (CM-1.n) als solche bestimmt. Die vier Mischungen (CM-1-n, n ∈ {1;2;3;4}) werden, ebenso wie die vier Mischungen mit der Verbindung NOH (M-1-n-1, n ∈ {1;2;3;4}) und die vier Mischungen mit der Verbindung des Substanzbeispiels 1 (M-1-n-2, n ∈ {1;2;3;4}), in einer Testzelle mit einem Orientierungsmaterial für homeotrope Ausrichtung und flächigen ITO-Elektroden auf ihre Stabilität gegen die Beleuchtung mit einer Kaltkathoden (CCFL)-LCD-Hintergrundbeleuchtung (Englisch: "backlight") untersucht. Dazu werden entsprechende Testzellen 750 Stunden der Beleuchtung ausgesetzt. Danach wird jeweils die Voltage Holding Ratio nach 5 Minuten bei einer Temperatur von 100°C bestimmt. Die Ergebnisse sind unten Tabelle 1 zusammengestellt. Hier, wie im Folgenden, werden für jede einzelne Mischung jeweils sechs Testzellen gefüllt und untersucht. Die angegeben Werte sind der Mittelwert der sechs Einzelwerte und deren Standardabweichung (σ), auch für den Fall, dass die Standardabweichung kleiner ist als die oben angegebene Genauigkeit der Messwerte.

Die relativen Abweichungen der "Voltage Holding Ratio"-Werte bei verschiedenen Meßserien, liegt typischer Weise im Bereich von ca. 3 bis 4 %, wenn nicht anders angegeben.

Die üblicherweise durch die Belastung verursachte Abnahme der Voltage Holding Ratio (ΔVHR) wird, wie oben im Text beschrieben, bestimmt.

Tabelle 1

| Bsp. | Nr. | Mischung | Stabilisator | C(Stab.) / ppm | VHR(t) / % | |
|---|---|---|---|---|---|---|
| | | | | | t=0 h | t=750 h |
| V1.1.0 | 1 | CM-1-1-0 | keiner | 0 | 95 ± 1,0 | 80 ± 2,0 |
| B1.1.1 | 3 | M-1-1-1 | I(N)* | 200 | 88 ± 1,8 | 82 ± 2,3 |
| B1.1.2 | 4 | M-1-1-2 | I(1)* | 200 | n.z.b. | n.z.b. |

(fortgesetzt)

| Bsp. | Nr. | Mischung | Stabilisator | C(Stab.) / ppm | VHR(t) / % | |
|---|---|---|---|---|---|---|
| | | | | | t=0 h | t=750 h |
| V1.2.0 | 5 | CM-1-2-0 | keiner | 0 | 98 ± 0,3 | 88 ± 1,0 |
| B1.2.1 | 7 | M-1-2-1 | I(N)* | 200 | 93 ± 1,0 | 91 ± 0,8 |
| B1.2.2 | 8 | M-1-2-2 | I(1)* | 200 | n.z.b. | n.z.b. |
| V1.3.0 | 9 | CM-1-3-0 | keiner | 0 | 94 ± 1,7 | 88 ± 1,1 |
| B1.3.1 | 11 | M-1-3-1 | I(N)* | 200 | 92 ± 2,3 | 91 ± 0,6 |
| B1.3.2 | 12 | M-1-3-2 | I(1)* | 200 | n.z.b. | n.z.b. |
| V1.4.0 | 13 | CM-1-4-0 | keiner | 0 | 96 ± 0,7 | 85 ± 1,3 |
| B1.4.1 | 15 | M-1-4-1 | I(N)* | 200 | 95 ± 1,0 | 89 ± 1,1 |
| B1.4.2 | 16 | M-1-4-2 | I(1)* | 200 | n.z.b. | n.z.b. |
| Bemerkungen: I(N)*: Verbindung NOH<br>I(1)*: Verbindung des Synthesebeispiels 1<br>n.z.b.: noch zu bestimmen | | | | | | |

Die Verbindung des Synthesebeispiels 1 weisen bereits in relativ niedrigen Konzentrationen deutlich stabilisierende Eigenschaften auf. Auch die Verbindung NOH zeigt eine gewisse Stabilisierung. Für die Verbindung des Synthesebeispiels 1 sieht das Verhalten jedoch wesentlich günstiger aus.

Beispiel 2:

Die folgende Mischung (M-2), die 11,5 % einer der Formel II mit einer Alkenylendgruppe enthält, wird hergestellt und untersucht.

| Mischung M-2 | | | | | | |
|---|---|---|---|---|---|---|
| Zusammensetzung | | | Physikalische Eigenschaften | | | |
| Verbindung | | Konzentration | T(N,I) | = | 75,5 | °C |
| No. | Abkürzung | /Massen-% | $\Delta n$(20°C, 589 nm) | = | 0,1074 | |
| 1 | PY-V2-O2 | 11,5 | $\Delta\varepsilon$(20°, 1 kHz) | = | -3,1 | |
| 2 | CY-3-O2 | 11,0 | $\gamma_1$(20°C) | = | 87 | mPa·s |
| 3 | CCY-3-O1 | 9,0 | $k_{11}$(20°C) | = | 13,0 | pN |
| 4 | CCY-3-O2 | 4,0 | $k_{33}$(20°C) | = | 14,7 | pN |
| 5 | CPY-2-02 | 12,0 | $V_0$(20°C) | = | 2,29 | V |
| 6 | CPY-3-O2 | 9,0 | | | | |
| 7 | CC-3-V | 37,0 | | | | |
| 8 | CPP-3-2 | 6,0 | | | | |
| 9 | PPGU-3-F | 0,5 | | | | |
| Σ | | 100,0 | | | | |

Die Mischung M-2 wird in fünf Teile geteilt und wie im weiteren beschrieben untersucht.

Der erste Teil der Mischung M-2 wird, wie bei Beispiel 1, ohne Zugabe einer weiteren Verbindung untersucht. Den weiteren vier Teilen der Mischung M-2 werden alternativ 100 ppm der Verbindung des Synthesebeispiels 1: (also des Substanzbeispiels 1)

Es werden Flüssigkristallmischungen mit den Zusammensetzungen und den Eigenschaften wie in den folgenden Tabellen angegeben hergestellt und untersucht.

Dabei stellen die Mischungen M-1-1-1, M-1-2-1, M-1-3-1 und M-1-4-1, der Beispiele B1.1.1, B1.2.1, B1.3.1 bzw. B1.4.1, die Mischungen M-2-3 und M-2-4 der Beispiele B2.3 und B2.4, die Mischungen M-3-3 und M-3-4 der

Beispiele B3.3 und B3.4, die Mischungen M-4-3 und M-4-4 der Beispiele B4.3 und B4.4, die Mischungen M-5-3 und M-5-4 der Beispiele B5.3 und B5.4, sowie die Mischungen M-6-3 und M-6-4 der Beispiele B6.3 und B6.4 Vergleichsbeispiel dar.

Beispiele 1.1.1 bis 1.4.2 und Vergleichsbeispiele 1.1.0 bis 1.4.0:

Die folgende Hostmischung (H-1) wird hergestellt und untersucht.

Tabelle 2

| Bsp. | Nr. | Mischung | Stabilisator | C(Stab.) / ppm | VHR(t) / % | |
|---|---|---|---|---|---|---|
| | | | | | t = 0 h | t = 500 h |
| V2.0 | 1 | M-2 | keiner | 0 | 93,9 ± 0,8 | 44 ± 2,0 |
| B2.1 | 3 | M-2-1 | I(1)* | 100 | 91,5 ± 0,8 | 64 ± 1,1 |
| B2.2 | 4 | M-2-2 | I(2)* | 50 | 91,9 ± 1,0 | 58 ± 1,5 |
| B2.3 | 5 | M-2-3 | I(3)* | 250 | 93,1 ± 1,1 | 67 ± 0,5 |
| B2.4 | 6 | M-2-4 | I(N)* | 250 | 91,3 ± 1,5 | 60 ± 1,6 |
| Bemerkungen: I(1)*: Verbindung des Synthesebeispiels 1<br>I(2)*: Verbindung des Synthesebeispiels 2<br>I(3)*: Verbindung des Synthesebeispiels 7<br>I(N)*: Verbindung NOH | | | | | | |

Alle hier verwendeten Stabilisatoren, und insbesondere die Verbindungen der Formel I, verbessern deutlich die Stabilität der verwendeten Mischung. Dieses gilt insbesondere für die Werte nach den Belastungstests. Der Stabilisator I(3) zeigt hierbei eine besonders hohe Aktivität und einen unverändert hohen VHR vor der Belastung durch das "backlight". Ohne den Einsatz von Stabilisatoren könnte die Mischung M-2 nur begrenzt in einem LCD eingesetzt werden. Durch Verwendung der beschriebenen Stabilisatoren, insbesondere der Verbindungen der Formel I, gelingt eine Erhöhung der VHR auf einen Wert, der den Einsatz in den meisten entsprechenden LCDs ermöglicht.

Z.B. die Verbindungen der Synthesebeispiele 1 und 7 weisen in einer Konzentration von 100 ppm bzw. von 250 ppm eine allen anderen hier untersuchten Stabilisatoren überlegene Stabilisierungsaktivität auf. Dieses führt zu einer Reduzierung des Auftretens von Imagesticking bei Belastung durch die Hintergrundbeleuchtung.

Beispiel 3:

Die folgende Mischung (M-3), die 9 % einer Verbindung der Formel II mit einer Alkenylendgruppe enthält, wird hergestellt und untersucht.

| Mischung M-3 | | | | | | |
|---|---|---|---|---|---|---|
| Zusammensetzung | | | Physikalische Eigenschaften | | | |
| Verbindung | | Konzentration | | | | |
| No. | Abkürzung | /Massen-% | | | | |
| 1 | CY-V-O2 | 9,0 | T(N, I) | = | 75,5 | °C |
| 2 | PY-3-O2 | 12,5 | $\Delta n$(20°C, 589 nm) | = | 0,1080 | |
| 3 | CCY-3-O2 | 7,0 | $\Delta\varepsilon$(20°, 1 kHz) | = | -3,1 | |
| 4 | CCY-4-O2 | 8,0 | $\gamma_1$(20°C) | = | 88 | mPa·s |
| 5 | CPY-2-O2 | 10,0 | $k_{11}$(20°C) | = | 12,9 | pN |
| 6 | CPY-3-O2 | 10,0 | $k_{33}$(20°C) | = | 14,5 | pN |
| 7 | CC-3-V | 36,5 | $V_0$(20°C) | = | 2,27 | V |
| 8 | CPP-3-2 | 6,5 | | | | |
| 9 | PPGU-3-F | 0,5 | | | | |
| Σ | | 100,0 | | | | |

Die Mischung M-3 wird, wie bei Beispiel 2 beschrieben, in fünf Teile geteilt und, wie dort beschrieben, behandelt und untersucht. Die Ergebnisse sind in der folgenden Tabelle angegeben.

Tabelle 3

| Bsp. | Nr. | Mischunq | Stabilisator | c(Stab.) / ppm | VHR(t) / % | |
|---|---|---|---|---|---|---|
| | | | | | t = 0 h | t = 500 h |
| V3.0 | 1 | M-3 | keiner | 0 | 93,6 ± 0,8 | 51 ± 1,5 |
| B3.1 | 3 | M-3-1 | I(1)* | 100 | 92,4 ± 0,8 | 65 ± 2,1 |
| B3.2 | 4 | M-3-2 | I(2)* | 50 | 92,4 ± 0,8 | 60 ± 1,4 |
| B3.3 | 5 | M-3-3 | I(3)* | 250 | 92,7 ± 0,7 | 68 ± 1,5 |
| B3.4 | 6 | M-3-4 | I(N)* | 250 | 92,1 ± 0,9 | 61 ± 1,2 |
| Bemerkungen: I(1)*: Verbindung des Synthesebeispiels 1<br>I(2)*: Verbindung des Synthesebeispiels 2<br>I(3)*: Verbindung des Synthesebeispiels 7<br>I(N)*: Verbindung NOH | | | | | | |

Alle hier verwendeten Stabilisatoren, und insbesondere die Verbindungen der Formel I, verbessern deutlich die Stabilität der verwendeten Mischungen. Dieses gilt insbesondere für die Werte nach den Belastungstests. Der Stabilisator I(3) zeigt hierbei eine besonders hohe Aktivität und einen unverändert hohen VHR vor der Belastung durch das "backlight". Ohne den Einsatz von Stabilisatoren könnte die Mischung M-3 nur begrenzt in einem LCD eingesetzt werden. Durch Verwendung der beschriebenen Stabilisatoren, insbesondere der Verbindungen der Formel I, gelingt eine Erhöhung der VHR auf einen Wert, der den Einsatz in den meisten entsprechenden LCDs ermöglicht.

Auch hier, wie bereits bei Beispiel 2, weisen die Verbindungen der Synthesebeispiele 1 und 7 in einer Konzentration von 100 ppm bzw. von 250 ppm eine allen anderen hier untersuchten Stabilisatoren überlegene Stabilisierungsaktivität auf.

Beispiel 4:

Die folgende Mischung (M-4), die 5 % einer Verbindung der Formel II mit einer Alkenylendgruppe enthält, wird hergestellt und untersucht.

| Mischung M-4 | | | | | | |
|---|---|---|---|---|---|---|
| Zusammensetzung | | | Physikalische Eigenschaften | | | |
| Verbindung | | Konzentration | T(N, I) | = | 74,5 | °C |
| No. | Abkürzung | /Massen-% | Δn(20°C, 589 nm) | = | 0,1086 | |
| 1 | CCY-V2-O2 | 5,0 | Δε(20°, 1 kHz) | = | -3,1 | |
| 2 | CY-3-O2 | 9,0 | $\gamma_1$(20°C) | = | 89 | mPa·s |
| 3 | PY-3-O2 | 13,5 | $k_{11}$(20°C) | = | 13,2 | pN |
| 4 | CCY-3-O1 | 9,0 | $k_{33}$(20°C) | = | 14,7 | pN |
| 5 | CPY-2-O2 | 10,0 | $V_0$(20°C) | = | 2,29 | V |
| 6 | CPY-3-O2 | 10,0 | | | | |
| 7 | CC-3-V | 36,5 | | | | |
| 8 | CPP-3-2 | 6,5 | | | | |
| 9 | PPGU-3-F | 0,5 | | | | |
| Σ | | 100,0 | | | | |

Die Mischung M-4 wird, wie bei Beispiel 2 beschrieben, in fünf Teile geteilt und, wie dort beschrieben, behandelt und untersucht. Die Ergebnisse sind in der folgenden Tabelle angegeben.

| Bsp. | Nr. | Mischung | Stabilisator | c(Stab.) / ppm | VHR(t) / % | |
|---|---|---|---|---|---|---|
| | | | | | t = 0 h | t = 500 h |
| V4.0 | 1 | M-4 | keiner | 0 | n.z.b. | n.z.b. |
| B4.1 | 3 | M-4-1 | I(1)* | 100 | n.z.b. | n.z.b. |
| B4.2 | 4 | M-4-2 | I(2)* | 50 | n.z.b. | n.z.b. |
| B4.3 | 5 | M-4-3 | I(3)* | 250 | n.z.b. | n.z.b. |
| B4.4 | 6 | M-4-4 | I(N)* | 250 | n.z.b. | n.z.b. |
| Bemerkungen: I(1)*: Verbindung des Synthesebeispiels 1<br>I(2)*: Verbindung des Synthesebeispiels 2<br>I(3)*: Verbindung des Synthesebeispiels 7<br>I(N)*: Verbindung NOH<br>n.z.b.: noch zu bestimmen | | | | | | |

Auch bei diesem Beispiel werden ähnlich gute Ergebnisse erzielt, die mit denen der vorhergehenden Beispielen vergleichbar sind.

Beispiel 5:

Die folgende Mischung (M-5), die 8 % einer Verbindung der Formel II mit einer Alkenylendgruppe enthält, wird hergestellt und untersucht.

| Mischung M-5 | | | | | | |
|---|---|---|---|---|---|---|
| Zusammensetzung | | | Physikalische Eigenschaften | | | |
| Verbindung | | Konzentration | T(N, I) | = | 75,0 | °C |
| No. | Abkürzung | /Massen-% | Δn(20°C, 589 nm) | = | 0,1079 | |
| 1 | CPY-V-O2 | 8,0 | Δε(20°, 1 kHz) | = | -3,1 | |
| 2 | CY-3-O2 | 8,0 | $\gamma_1$(20°C) | = | 89 | mPa·s |
| 3 | PY-3-O2 | 13,5 | $k_{11}$(20°C) | = | 13,2 | pN |
| 4 | CCY-3-O1 | 5,0 | $k_{33}$(20°C) | = | 14,4 | pN |
| 5 | CCY-3-O2 | 6,0 | $V_0$(20°C) | = | 2,26 | V |
| 6 | CCY-4-O2 | 5,0 | | | | |
| 7 | CPY-2-O2 | 11,0 | | | | |
| 8 | CC-3-V | 36,5 | | | | |
| 9 | CPP-3-2 | 6,5 | | | | |
| 10 | PPGU-3-F | 0,5 | | | | |
| Σ | | 100,0 | | | | |

Die Mischung M-5 wird, wie bei Beispiel 2 beschrieben, in fünf Teile geteilt und, wie dort beschrieben, behandelt und untersucht. Die Ergebnisse sind in der folgenden Tabelle angegeben.

| Bsp. | Nr. | Mischung | Stabilisator | c(Stab.) / ppm | VHR(t) / % | |
|---|---|---|---|---|---|---|
| | | | | | t = 0 h | t = 500 h |
| V5.0 | 1 | M-5 | keiner | 0 | 93,9 ± 0,7 | 49 ± 1,3 |
| B5.1 | 3 | M-5-1 | I(1)* | 100 | 91,1 ± 0,6 | 65 ± 1,0 |
| B5.2 | 4 | M-5-2 | I(2)* | 50 | 92,2 ± 1,2 | 60 ± 2,5 |
| B5.3 | 5 | M-5-3 | I(3)* | 250 | 93,1 ± 0,7 | 69 ± 1,0 |

(fortgesetzt)

| Bsp. | Nr. | Mischung | Stabilisator | c(Stab.) / ppm | VHR(t) / % | |
|---|---|---|---|---|---|---|
| | | | | | t = 0 h | t = 500 h |
| B5.4 | 6 | M-5-4 | I(N)* | 250 | 92,4 ± 0,3 | 62 ± 2,3 |
| Bemerkungen: I(1)*: Verbindung des Synthesebeispiels 1<br>I(2)*: Verbindung des Synthesebeispiels 2<br>I(3)*: Verbindung des Synthesebeispiels 7<br>I(N)*: Verbindung NOH | | | | | | |

Alle hier verwendeten Stabilisatoren, und insbesondere die Verbindungen der Formel I, verbessern deutlich die Stabilität der verwendeten Mischungen. Dieses gilt insbesondere für die Werte nach den Belastungstests. Der Stabilisator I(3) zeigt hierbei eine besonders hohe Aktivität und einen unverändert hohen VHR vor der Belastung durch das "backlight". Ohne den Einsatz von Stabilisatoren könnte die Mischung M-5 nur begrenzt in einem LCD eingesetzt werden. Durch Verwendung der beschriebenen Stabilisatoren, insbesondere der Verbindungen der Formel I, gelingt eine Erhöhung der VHR auf einen Wert, der den Einsatz in den meisten entsprechenden LCDs ermöglicht.

Auch hier, wie bereits bei den drei vorhergehenden Beispielen, weisen die Verbindungen der Synthesebeispiele 1 und 7 in einer Konzentration von 100 ppm bzw. von 250 ppm eine allen anderen hier untersuchten Stabilisatoren überlegene Stabilisierungsaktivität auf.

Beispiel 6:

Die folgende Mischung (M-6), die insgesamt 39,5 % an Verbindungen der Formel II mit einer Alkenylendgruppe enthält, wird hergestellt und untersucht.

| Mischung M-6 | | | | | | | |
|---|---|---|---|---|---|---|---|
| Zusammensetzung | | | Physikalische Eigenschaften | | | | |
| Verbindung | | Konzentration | | | | | |
| No. | Abkürzung | /Massen-% | | | | | |
| 1 | CY-V-O2 | 9,0 | T(N, I) | = | 74,5 | °C |
| 2 | PY-V2-O2 | 12,0 | $\Delta n$(20°C, 589 nm) | = | 0,1086 | |
| 3 | CCY-V2-O2 | 8,0 | $\Delta\varepsilon$(20°, 1 kHz) | = | -3,1 | |
| 4 | CPY-V-02 | 10,5 | $\gamma_1$(20°C) | = | 89 | mPa·s |
| 5 | CCY-3-O1 | 9,0 | $k_{11}$(20°C) | = | 13,2 | pN |
| 6 | CPY-2-O2 | 8,0 | $k_{33}$(20°C) | = | 14,7 | pN |
| 7 | CC-3-V | 36,5 | $V_0$(20°C) | = | 2,29 | V |
| 8 | CPP-3-2 | 6,5 | | | | |
| 9 | PPGU-3-F | 0,5 | | | | |
| Σ | | 100,0 | | | | |

Die Mischung M-6 wird, wie bei Beispiel 2 beschrieben, in fünf Teile geteilt und, wie dort beschrieben, behandelt und untersucht. Die Ergebnisse sind in der folgenden Tabelle angegeben.

| Bsp. | Nr. | Mischung | Stabilisator | c(Stab.) / ppm | VHR(t) / % | |
|---|---|---|---|---|---|---|
| | | | | | t =0 h | t = 500 h |
| V6.0 | 1 | M-6 | keiner | 0 | 92,3 ± 0,7 | 40 ± 3,5 |
| B6.1 | 3 | M-6-1 | I(1)* | 100 | 89,4 ± 0,6 | 59 ± 1,6 |
| B6.2 | 4 | M-6-2 | I(2)* | 50 | 90,2 ± 1,0 | 55 ± 1,5 |
| B6.3 | 5 | M-6-3 | I(3)* | 250 | 92,6 ± 0,8 | 64 ± 1,2 |

(fortgesetzt)

| Bsp. | Nr. | Mischung | Stabilisator | c(Stab.) / ppm | VHR(t) / % | |
|---|---|---|---|---|---|---|
| | | | | | t =0 h | t = 500 h |
| B6.4 | 6 | M-6-4 | I(N)* | 250 | 91,4 ± 0,6 | 57 ± 1,8 |
| Bemerkungen: I(1)*: Verbindung des Synthesebeispiels 1<br>I(2)*: Verbindung des Synthesebeispiels 2<br>I(3)*: Verbindung des Synthesebeispiels 7<br>I(N)*: Verbindung NOH | | | | | | |

Auch bei diesem Beispiel werden ähnlich gute Ergebnisse erzielt wie bei den vorhergehenden Beispielen.

## Patentansprüche

1. Flüssigkristallines Medium enthaltend

a) eine oder mehrere Verbindungen der Formel I

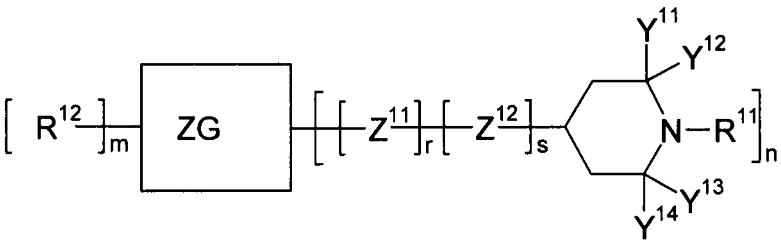

I

worin

n 1, 2, 3 oder 4,
m (4-n),

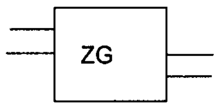

ein organischer Rest mit 4 Bindungsstellen
$Z^{11}$ und $Z^{12}$ unabhängig voneinander -O-, -(C=O)-, -(N-$R^{14}$)- oder eine Einfachbindung, jedoch nicht beide gleichzeitig -O-,
r und s unabhängig voneinander 0 oder 1,
$Y^{11}$ bis $Y^{14}$ jeweils unabhängig voneinander Alkyl mit 1 bis 4 C-Atomen und alternativ auch unabhängig voneinander eines oder beide der Paare ($Y^{11}$ und $Y^{12}$) und ($Y^{13}$ und $Y^{14}$) gemeinsam eine zweibindige Gruppe mit 3 bis 6 C-Atomen,
$R^{11}$ O
$R^{12}$ bei jedem Auftreten unabhängig voneinander H, F, $OR^{14}$, $NR^{14}R^{15}$ eine geradkettige oder verzweigte Alkylkette mit 1-20 C-Atomen, in der eine -$CH_2$- -Gruppe oder mehrere -$CH_2$- -Gruppen durch -O- oder -C(=O)-ersetzt sein können, jedoch nicht zwei benachbarte -$CH_2$- -Gruppen durch -O- ersetzt sein können, einen Kohlenwasserstoffrest, der eine Cycloalkyl- oder eine Alkylcycloalkyleinheit enthält, und, in dem eine -$CH_2$--Gruppe oder mehrere -$CH_2$- -Gruppen durch -O- oder -C(=O)- ersetzt sein können, jedoch nicht zwei benachbarte -$CH_2$- -Gruppen durch -O- ersetzt sein können, und in dem ein H-Atom oder mehrere H-Atome durch $OR^{14}$, $N(R^{14})(R^{15})$ oder $R^{16}$ ersetzt sein können, oder ein aromatischer oder heteroaromatischen Kohlenwasserstoffrest, in denen ein H-Atom oder mehrere H-Atome durch $OR^{14}$, $N(R^{14})(R^{15})$ oder $R^{16}$ ersetzt sein können,
$R^{14}$ bei jedem Auftreten unabhängig voneinander eine geradkettige oder verzweigte Alkyl- oder Acylgruppe mit 1 bis 10 C-Atomen oder einen aromatischen Kohlenwasserstoff- oder Carbonsäurerest mit 6-12 C-

Atomen,

$R^{15}$ bei jedem Auftreten unabhängig voneinander eine geradkettige oder verzweigte Alkyl- oder Acylgruppe mit 1 bis 10 C-Atomen oder einen aromatischen Kohlenwasserstoff- oder Carbonsäurerest mit 6-12 C-Atomen,

$R^{16}$ bei jedem Auftreten unabhängig voneinander eine geradkettige oder verzweigte Alkylgruppe mit 1 bis 10 C-Atomen in dem eine $-CH_2-$ -Gruppe oder mehrere $-CH_2-$ -Gruppen durch -O- oder -C(=O)- ersetzt sein können, jedoch nicht zwei benachbarte $-CH_2-$ -Gruppen durch -O- ersetzt sein können,

bedeutet, mit den Maßgaben, dass,
Verbindungen der Formel

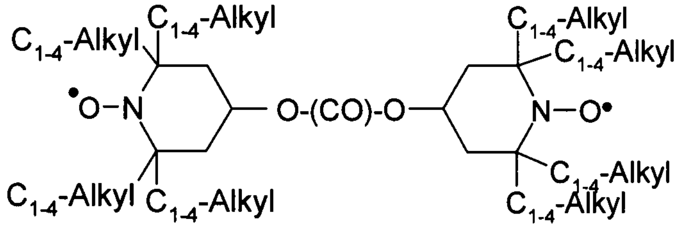

von den Verbindungen der Formel I ausgeschlossen sind, und
im Fall n = 1 und
$-[Z^{11}-]_r-[Z^{12}]_s-$ = -O-, -(CO)-O-, -O-(CO)-, -O-(CO)-O-, $-NR^{14}-$ oder $-NR^{14}-(CO)-$,

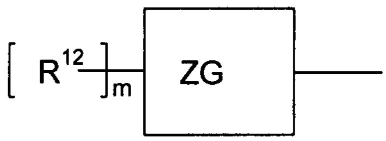

nicht
ein geradkettiges oder verzweigtes Alkyl mit 1 bis 10 C-Atomen, auch Cycloalkyl, Cycloalkylalkyl, oder Alkylcyloalkyl, wobei in allen diesen Gruppen eine oder mehrere $-CH_2-$ Gruppen durch -O- so ersetzt sein können, dass im Molekül keine zwei O-Atome direkt miteinander verbunden sind, bedeutet, und
b) eine oder mehrere Verbindungen der Formel II

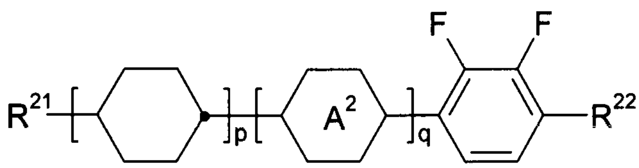

II

worin

$R^{21}$ einen unsubstituierten Alkenylrest mit 2 bis 7 C-Atomen,
$R^{22}$ einen unsubstituierten Alkylrest mit 1 bis 7 C-Atomen, einen unsubstituierten Alkoxyrest mit 1 bis 6 C-Atomen oder einen unsubstituierten Alkenyloxyrest mit 2 bis 6 C-Atomen,

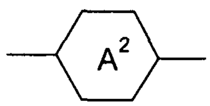

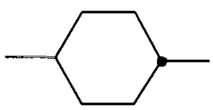

,

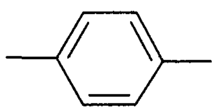

,

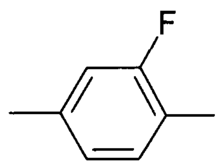

,

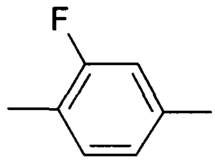

oder

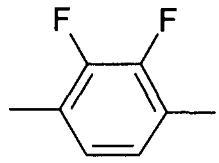

,

p und q jeweils unabhängig voneinander 0, 1 oder 2 und
(p + q) 1, 2, oder 3,

bedeuten.

2. Medium nach Anspruch 1, **dadurch gekennzeichnet, dass** es eine oder mehrere Verbindungen der Formel I ausgewählt aus der Gruppe der Verbindungen der Formeln I-1, I-2, und I-5 bis I-8

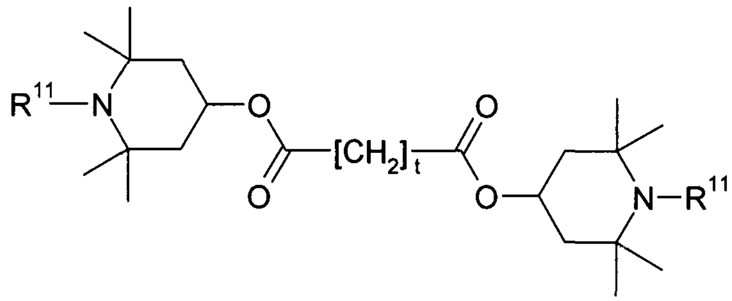

I-1

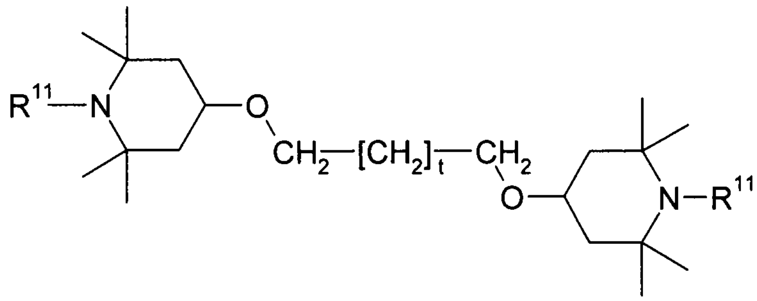

I-2

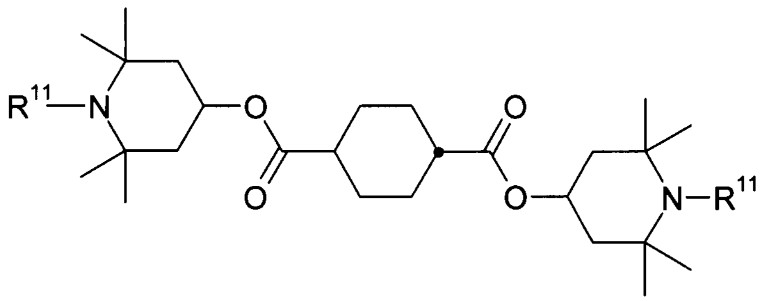

I-5

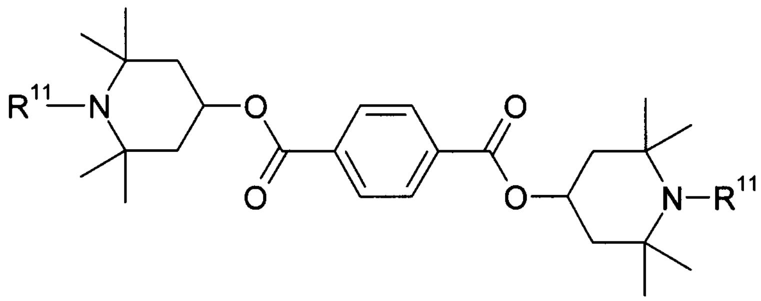

I-6

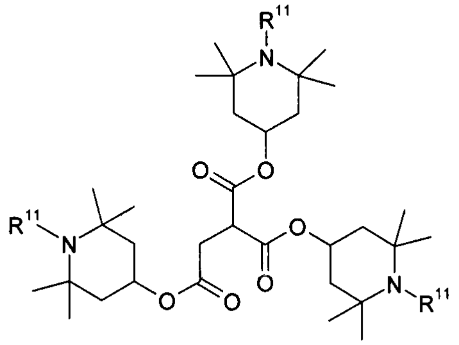

I-7

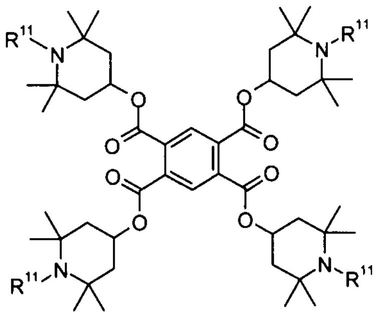

I-8

worin die Parameter die in Anspruch 1 angegebenen Bedeutungen haben und

t eine ganze Zahl von 1 bis12
$R^{17}$ eine geradkettige oder verzweigte Alkylkette mit 1-12 C-Atomen in der eine $-CH_2-$ -Gruppe oder mehrere $-CH_2-$-Gruppen durch -O- oder -C(=O)- ersetzt sein können, jedoch nicht zwei benachbarte $-CH_2-$ -Gruppen durch -O- ersetzt sein können oder ein aromatischer oder heteroaromatischer Kohlenwasserstoffrest in denen ein H-Atom oder mehrere H-Atome durch $OR^{14}$, $N(R^{14})(R^{15})$ oder $R^{16}$ ersetzt sein können,

bedeuten, enthält.

3. Medium nach Anspruch 1 oder 2, **dadurch gekennzeichnet, dass** es eine oder mehrere Verbindungen der Formel I ausgewählt aus der Gruppe der Verbindungen der Formeln I-1a-1 bis I-2a-2 und I-5a-1 bis I-8a-1

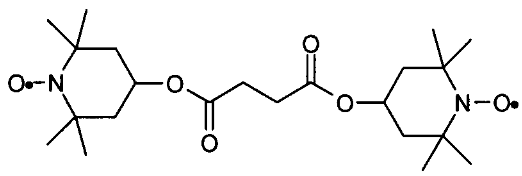

I-1a-1

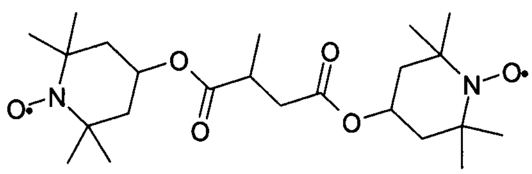

I-1a-2

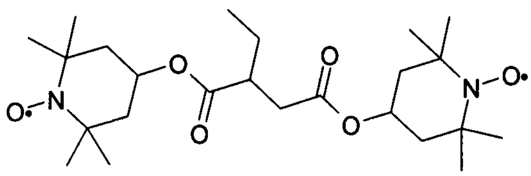

I-1a-3

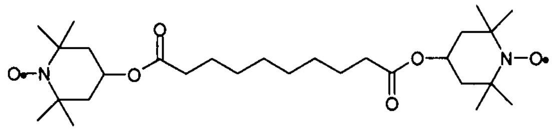

I-1a-4

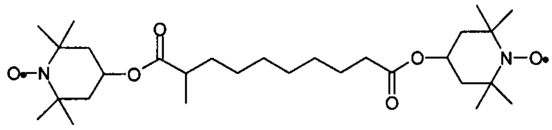

I-1a-5

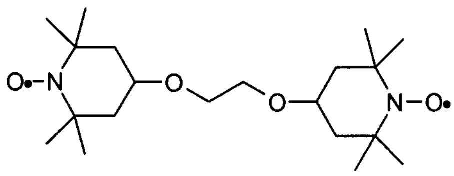

I-2a-1

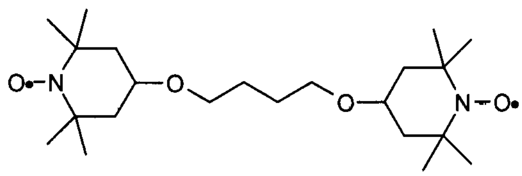

I-2a-2

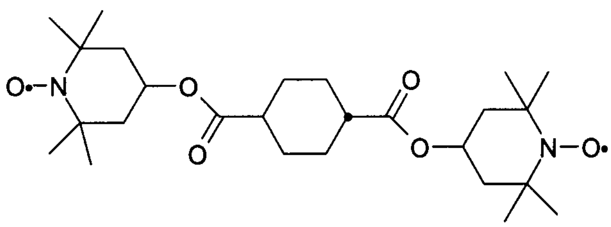

I-5a-1

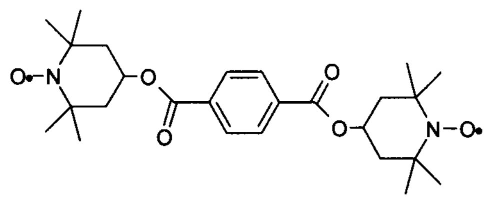

I-6a-1

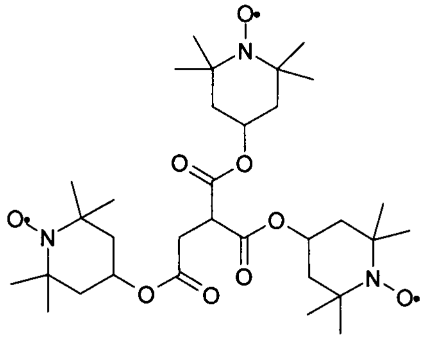

I-7a-1

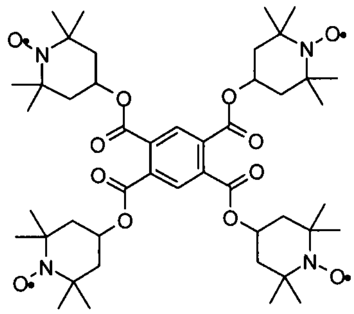

I-8a-1 ,

enthält.

**4.** Flüssigkristallines Medium nach Anspruch 3, **dadurch gekennzeichnet, dass** die Verbindung der Formel I-1a-1

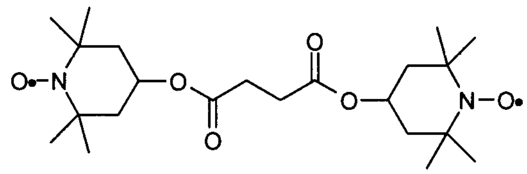

I-1a-1

enthält.

**5.** Flüssigkristallines Medium nach mindestens einem der Ansprüche 1 bis 4, **dadurch gekennzeichnet, dass** es eine oder mehrere Verbindungen der Formel II ausgewählt aus der Gruppe der Verbindungen der Formeln II-1 bis II-4

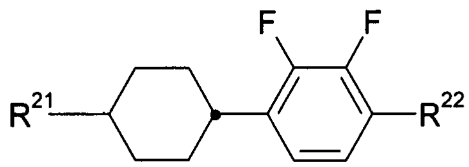

II-1

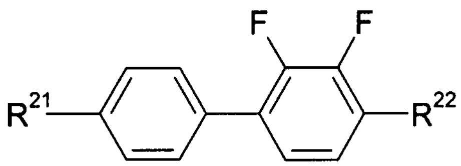

II-2

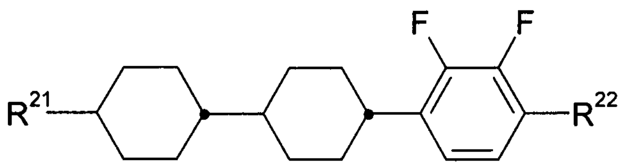

II-3

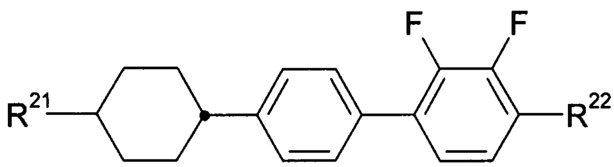

II-4

worin die Parameter die jeweiligen in Anspruch 1 gegebenen Bedeutungen haben, enthält.

6. Flüssigkristallines Medium nach mindestens einem der Ansprüche 1 bis 5, **dadurch gekennzeichnet, dass** es zusätzlich

c) eine oder mehrere Verbindungen ausgewählt aus der Gruppe der Verbindungen der Formeln III-1 bis III-4

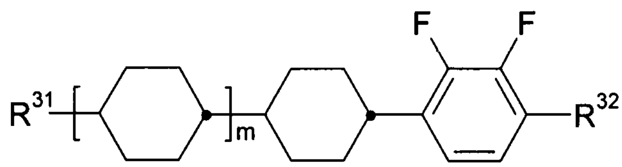

III-1

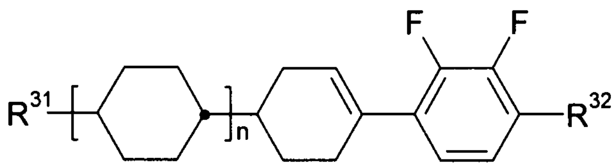

III-2

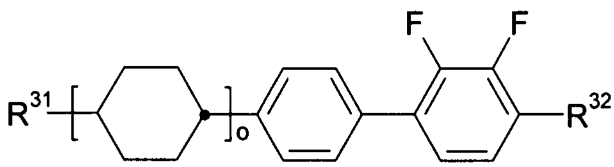

III-3

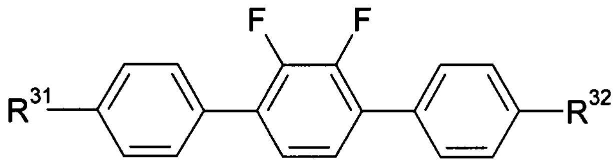

III-4

worin

$R^{31}$ einen unsubstituierten Alkylrest mit 1 bis 7 C-Atomen,
$R^{32}$ einen unsubstituierten Alkylrest mit 1 bis 7 C-Atomen, einen unsubstituierten Alkoxyrest mit 1 bis 6 C-Atomen oder einen unsubstituierten Alkenyloxyrest mit 2 bis 6 C-Atomen, und
m, n und o jeweils unabhängig voneinander 0 oder 1

bedeuten, enthält.

**7.** Medium nach mindestens einem der Ansprüche 1 bis 6, **dadurch gekennzeichnet, dass** die Gesamtkonzentration der Verbindungen der Formel I, im Gesamtmedium 1 ppm oder mehr bis 1.000 ppm oder weniger beträgt.

**8.** Medium nach mindestens einem der Ansprüche 1 bis 7, **dadurch gekennzeichnet, dass** es eine zusätzlich

d) eine oder mehrere Verbindungen der Formel IV,

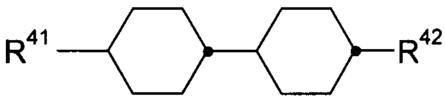

IV

worin

$R^{41}$ einen unsubstituierten Alkylrest mit 1 bis 7 C-Atomen oder einen unsubstituierten Alkenylrest mit 2 bis 7 C-Atomen, und
$R^{42}$ einen unsubstituierten Alkylrest mit 1 bis 7 C-Atomen, einen unsubstituierten Alkoxyrest mit 1 bis 6 C-Atomen, oder einen unsubstituierten Alkenylrest mit 2 bis 7 C-Atomen,

bedeuten, enthält.

**9.** Medium nach mindestens einem der Ansprüche 1 bis 8, **dadurch gekennzeichnet, dass** die Gesamtkonzentration der Verbindungen der Formel II im Gesamtmedium 25 % oder mehr bis 45 % oder weniger beträgt.

**10.** Medium nach mindestens einem der Ansprüche 1 bis 9, **dadurch gekennzeichnet, dass** es eine oder mehrere Verbindungen der Formel II-3, wie in Anspruch 4 angegeben, enthält.

**11.** Medium nach mindestens einem der Ansprüche 1 bis 10, **dadurch gekennzeichnet, dass** es zusätzlich eine oder mehrere chirale Verbindungen enthält.

**12.** Elektrooptische Anzeige oder elektrooptische Komponente **dadurch gekennzeichnet, dass** sie ein flüssigkristallines Medium nach mindestens einem der Ansprüche 1 bis 11 enthält.

**13.** Anzeige nach Anspruch 12, **dadurch gekennzeichnet, dass** sie auf dem VA- oder dem ECB-Effekt basiert.

**14.** Anzeige nach Anspruch 12 oder 13, **dadurch gekennzeichnet, dass** sie eine Aktivmatrix-Adressierungsvorrichtung aufweist.

**15.** Verwendung eines Mediums nach mindestens einem der Ansprüche 1 bis 11 in einer elektrooptischen Anzeige oder in einer elektrooptischen Komponente.

**16.** Verfahren zur Herstellung eines flüssigkristallinen Mediums nach einem oder mehreren der Ansprüche 1 bis 11,

**dadurch gekennzeichnet, dass** eine oder mehrere Verbindungen der Formel I mit einer oder mehreren Verbindungen der Formel II und/oder einer oder mehreren Verbindungen ausgewählt aus der Gruppe der Verbindungen der Formeln III-1 bis III-4 und/oder einer oder mehreren Verbindungen der Formel IV gemischt wird.

**17.** Verfahren zur Stabilisierung eines flüssigkristallinen Mediums, **dadurch gekennzeichnet, dass** das Medium eine oder mehrere Verbindungen der Formel II, wie in Anspruch 1 angegeben, enthält und dem Medium eine oder mehrere Verbindungen der Formeln I, wie in Anspruch 1 angegeben, und gegebenenfalls eine oder mehrere Verbindungen ausgewählt aus der Gruppe der Verbindungen der Formeln OH-1 bis OH-6

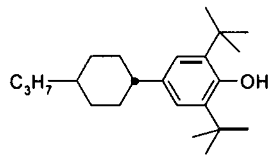

OH-1

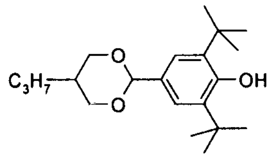

OH-2

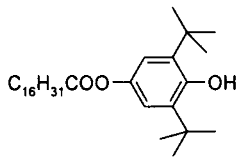

OH-3

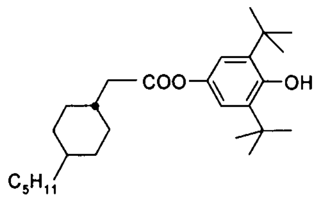

OH-4

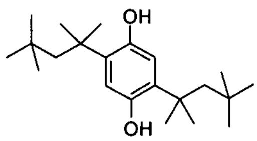

OH-5

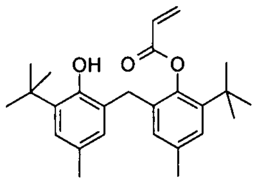

OH-6

zugesetzt wird.

## Claims

**1.** Liquid-crystalline medium comprising

a) one or more compounds of the formula I

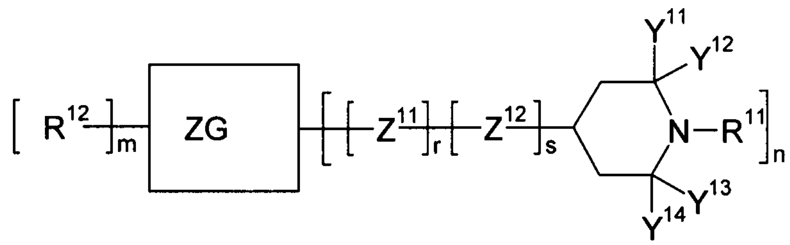

I

in which

n denotes 1, 2, 3 or 4,
m denotes (4-n),

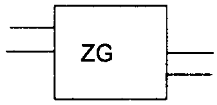

denotes an organic radical having 4 bonding sites,
$Z^{11}$ and $Z^{12}$, independently of one another, denote -O-, -(C=O)-, -(N-$R^{14}$)- or a single bond, but do not both simultaneously denote -O-,
r and s, independently of one another, denote 0 or 1,
$Y^{11}$ to $Y^{14}$ each, independently of one another, denote alkyl having 1 to 4 C atoms and alternatively, also independently of one another, one or both of the pairs ($Y^{11}$ and $Y^{12}$) and ($Y^{13}$ and $Y^{14}$) together denote a divalent group having 3 to 6 C atoms,
$R^{11}$ denotes $O^•$,
$R^{12}$ on each occurrence, independently of one another, denotes H, F, $OR^{14}$, $NR^{14}R^{15}$, a straight-chain or branched alkyl chain having 1-20 C atoms, in which one $-CH_2$-group or a plurality of $-CH_2$- groups may be replaced by -O- or -C(=O)-, but two adjacent $-CH_2$- groups cannot be replaced by -O-, or denotes a hydrocarbon radical which contains a cycloalkyl or alkylcycloalkyl unit, and in which one $-CH_2$- group or a plurality of $-CH_2$- groups may be replaced by -O- or -C(=O)-, but two adjacent $-CH_2$-groups cannot be replaced by -O-, and in which one H atom or a plurality of H atoms may be replaced by $OR^{14}$, $N(R^{14})(R^{15})$ or $R^{16}$, or denotes an aromatic or heteroaromatic hydrocarbon radical, in which one H atom or a plurality of H atoms may be replaced by $OR^{14}$, $N(R^{14})(R^{15})$ or $R^{16}$,
$R^{14}$ on each occurrence, independently of one another, denotes a straight-chain or branched alkyl or acyl group having 1 to 10 C atoms or an aromatic hydrocarbon or carboxyl radical having 6-12 C atoms,
$R^{15}$ on each occurrence, independently of one another, denotes a straight-chain or branched alkyl or acyl group having 1 to 10 C atoms or an aromatic hydrocarbon or carboxyl radical having 6-12 C atoms,
$R^{16}$ on each occurrence, independently of one another, denotes a straight-chain or branched alkyl group having 1 to 10 C atoms, in which one $-CH_2$- group or a plurality of $-CH_2$- groups may be replaced by -O- or -C(=O)-, but two adjacent $-CH_2$- groups cannot be replaced by -O-,

with the provisos that
compounds of the formula

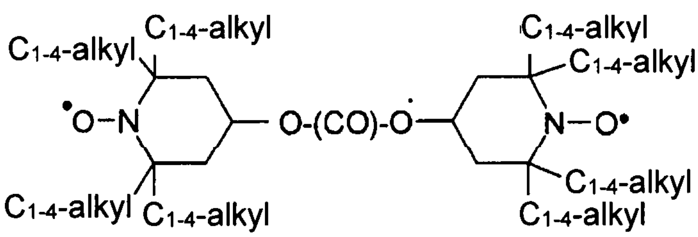

are excluded from the compounds of the formula I, and
in the case where n = 1 and

$-[Z^{11}-]_r-[Z^{12}]_s- $ = -O-, -(CO)-O-, -O-(CO)-, -O-(CO)-O-, $-NR^{14}-$ or $-NR^{14}-(CO)-$,

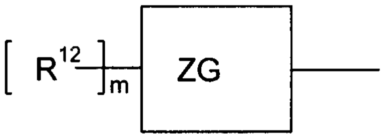

does not denote
a straight-chain or branched alkyl having 1 to 10 C atoms, also cycloalkyl, cycloalkylalkyl or alkylcycloalkyl, where one or more $-CH_2-$ groups in all these groups may be replaced by -O- in such a way that two O atoms in the molecule are not bonded directly to one another, and
b) one or more compounds of the formula II

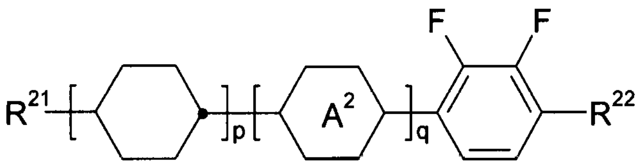

II

in which

$R^{21}$ denotes an unsubstituted alkenyl radical having 2 to 7 C atoms,
$R^{22}$ denotes an unsubstituted alkyl radical having 1 to 7 C atoms, an unsubstituted alkoxy radical having 1 to 6 C atoms or an unsubstituted alkenyloxy radical having 2 to 6 C atoms,

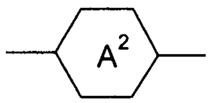

denotes

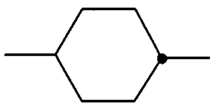
,
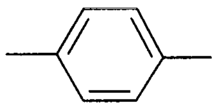
,
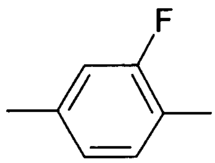
,

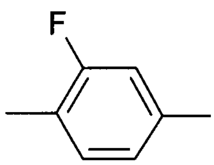
or
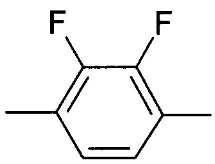
,

p and q each, independently of one another, denote 0, 1 or 2, and
(p + q) denotes 1, 2 or 3.

2. Medium according to Claim 1, **characterised in that** it comprises one or more compounds of the formula I selected from the group of the compounds of the formulae I-1, I-2, and I-5 to I-8

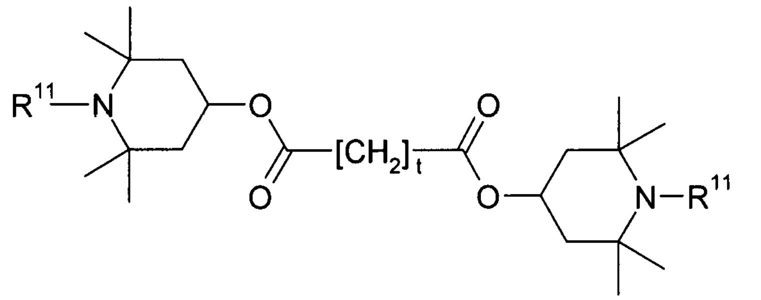

I-1

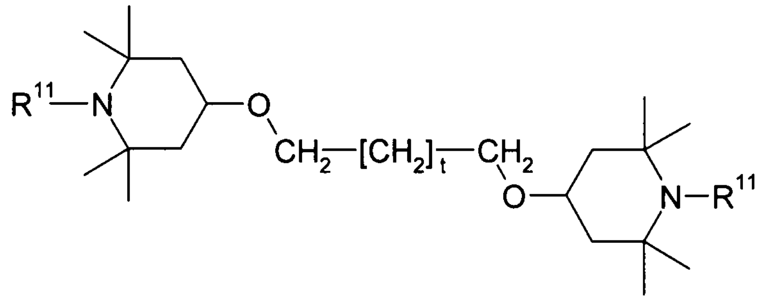

I-2

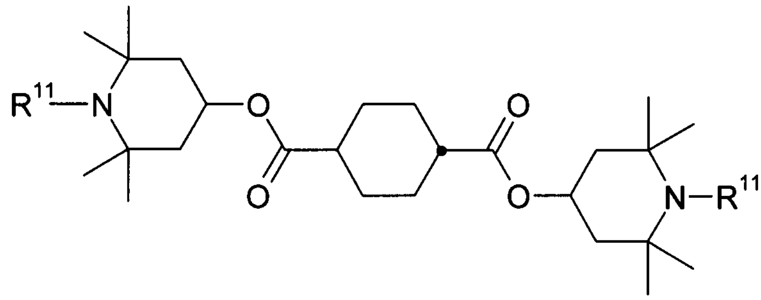

I-5

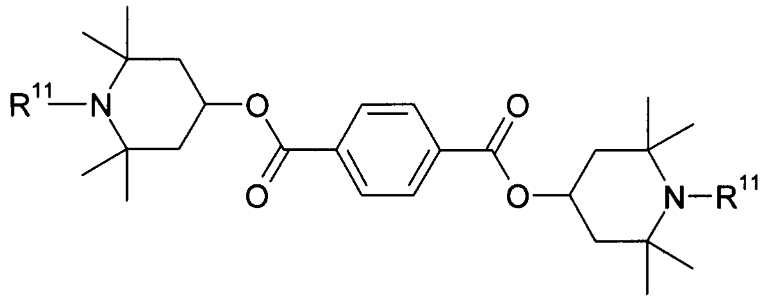

I-6

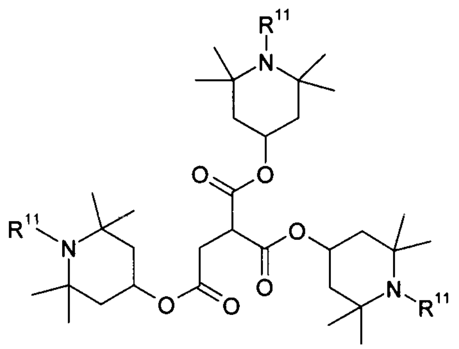

I-7

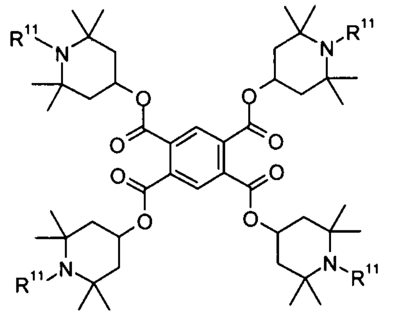

I-8

in which the parameters have the meanings indicated in Claim 1, and

t denotes an integer from 1 to 12,
$R^{17}$ denotes a straight-chain or branched alkyl chain having 1-12 C atoms, in which one $-CH_2-$ group or a plurality of $-CH_2-$ groups may be replaced by -O- or -C(=O)-, but two adjacent $-CH_2-$ groups cannot be replaced by -O-, or denotes an aromatic or heteroaromatic hydrocarbon radical, in which one H atom or a plurality of H atoms may be replaced by $OR^{14}$, $N(R^{14})(R^{15})$ or $R^{16}$.

3. Medium according to Claim 1 or 2, **characterised in that** it comprises one or more compounds of the formula I selected from the group of the compounds of the formulae I-1a-1 to I-2a-2 and I-5a-1 to I-8a-1

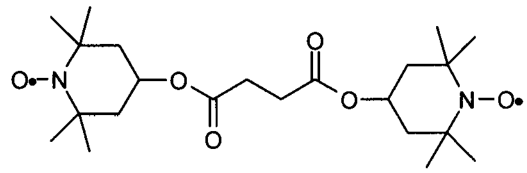

I-1a-1

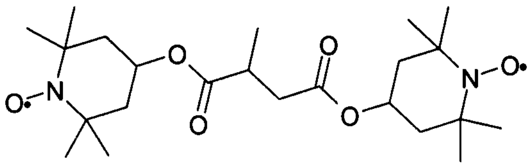

I-1a-2

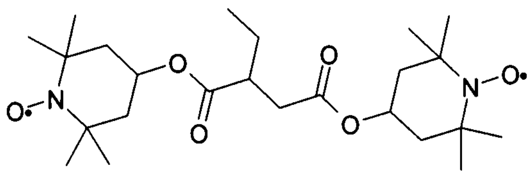

I-1a-3

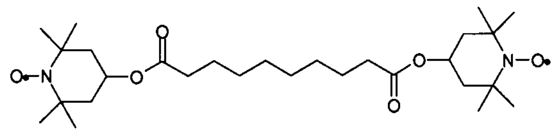

I-1a-4

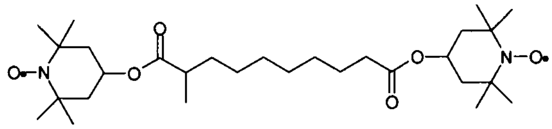

I-1a-5

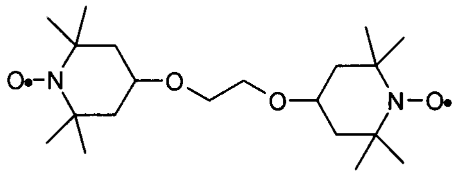

I-2a-1

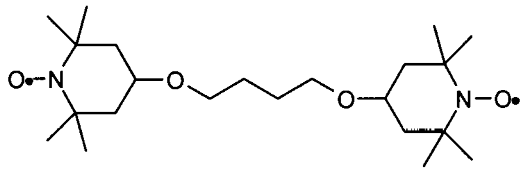

I-2a-2

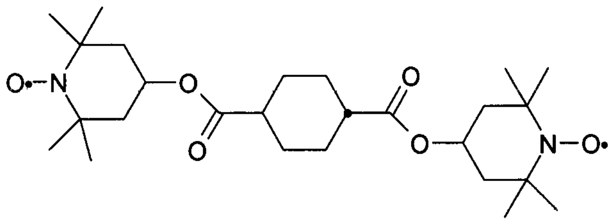

I-5a-1

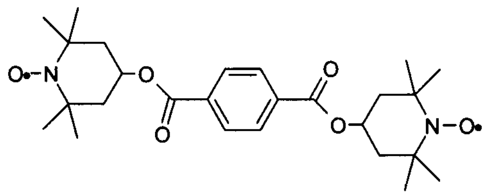

I-6a-1

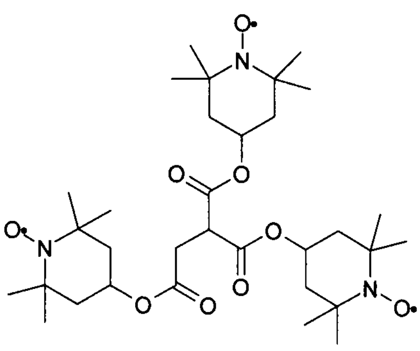

I-7a-1

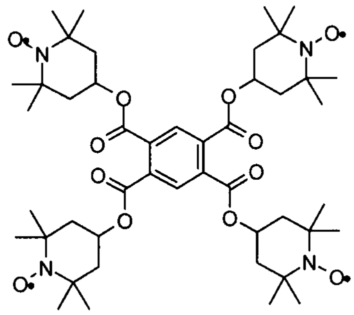

I-8a-1.

4. Liquid-crystalline medium according to Claim 3, **characterised in that** it comprises the compound of the formula I-1a-1

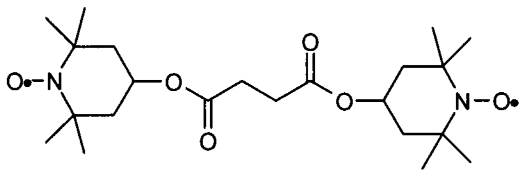

I-1a-1.

5. Liquid-crystalline medium according to at least one of Claims 1 to 4, **characterised in that** it comprises one or more compounds of the formula II selected from the group of the compounds of the formulae II-1 to II-4

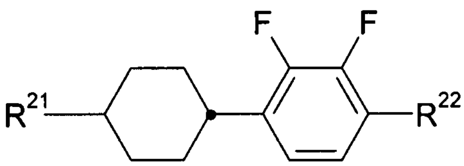

II-1

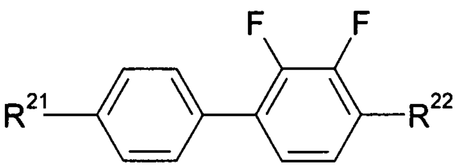

II-2

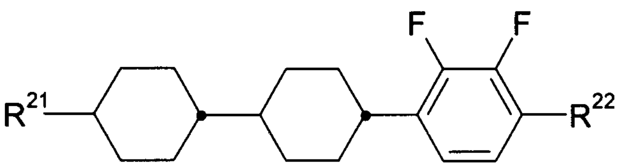

II-3

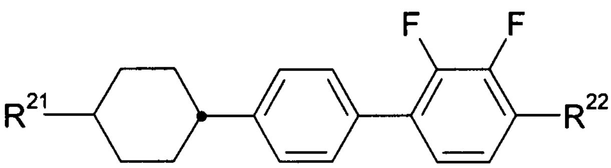

II-4

in which the parameters have the respective meanings given in Claim 1.

**6.** Liquid-crystalline medium according to at least one of Claims 1 to 5, **characterised in that** it additionally comprises

c) one or more compounds selected from the group of the compounds of the formulae III-1 to III-4

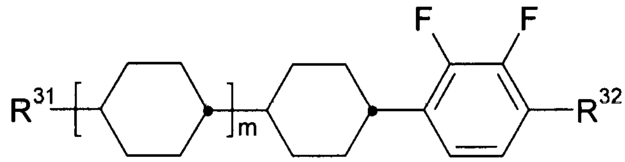

III-1

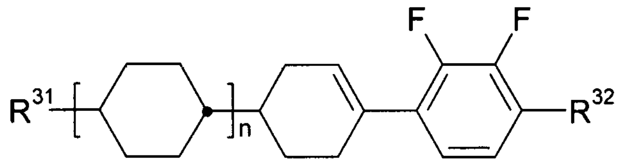

III-2

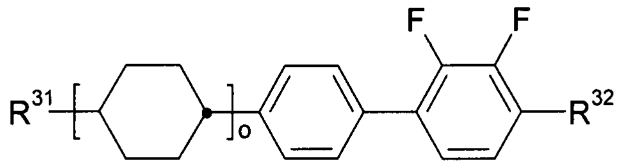

III-3

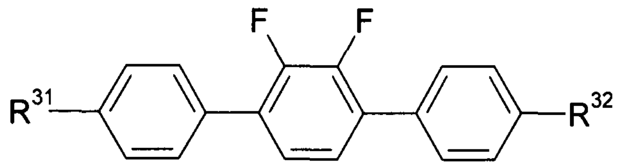

III-4

in which

$R^{31}$ denotes an unsubstituted alkyl radical having 1 to 7 C atoms,
$R^{32}$ denotes an unsubstituted alkyl radical having 1 to 7 C atoms, an unsubstituted alkoxy radical having 1 to 6 C atom or an unsubstituted alkenyloxy radical having 2 to 6 C atoms, and
m, n and o each, independently of one another, denote 0 or 1.

**7.** Medium according to at least one of Claims 1 to 6, **characterised in that** the total concentration of the compounds of the formula I in the medium as a whole is 1 ppm or more to 1000 ppm or less.

**8.** Medium according to at least one of Claims 1 to 7, **characterised in that** it additionally comprises

d) one or more compounds of the formula IV,

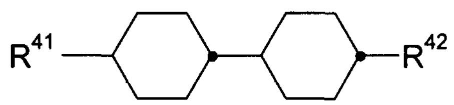

IV

in which

$R^{41}$ denotes an unsubstituted alkyl radical having 1 to 7 C atoms or an unsubstituted alkenyl radical having 2 to 7 C atoms, and
$R^{42}$ denotes an unsubstituted alkyl radical having 1 to 7 C atoms, an unsubstituted alkoxy radical having 1 to 6 C atoms, or an unsubstituted alkenyl radical having 2 to 7 C atoms.

**9.** Medium according to at least one of Claims 1 to 8, **characterised in that** the total concentration of the compounds of the formula II in the medium as a whole is 25% or more to 45% or less.

**10.** Medium according to at least one of Claims 1 to 9, **characterised in that** it comprises one or more compounds of the formula II-3, as indicated in Claim 4.

**11.** Medium according to at least one of Claims 1 to 10, **characterised in that** it additionally comprises one or more chiral compounds.

**12.** Electro-optical display or electro-optical component, **characterised in that** it contains a liquid-crystalline medium according to at least one of Claims 1 to 11.

**13.** Display according to Claim 12, **characterised in that** it is based on the VA or ECB effect.

**14.** Display according to Claim 12 or 13, **characterised in that** it contains an active-matrix addressing device.

**15.** Use of a medium according to at least one of Claims 1 to 11 in an electro-optical display or in an electro-optical component.

**16.** Process for the preparation of a liquid-crystalline medium according to one or more of Claims 1 to 11, **characterised in that** one or more compounds of the formula I are mixed with one or more compounds of the formula II and/or one or more compounds selected from the group of the compounds of the formulae III-1 to III-4 and/or one or more compounds of the formula IV.

**17.** Method for the stabilisation of a liquid-crystalline medium, **characterised in that** the medium comprises one or more compounds of the formula II, as indicated in Claim 1, and one or more compounds of the formula I, as indicated in Claim 1, and optionally one or more compounds selected from the group of the compounds of the formulae OH-1 to OH-6

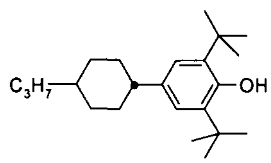

OH-1

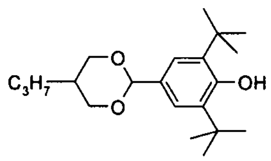

OH-2

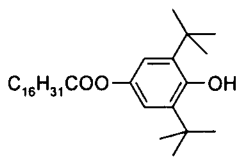

OH-3

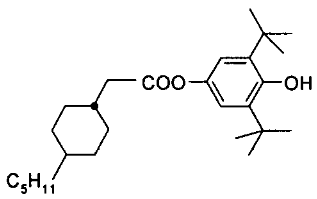

OH-4

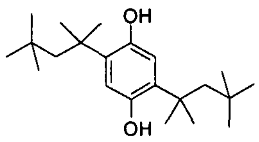

OH-5

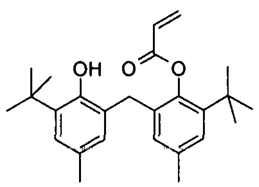

OH-6

are added to the medium.

## Revendications

**1.** Milieu cristallin liquide comprenant :

a) un ou plusieurs composé(s) de la formule I :

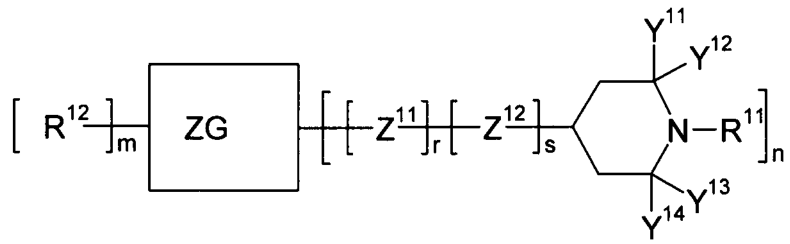

I

formule dans laquelle :

n représente 1, 2, 3 ou 4,
m représente (4-n),

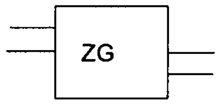

représente un radical organique qui comporte 4 sites de liaison,
$Z^{11}$ et $Z^{12}$ représentent, indépendamment l'un de l'autre, -O-, -(C=O)-, -(N-$R^{14}$)- ou une liaison simple, mais ne représentent pas tous deux simultanément -O-,
r et s représentent, indépendamment l'un de l'autre, 0 ou 1,
$Y^{11}$ à $Y^{14}$ représentent chacun, indépendamment les uns des autres, alkyle qui comporte 1 à 4 atome(s) de C et à titre d'alternative, également indépendamment l'une de l'autre, l'une des paires ($Y^{11}$ et $Y^{12}$) et ($Y^{13}$ et $Y^{14}$) ou ces deux paires représente(nt) ensemble un groupe divalent qui comporte 3 à 6 atomes de C,
$R^{11}$ représente $O^{\bullet}$,
$R^{12}$ représente pour chaque occurrence, indépendamment des autres, H, F, $OR^{14}$, $NR^{14}R^{15}$, une chaîne alkyle en chaîne droite ou ramifiée qui comporte 1 à 20 atome(s) de C, où un groupe $-CH_2-$ ou les groupes d'une pluralité de groupes $-CH_2-$ peut/peuvent être remplacé(s) par -O- ou -C(=O)-, mais deux groupes $-CH_2-$ adjacents ne peuvent pas être remplacés par -O-, ou représente un radical hydrocarbone qui contient une unité cycloalkyle ou alkylcycloalkyle, et où un groupe $-CH_2-$ ou les groupes d'une pluralité de groupes $-CH_2-$ peut/peuvent être remplacé(s) par -O- ou -C(=O)-, mais deux groupes $-CH_2$-adjacents ne peuvent pas être remplacés par -O-, et où un atome de H ou les atomes d'une pluralité d'atomes de H peut/peuvent être remplacé(s) par $OR^{14}$, $N(R^{14})(R^{15})$ ou $R^{16}$, ou représente un radical hydrocarbone aromatique ou hétéroaromatique, où un atome de H ou les atomes d'une pluralité d'atomes de H peut/ peuvent être remplacé(s) par $OR^{14}$, $N(R^{14})(R^{15})$ ou $R^{16}$,

$R^{14}$ représente pour chaque occurrence, indépendamment des autres, un groupe alkyle ou acyle en chaîne droite ou ramifié qui comporte 1 à 10 atome(s) de C ou un radical hydrocarbone ou carboxyle aromatique qui comporte 6 à 12 atomes de C,

$R^{15}$ représente pour chaque occurrence, indépendamment des autres, un groupe alkyle ou acyle en chaîne droite ou ramifié qui comporte 1 à 10 atome(s) de C ou un radical hydrocarbone ou carboxyle aromatique qui comporte 6 à 12 atomes de C,

$R^{16}$ représente pour chaque occurrence, indépendamment des autres, un groupe alkyle en chaîne droite ou ramifié qui comporte 1 à 10 atome(s) de C, où un groupe $-CH_2-$ ou les groupes d'une pluralité de groupes $-CH_2-$ peut/ peuvent être remplacé(s) par -O- ou -C(=O)-, mais deux groupes $-CH_2-$ adjacents ne peuvent pas être remplacés par -O-,

étant entendu que :

les composés de la formule :

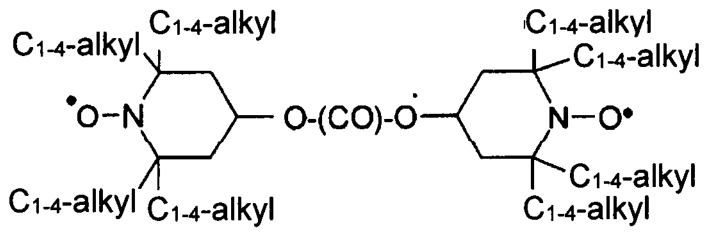

sont exclus des composés de la formule I, et

dans le cas où n = 1 et

$-[Z^{11}-]_r-[Z^{12}]_s-$ = -O-, -(CO)-O-, -O-(CO)-, -O-(CO)-O-, $-NR^{14}-$ ou $-NR^{14}-(CO)-$,

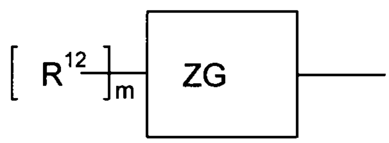

ne représente pas

un alkyle en chaîne droite ou ramifié qui comporte 1 à 10 atome(s) de C, également cycloalkyle, cycloalkylalkyle ou alkylcycloalkyle, où un ou plusieurs groupe(s) $-CH_2-$ dans tous ces groupes peut/peuvent être remplacé(s) par -O- de telle sorte que deux atomes de O dans la molécule ne soient pas liés directement l'un à l'autre, et

b) un ou plusieurs composé(s) de la formule II :

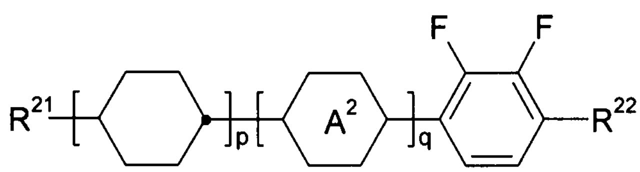

II

formule dans laquelle :

$R^{21}$ représente un radical alkényle non substitué qui comporte 2 à 7 atomes de C,

$R^{22}$ représente un radical alkyle non substitué qui comporte 1 à 7 atome(s) de C, un radical alcoxy non substitué qui comporte 1 à 6 atome(s) de C ou un radical alkényloxy non substitué qui comporte 2 à 6 atomes de C,

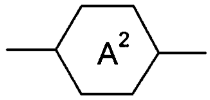

représente

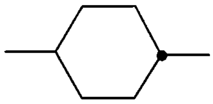,
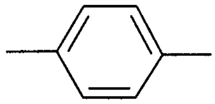,
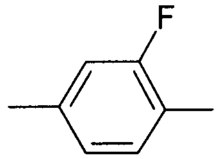,

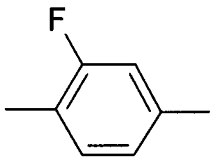
ou
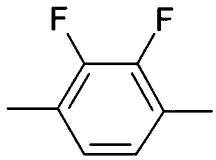,

p et q représentent chacun, indépendamment l'un de l'autre, 0, 1 ou 2, et
(p + q) représente 1, 2 ou 3.

**2.** Milieu selon la revendication 1, **caractérisé en ce qu'**il comprend un ou plusieurs composé(s) de la formule I qui est/sont sélectionné(s) parmi le groupe des composés des formules I-1, I-2, et I-5 à I-8 :

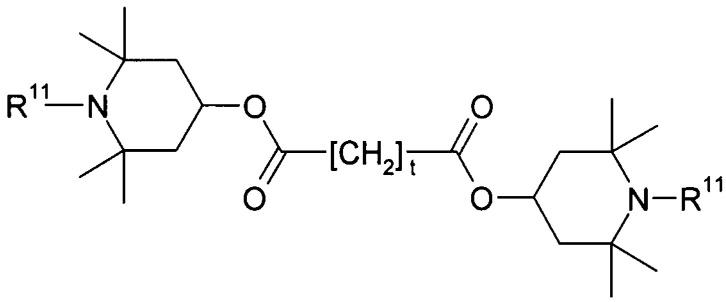
I-1

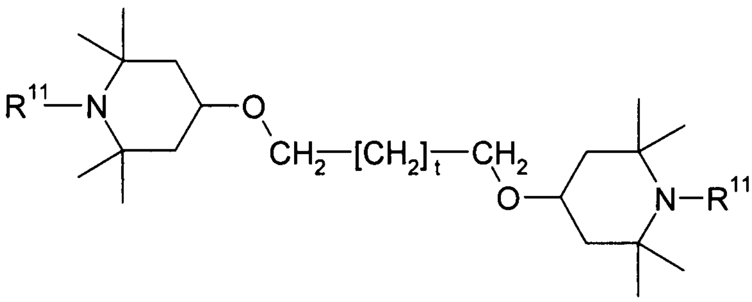
I-2

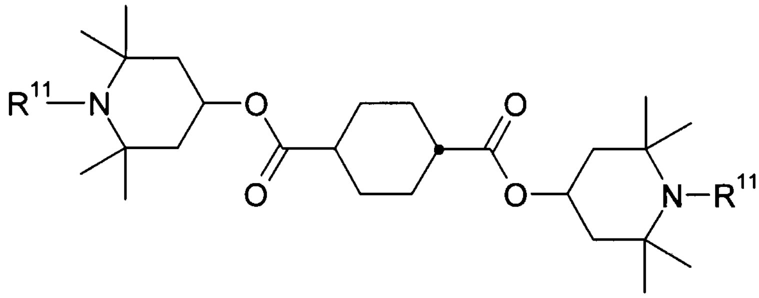

I-5

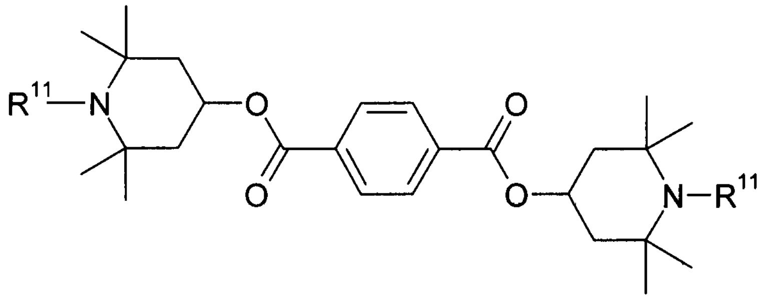

I-6

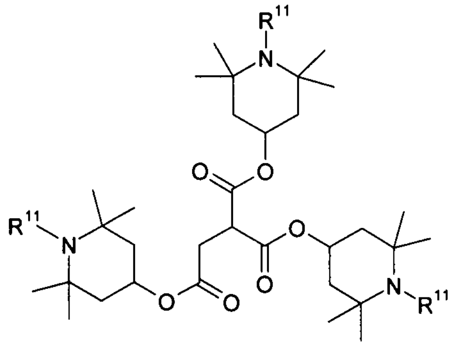

I-7

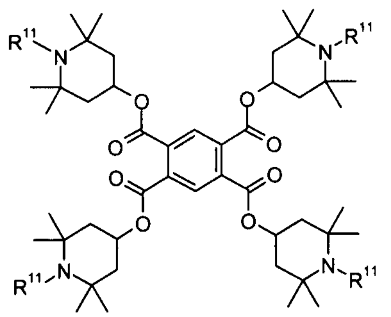

I-8

formules dans lesquelles les paramètres présentent les significations qui ont été indiquées selon la revendication 1, et

t représente un entier de 1 à 12,
$R^{17}$ représente une chaîne alkyle en chaîne droite ou ramifiée qui comporte 1 à 12 atome(s) de C, où un groupe $-CH_2-$ ou les groupes d'une pluralité de groupes $-CH_2-$ peut/peuvent être remplacé(s) par -O- ou -C(=O)-, mais deux groupes $-CH_2-$ adjacents ne peuvent pas être remplacés par -O-, ou représente un radical hydrocarbone aromatique ou hétéroaromatique, où un atome de H ou les atomes d'une pluralité d'atomes de H peut/ peuvent être remplacé(s) par $OR^{14}$, $N(R^{14})(R^{15})$ ou $R^{16}$.

**3.** Milieu selon la revendication 1 ou 2, **caractérisé en ce qu'**il comprend un ou plusieurs composé(s) de la formule I qui est/sont sélectionné(s) parmi le groupe des composés des formules I-1a-1 à I-2a-2 et I-5a-1 à I-8a-1 :

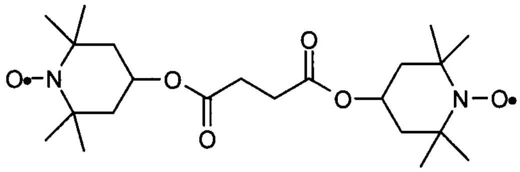

I-1a-1

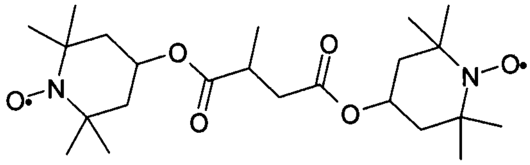

I-1a-2

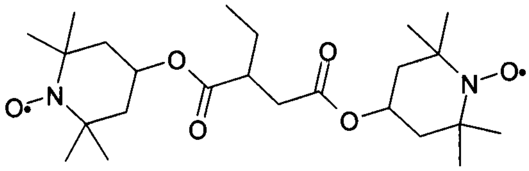

I-1a-3

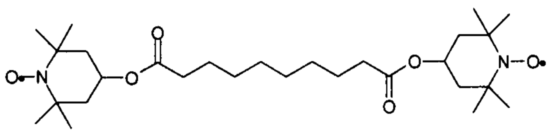

I-1a-4

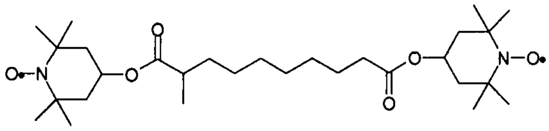

I-1a-5

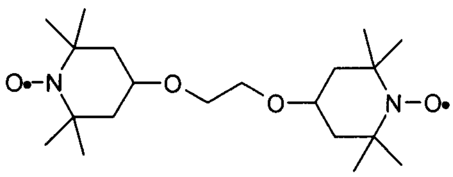

I-2a-1

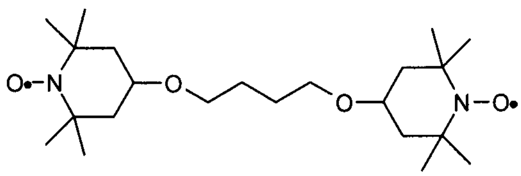

I-2a-2

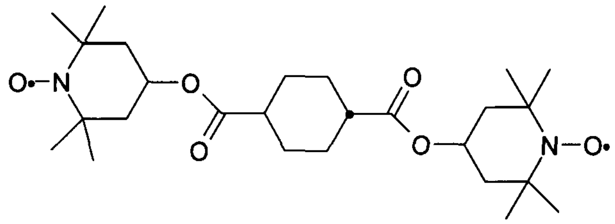

I-5a-1

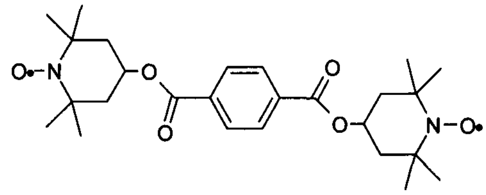

I-6a-1

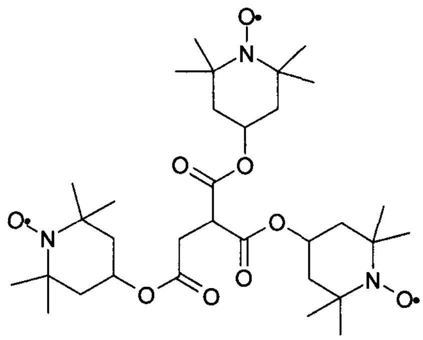

I-7a-1

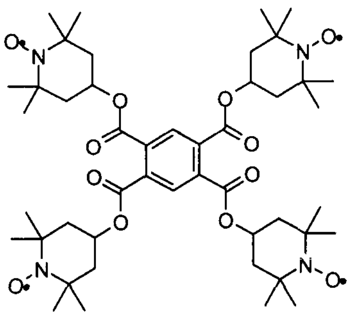

I-8a-1.

**4.** Milieu cristallin liquide selon la revendication 3, **caractérisé en ce qu'**il comprend le composé de la formule I-1a-1 :

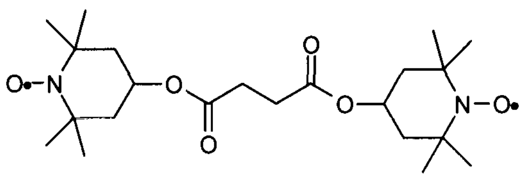

I-1a-1.

5. Milieu cristallin liquide selon au moins l'une des revendications 1 à 4, **caractérisé en ce qu'**il comprend un ou plusieurs composé(s) de la formule II qui est/sont sélectionné(s) parmi le groupe des composés des formules II-1 à II-4 :

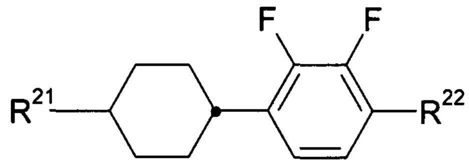

II-1

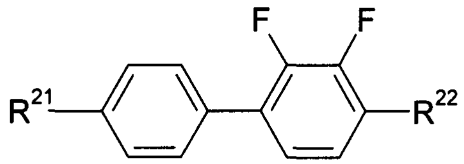

II-2

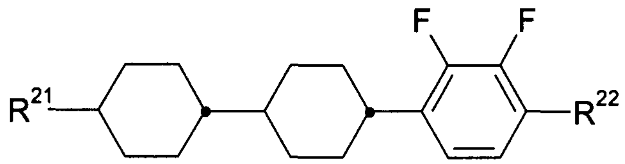

II-3

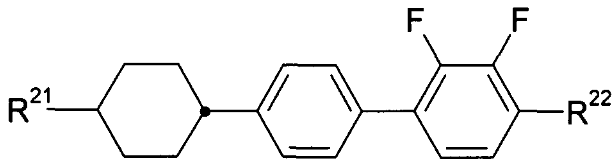

II-4

formules dans lesquelles les paramètres présentent les significations respectives qui ont été données selon la revendication 1.

6. Milieu cristallin liquide selon au moins l'une des revendications 1 à 5, **caractérisé en ce qu'**il comprend de façon additionnelle :

c) un ou plusieurs composé(s) qui est/sont sélectionné(s) parmi le groupe des composés des formules III-1 à III-4 :

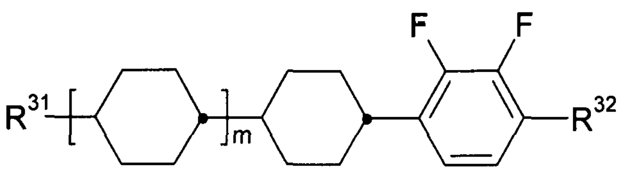

III-1

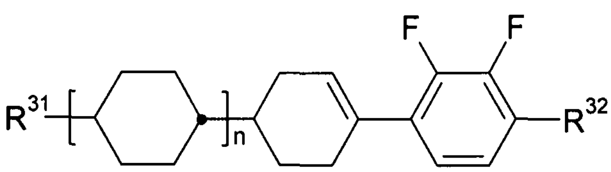

III-2

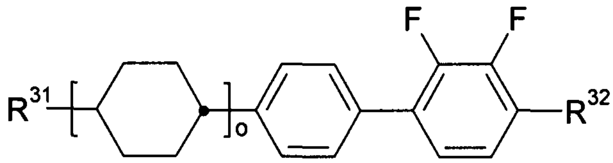

III-3

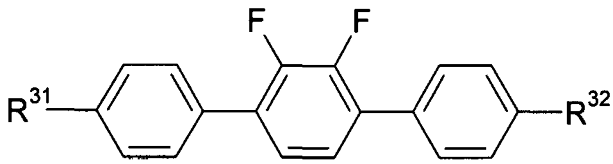

III-4

formules dans lesquelles :

$R^{31}$ représente un radical alkyle non substitué qui comporte 1 à 7 atome(s) de C,
$R^{32}$ représente un radical alkyle non substitué qui comporte 1 à 7 atome(s) de C, un radical alcoxy non substitué qui comporte 1 à 6 atome(s) de C ou un radical alkényloxy non substitué qui comporte 2 à 6 atomes de C, et
m, n et o représentent chacun, indépendamment les uns des autres, 0 ou 1.

7. Milieu selon au moins l'une des revendications 1 à 6, **caractérisé en ce que** la concentration totale des composés de la formule I dans le milieu pris dans sa globalité est de 1 ppm ou plus jusqu'à 1000 ppm ou moins.

8. Milieu selon au moins l'une des revendications 1 à 7, **caractérisé en ce qu'**il comprend de manière additionnelle :

d) un ou plusieurs composé(s) de la formule IV :

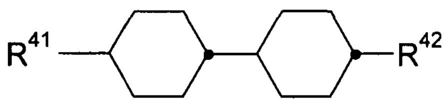

IV

formule dans laquelle :

$R^{41}$ représente un radical alkyle non substitué qui comporte 1 à 7 atome(s) de C ou un radical alkényle non substitué qui comporte 2 à 7 atomes de C, et
$R^{42}$ représente un radical alkyle non substitué qui comporte 1 à 7 atome(s) de C, un radical alcoxy non substitué qui comporte 1 à 6 atome(s) de C, ou un radical alkényle non substitué qui comporte 2 à 7 atomes de C.

9. Milieu selon au moins l'une des revendications 1 à 8, **caractérisé en ce que** la concentration totale des composés de la formule II dans le milieu pris dans sa globalité est de 25 % ou plus jusqu'à 45 % ou moins.

10. Milieu selon au moins l'une des revendications 1 à 9, **caractérisé en ce qu'**il comprend un ou plusieurs composé(s) de la formule II-3, comme indiqué selon la revendication 4.

11. Milieu selon au moins l'une des revendications 1 à 10, **caractérisé en ce qu'**il comprend de manière additionnelle un ou plusieurs composé(s) chiral/chiraux.

12. Affichage électro-optique ou composant électro-optique, **caractérisé en ce qu'**il contient un milieu cristallin liquide selon au moins l'une des revendications 1 à 11.

13. Affichage selon la revendication 12, **caractérisé en ce qu'**il est basé sur l'effet VA ou ECB.

14. Affichage selon la revendication 12 ou 13, **caractérisé en ce qu'**il contient un dispositif d'adressage par matrice active.

15. Utilisation d'un milieu selon au moins l'une des revendications 1 à 11 dans un affichage électro-optique ou un composant électro-optique.

16. Procédé pour la préparation d'un milieu cristallin liquide selon une ou plusieurs des revendications 1 à 11, **caractérisé en ce qu'**un ou plusieurs composé(s) de la formule I est/sont mélangé(s) avec un ou plusieurs composé(s) de la formule II et/ou un ou plusieurs composé(s) qui est/sont sélectionné(s) parmi le groupe des composés des formules III-1 à III-4 et/ou un ou plusieurs composé(s) de la formule IV.

17. Procédé pour la stabilisation d'un milieu cristallin liquide, **caractérisé en ce que** le milieu comprend un ou plusieurs composé(s) de la formule II, comme indiqué selon la revendication 1, et un ou plusieurs composé(s) de la formule I, comme indiqué selon la revendication 1, et en option, un ou plusieurs composé(s) qui est/sont sélectionné(s) parmi le groupe des composés des formules OH-1 à OH-6 :

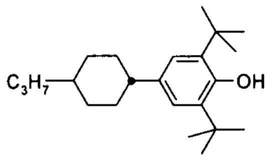

OH-1

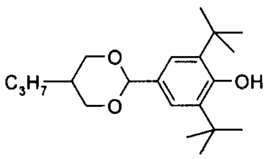

OH-2

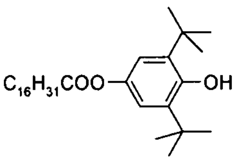

OH-3

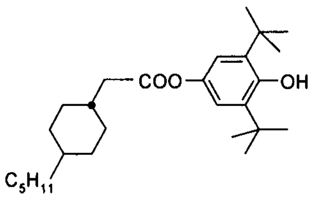

OH-4

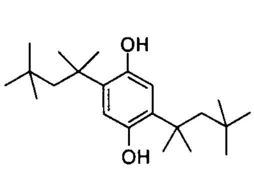

OH-5

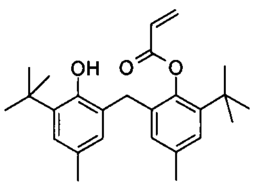

OH-6

est/sont ajoutés au milieu.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 2008006875 A1
- DE 102011008687 A1
- WO 2009129911 A1
- EP 2182046 A1
- WO 2008009417 A1
- WO 2009021671 A1
- WO 2009115186 A1
- JP S55023169 A
- JP H05117324 A
- WO 0218515 A1
- JP H09291282 A
- EP 11784442 A1
- US 2011278502 A1
- WO 2012076104 A1
- DE 102011117937
- DE 102012004871 A1
- EP 2514800 A2
- EP 0240379 A


### In der Beschreibung aufgeführte Nicht-Patentliteratur


- **M.F. SCHIECKEL ; K. FAHRENSCHON.** Deformation of nematic liquid crystals with vertical orientation in electrical fields. *Appl. Phys. Lett.,* 1971, vol. 19, 3912
- **ARBEITEN VON J.F. KAHN.** *Appl. Phys. Lett.,* 1972, vol. 20, 1193
- **G. LABRUNIE ; J. ROBERT.** *J. Appl. Phys.,* 1973, vol. 44, 4869
- **ARBEITEN VON J. ROBERT ; F. CLERC.** *SID 80 Digest Techn. Papers,* 1980, 30
- **J. DUCHENE.** *Displays 7,* 1986, 3
- **H. SCHAD.** *SID 82 Digest Techn. Papers,* 1982, 244
- **TOGASHI, S. ; SEKIGUCHI, K. ; TANABE, H. ; YAMAMOTO, E. ; SORIMACHI, K. ; TAJIMA, E. ; WATANABE, H. ; SHIMIZU, H.** A 210-288 Matrix LCD Controlled by Double Stage Diode Rings. *Proc. Eurodisplay,* September 1984, vol. 84, 141
- **STROMER, M.** Design of Thin Film Transistors for Matrix Adressing of Television Liquid Crystal Displays. *Proc. Eurodisplay,* September 1984, vol. 84, 145
- A LC Display for the TV Application. **YEO, S.D.** Digest of Technical Papers. International Symposium, 2004, vol. XXXV, 758, , 759
- MVA LCD for Notebook or Mobile PCs ... **YOSHIDE, H. et al.** Digest of Technical Papers. International Symposium, 2004, vol. XXXV, 6-9
- A 46-inch TFT-LCD HDTV Technology ... **LIU, C.T. et al.** Digest of Technical Papers. International Symposium, 2004, vol. XXXV, 750-753
- Super PVA Sets New State-of-the-Art for LCD-TV. **KIM ; SANG SOO.** Digest of Technical Papers. International Symposium, 2004, vol. XXXV, 760-763
- Development of High Quality LCDTV. **SHIGETA ; MITZUHIRO ; FUKUOKA ; HIROFUMI.** Digest of Technical Papers. International Symposium, 2004, vol. XXXV, 754-757
- **SOUK ; JUN.** Recent Advances in LCD Technology. *Seminar Lecture Notes,* 2004, M-6, , 1-M-6, 26
- **MILLER ; LAN.** LCD-Television. *Seminar Lecture Notes,* 2004, M-7, , 1-M-7, 32
- A 57-in. Wide UXGA TFT-LCD for HDTV Application. **KIM ; HYEON KYEONG et al.** Digest of Technical Papers. International Symposium, 2004, vol. XXXV, 106-109
- **OHKATSU, Y.** *J. of Japan Petroleum Institute,* 2008, vol. 51, 191-204
- **MIÉVILLE, P. et al.** *Angew. Chem.,* 2010, vol. 122, 6318-6321